(19) Europäisches Patentamt
European Patent Office
Office européen des brevets

(11) EP 2 164 979 B1

(12) # EUROPÄISCHE PATENTSCHRIFT

(45) Veröffentlichungstag und Bekanntmachung des Hinweises auf die Patenterteilung:
**17.07.2013 Patentblatt 2013/29**

(21) Anmeldenummer: **08760977.2**

(22) Anmeldetag: **12.06.2008**

(51) Int Cl.:
***C12Q 1/02*** *(2006.01)* ***C12Q 1/68*** *(2006.01)*
***G01N 33/18*** *(2006.01)*

(86) Internationale Anmeldenummer:
**PCT/EP2008/057444**

(87) Internationale Veröffentlichungsnummer:
**WO 2008/152124 (18.12.2008 Gazette 2008/51)**

(54) **BIOSENSOR**

BIOSENSOR

BIODÉTECTEUR

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.06.2007 DE 102007027619**

(43) Veröffentlichungstag der Anmeldung:
**24.03.2010 Patentblatt 2010/12**

(73) Patentinhaber:
- **Leibniz-Institut für Pflanzengenetik und Kulturpflanzenforschung
06466 Gatersleben (DE)**
- **Quodata-Gesellschaft für Qualitätsmanagement und Statistik mbH
01187 Dresden (DE)**

(72) Erfinder:
- **KUNZE, Gotthard
06466 Gatersleben (DE)**
- **FLORSCHÜTZ, Christina
06484 Quedlinburg (DE)**
- **STEINBORN, Gerhard
06466 Gatersleben (DE)**
- **KÖRNER, Martina
10409 Berlin (DE)**
- **SIMON, Kirsten
01187 Dresden (DE)**
- **HANKE, Gerold
01809 Dohna OT Meusegast (DE)**

(74) Vertreter: **Taruttis, Stefan Georg
TARUTTIS Patentanwaltskanzlei
Aegidientorplatz 2b
30159 Hannover (DE)**

(56) Entgegenhaltungen:
**DE-A1- 10 345 386 US-A- 4 513 280**

- **JUNGBAUER A ET AL: "Yeast reporter system for rapid determination of estrogenic activity" JOURNAL OF CHROMATOGRAPHY B: BIOMEDICAL SCIENCES & APPLICATIONS, ELSEVIER, AMSTERDAM, NL, Bd. 777, Nr. 1-2, 25. September 2002 (2002-09-25), Seiten 167-178, XP004381650 ISSN: 1570-0232**
- **HAHN ET AL: "A novel estrogen sensor based on recombinant Arxula adeninivorans cells" BIOSENSORS & BIOELECTRONICS, ELSEVIER SCIENCE PUBLISHERS, BARKING, GB, Bd. 21, Nr. 11, 15. Mai 2006 (2006-05-15), Seiten 2078-2085, XP005365037 ISSN: 0956-5663 in der Anmeldung erwähnt**
- **KARUBE I ET AL: "MICROBIAL BIOSENSORS FOR PROCESS AND ENVIRONMENTAL CONTROL" IEEE ENGINEERING IN MEDICINE AND BIOLOGY MAGAZINE, IEEE SERVICE CENTER, PISACATAWAY, NJ, US, Bd. 13, Nr. 3, 1. Juni 1994 (1994-06-01), Seiten 364-374, XP000456209 ISSN: 0739-5175**
- **LEIBNITZ-INSTITUT: "Jahresforschungsbericht 2004"[Online] Januar 2005 (2005-01), Seiten 1-192, XP002511869 Institut für Pflanzengenetik und Kulturpflanzenforschung, Gatersleben Gefunden im Internet: URL:http://www.ipk-gatersleben.de/Internet /Infrastruktur/ Oeffentlichkeitsarbeit/Jahr esberichte/JB2004.pdf> [gefunden am 2009-01-26]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 164 979 B1

- **AGAPHONOV MICHAEL O ET AL: "Vectors for rapid selection of integrants with different plasmid copy numbers in the yeast Hansenula polymorpha DL1", YEAST, vol. 15, no. 7, May 1999 (1999-05), pages 541-551, ISSN: 0749-503X**
- **ERHART E ET AL: "THE PRESENCE OF A DEFECTIVE LEU-2 GENE ON 2 MICRON DNA RECOMBINANT PLASMIDS OF SACCHAROMYCES-CEREVISIAE IS RESPONSIBLE FOR CURING AND HIGH COPY NUMBER", JOURNAL OF BACTERIOLOGY, vol. 156, no. 2, 1983, pages 625-635, ISSN: 0021-9193**
- **GATZKE R ET AL: "Stable multicopy integration of vector sequences in Hansenula polymorpha", APPLIED MICROBIOLOGY AND BIOTECHNOLOGY, vol. 43, no. 5, 1995, pages 844-849, ISSN: 0175-7598**
- **LOPES T S ET AL: "HIGH-COPY-NUMBER INTEGRATION INTO THE RIBOSOMAL DNA OF SACCHAROMYCES CEREVISIAE: A NEW VECTOR FOR HIGH-LEVEL EXPRESSION", GENE, ELSEVIER, AMSTERDAM, NL, vol. 79, no. 2, 1 January 1989 (1989-01-01), pages 199-206, ISSN: 0378-1119, DOI: 10.1016/0378-1119(89)90202-3**
- **JURETZEK T ET AL: "VECTORS FOR GENE EXPRESSION AND AMPLIFICATION IN THE YEAST YARROWIA LIPOLYTICA", YEAST, JOHN WILEY & SONS LTD, GB, vol. 18, no. 2, 30 January 2001 (2001-01-30), pages 97-113, ISSN: 0749-503X, DOI: 10.1002/1097-0061(20010130)18:2<97::AID-YE A652>3.0.CO;2-U**


Bemerkungen:

Die Akte enthält technische Angaben, die nach dem Eingang der Anmeldung eingereicht wurden und die nicht in dieser Patentschrift enthalten sind.

SEQUENCE LISTING

<110> Leibniz-Institut für Pflanzengenetik und Kulturpflanzenforschung Quodata-Gesellschaft für Qualitätsmanagement und Statistik mbH

<120> Biosensor

<130> I1003PCT

<160> 10

<170> PatentIn version 3.4

<210> 1
<211> 6404
<212> DNA
<213> Artificial

<220>
<223> Seq ID Nr. 1: Integrativer Abschnitt mit den Expressionskassetten des Östrogenrezeptors, des Reportergens und des Selektionsmarkers mit verkürztem Promoter

<220>
<221> rRNA
<222> (1)..(320)
<223> 25S rDNA2

<220>

<221> terminator
<222> (324)..(443)
<223> PHO5 -Terminator

<220>
<221> misc_feature
<222> (556)..(2293)
<223> orf hERalpha

<220>
<221> promoter
<222> (2591)..(2898)
<223> TEF1-Promotor

<220>
<221> promoter
<222> (2947)..(3040)
<223> dALEU2 -Promotor

<220>
<221> misc_feature
<222> (3045)..(3765)
<223> orf ATRP1

<220>
<221> promoter
<222> (3929)..(4610)
<223> GAA2ERE107

<220>
<221> misc_feature
<222> (4615)..(5881)
<223> orf phyK

<220>
<221> terminator
<222> (5926)..(6045)
<223> PHO5 -Terminator

<220>
<221> rRNA
<222> (6070)..(6371)
<223> 25S rDNA2

<400> 1

gccatggcca ccgtccggct gtctagatga actaacacct tttctggtgt ctgatgagcg 60
tgcattccgg caccttaact ctacgttcgg ttcatcccgc atcgccagtt ctgcttacca 120
aaaatggccc actaaaagct cttcattcta atgtccacgt tcaattaagt aacaaggact 180
tcttacatat tcaaagtttg agaataggtc aagatcgttt caatcccaat acctctaatc 240
attcgcttta cctcataaaa ctgatacgag cttctgctat cctgagggaa acttcggcag 300
gaaccagcta ctagatggtt cgaaatttta atctttcggc aaaatttaga taaaaaattt 360
gggtattcgt atttagtttc caatattatt tagttataca aaactattgt ctcaatagac 420
tggcgttgta atgagtagtg ttcccctcga ctctagggaa ttagcggccg ctaattccct 480
agaggatcct ccagccaggg agctctcaga ctgtggcagg gaaaccctct gcctccccg 540
tgatgtaata cttttgcaag gaatgcgatg aagtagagcc cgcagtggcc aagtggcttt 600
ggtccgtctc ctccacggat gcccctccac ggctagtggg cgcatgtagg cggtgggcgt 660
ccagcatctc cagcagcagg tcatagaggg gcaccacgtt cttgcacttc atgctgtaca 720
gatgctccat gcctttgtta ctcatgtgcc tgatgtggga gaggatgagg aggagctggg 780
ccagccgctg gtgctgctgc tgcagggtca ggcctgcctt ggccatcagg tggatcaaag 840
tgtctgtgat cttgtccagg actcggtgga tatggtcctt ctcttccaga gacttcaggg 900
tgctggacag aaatgtgtac actccagaat taagcaaaat aatagatttg aggcacacaa 960
actcctctcc ctgcagattc atcatgcgga accgagatga tgtagccagc agcatgtcga 1020
agatctccac catgccctct acacattttc cctggttcct gtccaagagc aagttaggag 1080
caaacagtag cttcactggg tgctccatgg agcgccagac gagaccaatc atcaggatct 1140
ctagccaggc acattctaga aggtggacct gatcatggag ggtcaaatcc acaaagcctg 1200
gcaccctctt cgcccagttg atcatgtgaa ccagctccct gtctgccagg ttggtcagta 1260
agcccatcat cgaagcttca ctgaagggtc tggtaggatc atactcggaa tagagtatgg 1320
ggggctcagc atccaacaag gcactgacca tctggtcggc cgtcagggac aaggccaggc 1380
tgttcttctt agagcgtttg atcatgagcg ggcttggcca aaggttggca gctctcatgt 1440
ctccagcaga ccccacttca cccctgccct ccccatcatc tctctggcgc ttgtgtttca 1500
acattctccc tcctcttcgg tcttttcgta tcccaccttt catcattccc acttcgtagc 1560
atttgcggag ccggcaggcc tggcagctct tcctcctgtt tttatcaatg gtgcactggt 1620
tggtggctgg acacatatag tcgttatgtc cttgaatact tctcttgaag aaggccttgc 1680
agccctcaca ggaccagact ccataatggt agcctgaagc atagtcattg cacactgcac 1740
agtagcgagt ctccttggca gattccatag ccatacttcc cttgtcattg gtactggcca 1800
atctttctct gccaccctgg cgtcgattat ctgaatttgg cctgtagaat gccggcgggc 1860

cggcctcgcg caccgtgtag ccgctgggct cgttctccag gtagtagggc acctgctggc 1920
cgtggggctg caggaaaggc gacagctgcg gcggcgggtg cagtagcatc agcgggctcg 1980
gagacacgct gttgagtggg gggaaacccc ccaggccgtt ggagccgaac gccgcagcct 2040
cagacccggg gccgtagggg aggccggtct gaccgtagac ctgcgcgttg gcggcggccg 2100
cggcgttgaa ctcgtaggcg gcgccctcgg ggtagttgta cacggcgggc ttgctgctgt 2160
ccaggtacac ctcgcccagg ggccgctcca gggggatctt gagctgcgga cggttcaggg 2220
gctccagctc gttcccttgg atctgatgca gtagggccat cccagatgct ttggtgtgga 2280
gggtcatggt catggtccgt ggccgcgggc agggtgcaga ccgtgtcccc gcagggcaga 2340
aggctcagaa accggcgggc cacctggaaa aagagcacag cccgaggtta gaggcgacgc 2400
agcgcatgtc ccgccgacac gcgagctctg gccccggccc tgccccggga gcctgcgggt 2460
ccggtgtgct ttcagtttgt ataacgctac aaagggctgg cctaaagtgt acatagtgta 2520
aacagtgatc attacagtat ttctccttca tcaaaaatac aagaaacatc acaattttgg 2580
aattcgatat ctctgttgat tatgttttta agaactactc agaatgaaat gccttgattt 2640
ccggtgtgag ttgacaagga aaaaaattcc aggagcaggg ggagctttat ataacaccag 2700
attagcggcg aaaaattttt tttctcgagg ggttacccgg tcgtcctttg cctaatgggg 2760
aaacatcccg ctcgccgcac gtaccactta gggctcccca aaaagcaacc atcctccgct 2820
catctcgagg gagatcacta aacgatatca actttgaggg ctcttgttga ctatggactg 2880
attatagatt gaagtcgaac tagtggatcc cccgggctgc aggcatgcaa gctggggccg 2940
ccgcgggctg ccccgggccg aaaccggcat tttgggttga ctcttttaag gaacgacatg 3000
cccaggggac tctccactca caaacccttt caaaaacctg tacaatgaca acgtckgcca 3060
atcgtcaagg tgtgcggaat caggaccgtg gaggcggccc aggtcgcttt ggatgctgga 3120
gccaacatga ttggaatgat tatggtccct aatagagcgc gcactgttga tatggagaca 3180
gcaggcaaga tatcacgact ggtacgatca tatagacgtc aaggatcctt tgagctggac 3240
actaatgagc tgggcggatc gactcactct gcactcgccc acatcattga agccaagaca 3300
aggacccggc cagcaatcgt aggcgtgttc cgcaaccagc cgctgtcaga agtcctgcgg 3360
ttgcaacagg aattgtcgct ggatttcgtt cagcttcacg gatcagagcc cctagaatgg 3420
tgtagactga ttccatgtcc tgtgatccgt cggttcaccc ccgatactcc agatttctat 3480
gcctcgccgg ttactggata ccacagccta tccctcatag acggtgaggt tggcggagag 3540
ggcaagatgg tggactggag cggagtccag tctcgggccc gaatgggagc gaggttcctt 3600
atggctggag gattgacagc cgacaatgtt gcccaagctc tagcggtcga gggtgcctgt 3660
ggtgttgatg tcagcggagg agtcgaatca agcccggggg tcaaagatct caacgagatt 3720
gagcggtttg tcaaagctgc caagggggtt actgtgaagt attaacactc cagcagcagc 3780
atcaataggt gatactgacg acaggtaatg tgtactaata ggtctgacag cagaatacag 3840
ctatattact tctcatttga aacttgaggt gattgacaat ttactattta tacaagctaa 3900

gcttcaatac agactcgatc gaattcaggt cgacacagac cagacggaga tcatcctaac 3960
attagcaatt ccaaatccgc cgacccgttc gttactcagg ttgatcataa ggggtcctca 4020
ttccccaaga ggagaaaacg gtgtcttaat tgcgcacctc ttgtttttgt agcatttttc 4080
aactaactag accgttcgtt ctatacctaa ttacaacatc cgcccacctt tctccttatt 4140
gatttgcaat tactatccga atagcgccga tattgtttgg tgccgtagat gcaatgttat 4200
tacggcagga aaaatatgta cggataattg ttccgcgaaa atccggaatc gtaaattgta 4260
ttatcggatc tgatgaatca gctatgaaag ataatatatg gtatagctac gactatagta 4320
aaccatagtc aaaatcaaaa gtttcaacgt tttcactgtt atctcattta tccccggatt 4380
ggccaagaaa ctaaggtttt ttggaccctg gaacatgcat aatgcggaga tctcactcgg 4440
cccgacttgg aagtgtaagg gaaggtcaga gtgacctagg tcagagtgac ctaggtcaga 4500
gtgaggggat ccaactcggc ccgactggaa ggtgcaatga gtgaatggaa gtagactgga 4560
ggactatata aacactcgca gcctggttac aattgacgtg gacgaaacag aattcatgcc 4620
tgcaagacat caggggctgt tacgcctgtt tatcgcctgc gcgctgccgc tgctggcgct 4680
gcactcagcc gccgccgcag actggcagct ggagaaagtg gtggagctta gccgccacgg 4740
cattcgtccg ccgacggccg gcaaccggga agccatcgag gccgccaccg gacgcccatg 4800
gaccgagtgg accacccatg acggcgagct caccggtcat ggctatgccg ccgtggtcaa 4860
caaagggcgt gaggaaggcc agcactatcg ccagctcggc ctgctgcagg cgggatgccc 4920
gacggcggag tcgatttacg tgcgcgccag cccgctgcag cggacgcggg cgaccgccca 4980
ggcgctggtg gatggcgcct tccccggctg tggcgtcgct atccattacg tcaacgggga 5040
tgccgatccg ctgtttcaga ccgacaagtt cgccgccacg caaaccgacc ccgcccgcca 5100
gctggcggcg gtgaaagaga aggccgggga tctggcgcag cgtcggcagg cgctggcgcc 5160
gactatccag ctattgaaac aggcggtttg ccaggccgat aagccctgcc cgatctttga 5220
taccccatgg cgggtcgagc agagcaaaag cggcaagacc accattagcg gactgagcgt 5280
gatggccaat atggtggaaa cgctgcgtct cggctggagt gaaaacctgc ctctcagcca 5340
gctggcgtgg ggcaagatcg cccaggccag tcagatcacg gccctgctgc cgctgttaac 5400
ggaaaactac gatctgagta acgacgtgct gtataccgcg caaaaacgcg ggtcggtgct 5460
gctcaacgcc atgctcgacg gcgtcaaacc agaggcgaat ccgaacgtac gctggctgct 5520
gctggtggcg catgacacca acatcgccat ggtgcgcacg ctgatgaact ttagctggca 5580
gctgccgggc tacagccggg ggaatatccc gccgggcagt agcctggtgc tggagcgctg 5640
gcgcgacgcg aagagcggtg aacgctatct gcgggtctat ttccaggcgc aaggcctcga 5700
cgacctgcgt cgtctgcaga cgccggacgc gcagcacccg atgctgcgtc aggagtggcg 5760
tcagccgggc tgccgtcaga ccgacgtcgg tacgctgtgc cccttccagg cggccatcac 5820
cgcgttgggt cagcgtatcg atcggccctc caccccggcg gtagccatgg tcctgccgta 5880
ggatcctcta gggaattagc ggccgctaat tccctagagt cgaggggaac actactcatt 5940
acaacgccag tctattgaga caatagtttt gtataactaa ataatattgg aaactaaata 6000

```
cgaataccca aatttttttat ctaaattttg ccgaaagatt aaaatctgca ggcatgcaag   6060
ctggggcccc atctcttagg atcgactaac ccgtggccaa ctgctgttca cacggaacct   6120
ttccccactt cagtcttcaa agttctcatt tgaatatttg ctactaccac caagatctgc   6180
actagaggcc gttcgaccca gcatcactgc aaaggcttcg tcactgacct ccacgcctgc   6240
ctactcgtta gggcatcgca ttaacctaac ggtagagtat aggtaacacg cttgagcgcc   6300
atccattttc agggctagtt cattcggccg gtgagttgtt acacactcct tagcggattc   6360
cgacttccat ggcgagcttg gcgtaatcat ggtcatagct gttt                      6404
```

<210> 2
<211> 6813
<212> DNA
<213> Artificial

<220>
<223> Seq ID Nr. 2: Integrativer Abschnitt mit den Expressionskassetten des Östrogenrezeptors, des Reportergens und des Selektionsmarkers mit unbeeinträchtigtem Promoter

<220>
<221> rRNA
<222> (1)..(322)
<223> 25S rDNA2

<220>
<221> terminator
<222> (324)..(443)
<223> PHO5 -Terminator

<220>
<221> misc_feature
<222> (556)..(2293)
<223> orf hERalpha

<220>
<221> promoter
<222> (2591)..(2898)
<223> TEF1-Promotor

<220>
<221> promoter
<222> (2919)..(3483)
<223> ALEU2-Promotor

<220>
<221> misc_feature
<222> (3512)..(4208)
<223> orf ATRP1

<220>
<221> promotor
<222> (4372)..(5052)
<223> GAA2ERE107

<220>

<221> misc_feature
<222> (5057)..(6320)
<223> orf phyK

<220>
<221> terminator
<222> (6377)..(6487)
<223> PHO5 -Terminator

<220>
<221> rRNA
<222> (6512)..(6813)
<223> 25S rDNA2

<400> 2

gccatggcca ccgtccggct gtctagatga actaacacct tttctggtgt ctgatgagcg 60
tgcattccgg caccttaact ctacgttcgg ttcatcccgc atcgccagtt ctgcttacca 120
aaaatggccc actaaaagct cttcattcta atgtccacgt tcaattaagt aacaaggact 180
tcttacatat tcaaagtttg agaataggtc aagatcgttt caatcccaat acctctaatc 240
attcgcttta cctcataaaa ctgatacgag cttctgctat cctgagggaa acttcggcag 300
gaaccagcta ctagatggtt cgaaatttta atctttcggc aaaatttaga taaaaaattt 360
gggtattcgt atttagtttc caatattatt tagttataca aaactattgt ctcaatagac 420
tggcgttgta atgagtagtg ttcccctcga ctctagggaa ttagcggccg ctaattccct 480
agaggatcct ccagccaggg agctctcaga ctgtggcagg gaaaccctct gcctcccccg 540
tgatgtaata cttttgcaag gaatgcgatg aagtagagcc cgcagtggcc aagtggcttt 600
ggtccgtctc ctccacggat gcccctccac ggctagtggg cgcatgtagg cggtgggcgt 660
ccagcatctc cagcagcagg tcatagaggg gcaccacgtt cttgcacttc atgctgtaca 720
gatgctccat gcctttgtta ctcatgtgcc tgatgtggga gaggatgagg aggagctggg 780
ccagccgctg gtgctgctgc tgcagggtca ggcctgcctt ggccatcagg tggatcaaag 840
tgtctgtgat cttgtccagg actcggtgga tatggtcctt ctcttccaga gacttcaggg 900
tgctggacag aaatgtgtac actccagaat taagcaaaat aatagatttg aggcacacaa 960
actcctctcc ctgcagattc atcatgcgga accgagatga tgtagccagc agcatgtcga 1020
agatctccac catgccctct acacattttc cctggttcct gtccaagagc aagttaggag 1080
caaacagtag cttcactggg tgctccatgg agcgccagac gagaccaatc atcaggatct 1140
ctagccaggc acattctaga aggtggacct gatcatggag ggtcaaatcc acaaagcctg 1200
gcaccctctt cgcccagttg atcatgtgaa ccagctccct gtctgccagg ttggtcagta 1260
agcccatcat cgaagcttca ctgaagggtc tggtaggatc atactcggaa tagagtatgg 1320
ggggctcagc atccaacaag gcactgacca tctggtcggc cgtcagggac aaggccaggc 1380
tgttcttctt agagcgtttg atcatgagcg ggcttggcca aaggttggca gctctcatgt 1440
ctccagcaga ccccacttca cccctgccct ccccatcatc tctctggcgc ttgtgtttca 1500
acattctccc tcctcttcgg tcttttcgta tcccaccttt catcattccc acttcgtagc 1560
atttgcggag ccggcaggcc tggcagctct tcctcctgtt tttatcaatg gtgcactggt 1620
tggtggctgg acacatatag tcgttatgtc cttgaatact tctcttgaag aaggccttgc 1680
agccctcaca ggaccagact ccataatggt agcctgaagc atagtcattg cacactgcac 1740
agtagcgagt ctccttggca gattccatag ccatacttcc cttgtcattg gtactggcca 1800

atctttctct gccaccctgg cgtcgattat ctgaatttgg cctgtagaat gccggcgggc 1860
cggcctcgcg caccgtgtag ccgctgggct cgttctccag gtagtagggc acctgctggc 1920
cgtggggctg caggaaaggc gacagctgcg gcggcgggtg cagtagcatc agcgggctcg 1980
gagacacgct gttgagtggg gggaaacccc ccaggccgtt ggagccgaac gccgcagcct 2040
cagacccggg gccgtagggg aggccggtct gaccgtagac ctgcgcgttg gcggcggccg 2100
cggcgttgaa ctcgtaggcg gcgccctcgg ggtagttgta cacggcgggc ttgctgctgt 2160
ccaggtacac ctcgcccagg ggccgctcca gggggatctt gagctgcgga cggttcaggg 2220
gctccagctc gttcccttgg atctgatgca gtagggccat cccagatgct ttggtgtgga 2280
gggtcatggt catggtccgt ggccgcgggc agggtgcaga ccgtgtcccc gcagggcaga 2340
aggctcagaa accggcgggc cacctggaaa aagagcacag cccgaggtta gaggcgacgc 2400
agcgcatgtc ccgccgacac gcgagctctg gccccggccc tgccccggga gcctgcgggt 2460
ccggtgtgct ttcagtttgt ataacgctac aaagggctgg cctaaagtgt acatagtgta 2520
aacagtgatc attacagtat ttctccttca tcaaaaatac aagaaacatc acaattttgg 2580
aattcgatat ctctgttgat tatgttttta agaactactc agaatgaaat gccttgattt 2640
ccggtgtgag ttgacaagga aaaaaattcc aggagcaggg ggagctttat ataacaccag 2700
attagcggcg aaaaattttt tttctcgagg ggttacccgg tcgtcctttg cctaatgggg 2760
aaacatcccg ctcgccgcac gtaccactta gggctcccca aaaagcaacc atcctccgct 2820
catctcgagg gagatcacta aacgatatca actttgaggg ctcttgttga ctatggactg 2880
attatagatt gaagtcgaac tagagactag ttcagcgctt tcaatcgacg aattgcaatt 2940
gacaaagaga gaacatgctc ttaagactga acatcttatc agacaagggc cgcatcgagc 3000
ggctacctcg taattgtggt cagtcattga caaccattag caagccattg gcaacgggac 3060
aacgcaggcg aatctcactc gcagttactg cctgcaatga caattgctcc tcttcgcgca 3120
aacataatag actttgataa gcaccacaat cgcttcttcc ctcggtcgta ctggcaaatt 3180
tcatgcatga atgcggctgc agcctcctgt cgatatggga cggagctctg ttgctttcgg 3240
cgccctttcc aagcttcaat ccactaatcg gagaatgttc atcaactggc tcagctaatc 3300
agataggtca acttgttccc cagcggtttc accacatgct gtcctttcct ccccccgctc 3360
ggaaggatac gcagaatgct ttatcgtggg gctgccccgg gccgaaaccg gcattttggg 3420
ttgactcttt taaggaacga catgcccagg ggactctcca ctcacaaacc ctttcaaaaa 3480
cctgtacaat gacaacgtcg ccaatcgtca aggtgtgcgg aatcaggacc gtggaggcgg 3540
cccaggtcgc tttggatgct ggagccaaca tgattggaat gattatggtc cctaatagag 3600
cgcgcactgt tgatatggag acagcaggca agatatcacg actggtacga tcatatagac 3660
gtcaaggatc ctttgagctg gacactaatg agctgggcgg atcgactcac tctgcactcg 3720
cccacatcat tgaagccaag acaaggaccc ggccagcaat cgtaggcgtg ttccgcaacc 3780
agccgctgtc agaagtcctg cggttgcaac aggaattgtc gctggatttc gttcagcttc 3840
acggatcaga gcccctagaa tggtgtagac tgattccatg tcctgtgatc cgtcggttca 3900

cccccgatac tccagatttc tatgcctcgc cggttactgg ataccacagc ctatccctca 3960
tagacggtga ggttggcgga gagggcaaga tggtggactg gagcggagtc cagtctcggg 4020
cgcgaatggg agcgaggttc cttatggctg gaggattgac agccgacaat gttgcccaag 4080
ctctagcggt cgagggtgcc tgtggtgttg atgtcagcgg aggagtcgaa tcaagcccgg 4140
gggtcaaaga tctcaacgag attgagcggt ttgtcaaagc tgccaagggg gttactgtga 4200
agtattaaca ctccagcagc agcatcaata ggtgatactg acgacaggta atgtgtacta 4260
ataggtctga cagcagaata cagctatatt acttctcatt tgaaacttga ggtgattgac 4320
aatttactat ttatacaagc taagcttcaa tacagactcg atcgaattca gtcgacacag 4380
accagacgga gatcatccta acattagcaa ttccaaatcc gccgacccgt tcgttactca 4440
ggttgatcat aaggggtcct cattccccaa gaggagaaaa cggtgtctta attgcgcacc 4500
tcttgttttt gtagcatttt tcaactaact agaccgttcg ttctatacct aattacaaca 4560
tccgcccacc tttctcctta ttgatttgca attactatcc gaatagcgcc gatattgttt 4620
ggtgccgtag atgcaatgtt attacggcag gaaaaatatg tacggataat tgttccgcga 4680
aaatccggaa tcgtaaattg tattatcgga tctgatgaat cagctatgaa agataatata 4740
tggtatagct acgactatag taaaccatag tcaaaatcaa aagtttcaac gttttcactg 4800
ttatctcatt tatccccgga ttggccaaga aactaaggtt ttttggaccc tggaacatgc 4860
ataatgcgga gatctcactc ggcccgactt ggaagtgtaa gggaaggtca gagtgaccta 4920
ggtcagagtg acctaggtca gagtgagggg atccaactcg gcccgactgg aaggtgcaat 4980
gagtgaatgg aagtagactg gaggactata taaacactcg cagcctggtt acaattgacg 5040
tggacgaaac agaattcatg cctgcaagac atcaggggct gttacgcctg tttatcgcct 5100
gcgcgctgcc gctgctggcg ctgcactcag ccgccgccgc agactggcag ctggagaaag 5160
tggtggagct tagccgccac ggcattcgtc cgccgacggc cggcaaccgg gaagccatcg 5220
aggccgccac cggacgccca tggaccgagt ggaccaccca tgacggcgag ctcaccggtc 5280
atggctatgc cgccgtggtc aacaaagggc gtgaggaagg ccagcactat cgccagctcg 5340
gcctgctgca ggcgggatgc ccgacggcgg agtcgattta cgtgcgcgcc agcccgctgc 5400
agcggacgcg ggcgaccgcc caggcgctgg tggatggcgc cttccccggc tgtggcgtcg 5460
ctatccatta cgtcaacggg gatgccgatc cgctgtttca gaccgacaag ttcgccgcca 5520
cgcaaaccga ccccgcccgc cagctggcgg cggtgaaaga gaaggccggg gatctggcgc 5580
agcgtcggca ggcgctggcg ccgactatcc agctattgaa acaggcggtt tgccaggccg 5640
ataagccctg cccgatcttt gataccccat ggcgggtcga gcagagcaaa agcggcaaga 5700
ccaccattag cggactgagc gtgatggcca atatggtgga aacgctgcgt ctcggctgga 5760
gtgaaaacct gcctctcagc cagctggcgt ggggcaagat cgcccaggcc agtcagatca 5820
cggccctgct gccgctgtta acggaaaact acgatctgag taacgacgtg ctgtataccg 5880
cgcaaaaacg cgggtcggtg ctgctcaacg ccatgctcga cggcgtcaaa ccagaggcga 5940

```
atccgaacgt acgctggctg ctgctggtgg cgcatgacac caacatcgcc atggtgcgca    6000
cgctgatgaa ctttagctgg cagctgccgg gctacagccg ggggaatatc ccgccgggca    6060
gtagcctggt gctggagcgc tggcgcgacg cgaagagcgg tgaacgctat ctgcgggtct    6120
atttccaggc gcaaggcctc gacgacctgc gtcgtctgca gacgccggac gcgcagcacc    6180
cgatgctgcg tcaggagtgg cgtcagccgg gctgccgtca gaccgacgtc ggtacgctgt    6240
gccccttcca ggcggccatc accgcgttgg gtcagcgtat cgatcggccc tccaccccgg    6300
cggtagccat ggtcctgccg taggatcctc tagggaatta gcggccgcta attccctaga    6360
gtcgagggga acactactca ttacaacgcc agtctattga gacaatagtt ttgtataact    6420
aaataatatt ggaaactaaa tacgaatacc caaatttttt atctaaattt tgccgaaaga    6480
ttaaaatctg caggcatgca agctggggcc ccatctctta ggatcgacta acccgtggcc    6540
aactgctgtt cacacggaac ctttccccac ttcagtcttc aaagttctca tttgaatatt    6600
tgctactacc accaagatct gcactagagg ccgttcgacc cagcatcact gcaaaggctt    6660
cgtcactgac ctccacgcct gcctactcgt tagggcatcg cattaaccta acggtagagt    6720
ataggtaaca cgcttgagcg ccatccattt tcagggctag ttcattcggc cggtgagttg    6780
ttacacactc cttagcggat tccgacttcc atg                                 6813
```

<210> 3
<211> 636
<212> DNA
<213> Artificial

<220>
<223> Seq ID Nr. 3: Glucoamylase-Promotor aus Arxula adeninivorans

<400> 3

```
cacagaccag acggagatca tcctaacatt agcaattcca aatccgccga cccgttcgtt      60
actcaggttg atcataaggg gtcctcattc cccaagagga gaaaacggtg tcttaattgc     120
gcacctcttg tttttgtagc atttttcaac taactagacc gttcgttcta tacctaatta     180
caacatccgc ccacctttct ccttattgat ttgcaattac tatccgaata gcgccgatat     240
tgtttggtgc cgtagatgca atgttattac ggcaggaaaa atatgtacgg ataattgttc     300
cgcgaaaatc cggaatcgta aattgtatta tcggatctga tgaatcagct atgaaagata     360
atatatggta tagctacgac tatagtaaac catagtcaaa atcaaaagtt tcaacgtttt     420
cactgttatc tcatttatcc ccggattggc caagaaacta aggtttttg gaccctggaa      480
catgcataat gcggagatct cactcggccc gacttggaag tgtaagggag gggatccaac     540
tcggcccgac tggaaggtgc aatgagtgaa tggaagtaga ctggaggact atataaacac     600
tcgcagcctg gttacaattg acgtggacga aacaga                              636
```

<210> 4
<211> 666
<212> DNA

<213> Artificial

<220>
<223> Seq ID Nr. 4: Glucoamylase-Promotor aus Arxula adeninivorans, in Position -107 sind 2 HRE inseriert.

<220>
<221> promoter
<222> (1)..(666)
<223> GAA-Promotor mit Insertion von 2 HRE in Position -107

<220>
<221> protein_bind
<222> (530)..(560)
<223> in Relation zum Transkriptionsstart bei +1 Insertion von 2 HRE unmittelbar aneinander in Position -107 des GAA-Promotors von -137 bis -107

<400> 4

```
cacagaccag acggagatca tcctaacatt agcaattcca aatccgccga cccgttcgtt     60
actcaggttg atcataaggg gtcctcattc cccaagagga gaaaacggtg tcttaattgc    120
gcacctcttg tttttgtagc atttttcaac taactagacc gttcgttcta tacctaatta    180
caacatccgc ccacctttct ccttattgat ttgcaattac tatccgaata gcgccgatat    240
tgtttggtgc cgtagatgca atgttattac ggcaggaaaa atatgtacgg ataattgttc    300
cgcgaaaatc cggaatcgta aattgtatta tcggatctga tgaatcagct atgaaagata    360
atatatggta tagctacgac tatagtaaac catagtcaaa atcaaaagtt tcaacgtttt    420
cactgttatc tcatttatcc ccggattggc caagaaacta aggtttttg gaccctggaa     480
catgcataat gcggagatct cactcggccc gacttggaag tgtaagggag gtacaggatg    540
ttctggtaca ggatgttctg gggatccaac tcggcccgac tggaaggtgc aatgagtgaa    600
tggaagtaga ctggaggact atataaacac tcgcagcctg gttacaattg acgtggacga    660
aacaga                                                               666
```

<210> 5
<211> 2802
<212> DNA
<213> Artificial

<220>
<223> Seq ID Nr. 5: Gen, das den humanen Progesteron-Rezeptor kodiert, Kodongebrauch angepasst an Arxula adeninivorans

<220>
<221> CDS
<222> (1)..(2799)
<223> kodiert die Aminosäuresequenz des natürlichen humanen Progesteron-Rezeptors

<400> 5

```
atg act gag ctg aag gct aag gga cct cga gct cct cac gtg gct gga        48
Met Thr Glu Leu Lys Ala Lys Gly Pro Arg Ala Pro His Val Ala Gly
1               5                   10                  15

gga cct cct tct cct gag gtg gga tct cct ctg ctg tgc cga cct gct        96
Gly Pro Pro Ser Pro Glu Val Gly Ser Pro Leu Leu Cys Arg Pro Ala
            20                  25                  30

gct gga cct ttc cct gga tct cag act tct gac act ctg cct gag gtg       144
Ala Gly Pro Phe Pro Gly Ser Gln Thr Ser Asp Thr Leu Pro Glu Val
```

```
        35                      40                       45

tct gct att cct att tct ctg gac gga ctg ctg ttc cct cga cct tgc      192
Ser Ala Ile Pro Ile Ser Leu Asp Gly Leu Leu Phe Pro Arg Pro Cys
    50                  55                  60

cag gga cag gac cct tct gac gag aag act cag gac cag cag tct ctg      240
Gln Gly Gln Asp Pro Ser Asp Glu Lys Thr Gln Asp Gln Gln Ser Leu
65                  70                  75                  80

tct gac gtg gag gga gct tac tct cga gct gag gct act cga gga gct      288
Ser Asp Val Glu Gly Ala Tyr Ser Arg Ala Glu Ala Thr Arg Gly Ala
                85                  90                  95

gga gga tct tct tct tct cct cct gag aag gac tct gga ctg ctg gac      336
Gly Gly Ser Ser Ser Ser Pro Pro Glu Lys Asp Ser Gly Leu Leu Asp
            100                 105                 110

tct gtg ctg gac act ctg ctg gct cct tct gga cct gga cag tct cag      384
Ser Val Leu Asp Thr Leu Leu Ala Pro Ser Gly Pro Gly Gln Ser Gln
        115                 120                 125

cct tct cct cct gct tgc gag gtg act tct tct tgg tgc ctg ttc gga      432
Pro Ser Pro Pro Ala Cys Glu Val Thr Ser Ser Trp Cys Leu Phe Gly
    130                 135                 140

cct gag ctg cct gag gac cct cct gct gct cct gct act cag cga gtg      480
Pro Glu Leu Pro Glu Asp Pro Pro Ala Ala Pro Ala Thr Gln Arg Val
145                 150                 155                 160

ctg tct cct ctg atg tct cga tct gga tgc aag gtg gga gac tct tct      528
Leu Ser Pro Leu Met Ser Arg Ser Gly Cys Lys Val Gly Asp Ser Ser
                165                 170                 175

gga act gct gct gct cac aag gtg ctg cct cga gga ctg tct cct gct      576
Gly Thr Ala Ala Ala His Lys Val Leu Pro Arg Gly Leu Ser Pro Ala
            180                 185                 190

cga cag ctg ctg ctg cct gct tct gag tct cct cac tgg tct gga gct      624
Arg Gln Leu Leu Leu Pro Ala Ser Glu Ser Pro His Trp Ser Gly Ala
        195                 200                 205

cct gtg aag cct tct cct cag gct gct gct gtg gag gtg gag gag gag      672
Pro Val Lys Pro Ser Pro Gln Ala Ala Ala Val Glu Val Glu Glu Glu
    210                 215                 220

gac gga tct gag tct gag gag tct gct gga cct ctg ctg aag gga aag      720
Asp Gly Ser Glu Ser Glu Glu Ser Ala Gly Pro Leu Leu Lys Gly Lys
225                 230                 235                 240

cct cga gct ctg gga gga gct gct gct gga gga gga gct gct gct gtg      768
Pro Arg Ala Leu Gly Gly Ala Ala Ala Gly Gly Gly Ala Ala Ala Val
                245                 250                 255

cct cct gga gct gct gct gga gga gtg gct ctg gtg cct aag gag gac      816
Pro Pro Gly Ala Ala Ala Gly Gly Val Ala Leu Val Pro Lys Glu Asp
            260                 265                 270

tct cga ttc tct gct cct cga gtg gct ctg gtg gag cag gac gct cct      864
Ser Arg Phe Ser Ala Pro Arg Val Ala Leu Val Glu Gln Asp Ala Pro
        275                 280                 285

atg gct cct gga cga tct cct ctg gct act act gtg atg gac ttc att      912
Met Ala Pro Gly Arg Ser Pro Leu Ala Thr Thr Val Met Asp Phe Ile
    290                 295                 300

cac gtg cct att ctg cct ctg aac cac gct ctg ctg gct gct cga act      960
His Val Pro Ile Leu Pro Leu Asn His Ala Leu Leu Ala Ala Arg Thr
305                 310                 315                 320
```

```
cga cag ctg ctg gag gac gag tct tac gac gga gga gct gga gct gct      1008
Arg Gln Leu Leu Glu Asp Glu Ser Tyr Asp Gly Gly Ala Gly Ala Ala
                325                 330                 335

tct gct ttc gct cct cct cga tct tct cct tgc gct tct tct act cct      1056
Ser Ala Phe Ala Pro Pro Arg Ser Ser Pro Cys Ala Ser Ser Thr Pro
            340                 345                 350

gtg gct gtg gga gac ttc cct gac tgc gct tac cct cct gac gct gag      1104
Val Ala Val Gly Asp Phe Pro Asp Cys Ala Tyr Pro Pro Asp Ala Glu
        355                 360                 365

cct aag gac gac gct tac cct ctg tac tct gac ttc cag cct cct gct      1152
Pro Lys Asp Asp Ala Tyr Pro Leu Tyr Ser Asp Phe Gln Pro Pro Ala
    370                 375                 380

ctg aag att aag gag gag gag gag gga gct gag gct tct gct cga tct      1200
Leu Lys Ile Lys Glu Glu Glu Glu Gly Ala Glu Ala Ser Ala Arg Ser
385                 390                 395                 400

cct cga tct tac ctg gtg gct gga gct aac cct gct gct ttc cct gac      1248
Pro Arg Ser Tyr Leu Val Ala Gly Ala Asn Pro Ala Ala Phe Pro Asp
                405                 410                 415

ttc cct ctg gga cct cct cct cct ctg cct cct cga gct act cct tct      1296
Phe Pro Leu Gly Pro Pro Pro Pro Leu Pro Pro Arg Ala Thr Pro Ser
            420                 425                 430

cga cct gga gag gct gct gtg act gct gct cct gct tct gct tct gtg      1344
Arg Pro Gly Glu Ala Ala Val Thr Ala Ala Pro Ala Ser Ala Ser Val
        435                 440                 445

tct tct gct tct tct tct gga tct act ctg gag tgc att ctg tac aag      1392
Ser Ser Ala Ser Ser Ser Gly Ser Thr Leu Glu Cys Ile Leu Tyr Lys
    450                 455                 460

gct gag gga gct cct cct cag cag gga cct ttc gct cct cct cct tgc      1440
Ala Glu Gly Ala Pro Pro Gln Gln Gly Pro Phe Ala Pro Pro Pro Cys
465                 470                 475                 480

aag gct cct gga gct tct gga tgc ctg ctg cct cga gac gga ctg cct      1488
Lys Ala Pro Gly Ala Ser Gly Cys Leu Leu Pro Arg Asp Gly Leu Pro
                485                 490                 495

tct act tct gct tct gct gct gct gct gga gct gct cct gct ctg tac      1536
Ser Thr Ser Ala Ser Ala Ala Ala Ala Gly Ala Ala Pro Ala Leu Tyr
            500                 505                 510

cct gct ctg gga ctg aac gga ctg cct cag ctg gga tac cag gct gct      1584
Pro Ala Leu Gly Leu Asn Gly Leu Pro Gln Leu Gly Tyr Gln Ala Ala
        515                 520                 525

gtg ctg aag gag gga ctg cct cag gtg tac cct cct tac ctg aac tac      1632
Val Leu Lys Glu Gly Leu Pro Gln Val Tyr Pro Pro Tyr Leu Asn Tyr
    530                 535                 540

ctg cga cct gac tct gag gct tct cag tct cct cag tac tct ttc gag      1680
Leu Arg Pro Asp Ser Glu Ala Ser Gln Ser Pro Gln Tyr Ser Phe Glu
545                 550                 555                 560

tct ctg cct cag aag att tgc ctg att tgc gga gac gag gct tct gga      1728
Ser Leu Pro Gln Lys Ile Cys Leu Ile Cys Gly Asp Glu Ala Ser Gly
                565                 570                 575

tgc cac tac gga gtg ctg act tgc gga tct tgc aag gtg ttc ttc aag      1776
Cys His Tyr Gly Val Leu Thr Cys Gly Ser Cys Lys Val Phe Phe Lys
            580                 585                 590
```

```
cga gct atg gag gga cag cac aac tac ctg tgc gct gga cga aac gac      1824
Arg Ala Met Glu Gly Gln His Asn Tyr Leu Cys Ala Gly Arg Asn Asp
        595                 600                 605

tgc att gtg gac aag att cga cga aag aac tgc cct gct tgc cga ctg      1872
Cys Ile Val Asp Lys Ile Arg Arg Lys Asn Cys Pro Ala Cys Arg Leu
    610                 615                 620

cga aag tgc tgc cag gct gga atg gtg ctg gga gga cga aag ttc aag      1920
Arg Lys Cys Cys Gln Ala Gly Met Val Leu Gly Gly Arg Lys Phe Lys
625                 630                 635                 640

aag ttc aac aag gtg cga gtg gtg cga gct ctg gac gct gtg gct ctg      1968
Lys Phe Asn Lys Val Arg Val Val Arg Ala Leu Asp Ala Val Ala Leu
                645                 650                 655

cct cag cct gtg gga gtg cct aac gag tct cag gct ctg tct cag cga      2016
Pro Gln Pro Val Gly Val Pro Asn Glu Ser Gln Ala Leu Ser Gln Arg
            660                 665                 670

ttc act ttc tct cct gga cag gac att cag ctg att cct cct ctg att      2064
Phe Thr Phe Ser Pro Gly Gln Asp Ile Gln Leu Ile Pro Pro Leu Ile
        675                 680                 685

aac ctg ctg atg tct att gag cct gac gtg att tac gct gga cac gac      2112
Asn Leu Leu Met Ser Ile Glu Pro Asp Val Ile Tyr Ala Gly His Asp
    690                 695                 700

aac act aag cct gac act tct tct tct ctg ctg act tct ctg aac cag      2160
Asn Thr Lys Pro Asp Thr Ser Ser Ser Leu Leu Thr Ser Leu Asn Gln
705                 710                 715                 720

ctg gga gag cga cag ctg ctg tct gtg gtg aag tgg tct aag tct ctg      2208
Leu Gly Glu Arg Gln Leu Leu Ser Val Val Lys Trp Ser Lys Ser Leu
                725                 730                 735

cct gga ttc cga aac ctg cac att gac gac cag att act ctg att cag      2256
Pro Gly Phe Arg Asn Leu His Ile Asp Asp Gln Ile Thr Leu Ile Gln
            740                 745                 750

tac tct tgg atg tct ctg atg gtg ttc gga ctg gga tgg cga tct tac      2304
Tyr Ser Trp Met Ser Leu Met Val Phe Gly Leu Gly Trp Arg Ser Tyr
        755                 760                 765

aag cac gtg tct gga cag atg ctg tac ttc gct cct gac ctg att ctg      2352
Lys His Val Ser Gly Gln Met Leu Tyr Phe Ala Pro Asp Leu Ile Leu
    770                 775                 780

aac gag cag cga atg aag gag tct tct ttc tac tct ctg tgc ctg act      2400
Asn Glu Gln Arg Met Lys Glu Ser Ser Phe Tyr Ser Leu Cys Leu Thr
785                 790                 795                 800

atg tgg cag att cct cag gag ttc gtg aag ctg cag gtg tct cag gag      2448
Met Trp Gln Ile Pro Gln Glu Phe Val Lys Leu Gln Val Ser Gln Glu
                805                 810                 815

gag ttc ctg tgc atg aag gtg ctg ctg ctg ctg aac act att cct ctg      2496
Glu Phe Leu Cys Met Lys Val Leu Leu Leu Leu Asn Thr Ile Pro Leu
            820                 825                 830

gag gga ctg cga tct cag act cag ttc gag gag atg cga tct tct tac      2544
Glu Gly Leu Arg Ser Gln Thr Gln Phe Glu Glu Met Arg Ser Ser Tyr
        835                 840                 845

att cga gag ctg att aag gct att gga ctg cga cag aag gga gtg gtg      2592
Ile Arg Glu Leu Ile Lys Ala Ile Gly Leu Arg Gln Lys Gly Val Val
    850                 855                 860

tct tct tct cag cga ttc tac cag ctg act aag ctg ctg gac aac ctg      2640
```

```
Ser Ser Ser Gln Arg Phe Tyr Gln Leu Thr Lys Leu Leu Asp Asn Leu
865                 870                 875                 880

cac gac ctg gtg aag cag ctg cac ctg tac tgc ctg aac act ttc att     2688
His Asp Leu Val Lys Gln Leu His Leu Tyr Cys Leu Asn Thr Phe Ile
                885                 890                 895

cag tct cga gct ctg tct gtg gag ttc cct gag atg atg tct gag gtg     2736
Gln Ser Arg Ala Leu Ser Val Glu Phe Pro Glu Met Met Ser Glu Val
            900                 905                 910

att gct gct cag ctg cct aag att ctg gct gga atg gtg aag cct ctg     2784
Ile Ala Ala Gln Leu Pro Lys Ile Leu Ala Gly Met Val Lys Pro Leu
        915                 920                 925

ctg ttc cac aag aag taa                                             2802
Leu Phe His Lys Lys
    930
```

<210> 6
<211> 933
<212> PRT
<213> Artificial

<220>
<223> Synthetic Construct

<400> 6

Met Thr Glu Leu Lys Ala Lys Gly Pro Arg Ala Pro His Val Ala Gly
1               5                   10                  15

Gly Pro Pro Ser Pro Glu Val Gly Ser Pro Leu Leu Cys Arg Pro Ala
            20                  25                  30

Ala Gly Pro Phe Pro Gly Ser Gln Thr Ser Asp Thr Leu Pro Glu Val
        35                  40                  45

Ser Ala Ile Pro Ile Ser Leu Asp Gly Leu Leu Phe Pro Arg Pro Cys
    50                  55                  60

Gln Gly Gln Asp Pro Ser Asp Glu Lys Thr Gln Asp Gln Gln Ser Leu
65                  70                  75                  80

Ser Asp Val Glu Gly Ala Tyr Ser Arg Ala Glu Ala Thr Arg Gly Ala
                85                  90                  95

Gly Gly Ser Ser Ser Ser Pro Pro Glu Lys Asp Ser Gly Leu Leu Asp
            100                 105                 110

Ser Val Leu Asp Thr Leu Leu Ala Pro Ser Gly Pro Gly Gln Ser Gln
        115                 120                 125

Pro Ser Pro Pro Ala Cys Glu Val Thr Ser Ser Trp Cys Leu Phe Gly
    130                 135                 140

Pro Glu Leu Pro Glu Asp Pro Pro Ala Ala Pro Ala Thr Gln Arg Val
145                 150                 155                 160

Leu Ser Pro Leu Met Ser Arg Ser Gly Cys Lys Val Gly Asp Ser Ser
165 170 175

Gly Thr Ala Ala Ala His Lys Val Leu Pro Arg Gly Leu Ser Pro Ala
180 185 190

Arg Gln Leu Leu Leu Pro Ala Ser Glu Ser Pro His Trp Ser Gly Ala
195 200 205

Pro Val Lys Pro Ser Pro Gln Ala Ala Ala Val Glu Val Glu Glu Glu
210 215 220

Asp Gly Ser Glu Ser Glu Glu Ser Ala Gly Pro Leu Leu Lys Gly Lys
225 230 235 240

Pro Arg Ala Leu Gly Gly Ala Ala Ala Gly Gly Gly Ala Ala Ala Val
245 250 255

Pro Pro Gly Ala Ala Ala Gly Gly Val Ala Leu Val Pro Lys Glu Asp
260 265 270

Ser Arg Phe Ser Ala Pro Arg Val Ala Leu Val Glu Gln Asp Ala Pro
275 280 285

Met Ala Pro Gly Arg Ser Pro Leu Ala Thr Thr Val Met Asp Phe Ile
290 295 300

His Val Pro Ile Leu Pro Leu Asn His Ala Leu Leu Ala Ala Arg Thr
305 310 315 320

Arg Gln Leu Leu Glu Asp Glu Ser Tyr Asp Gly Gly Ala Gly Ala Ala
325 330 335

Ser Ala Phe Ala Pro Pro Arg Ser Ser Pro Cys Ala Ser Ser Thr Pro
340 345 350

Val Ala Val Gly Asp Phe Pro Asp Cys Ala Tyr Pro Pro Asp Ala Glu
355 360 365

Pro Lys Asp Asp Ala Tyr Pro Leu Tyr Ser Asp Phe Gln Pro Pro Ala
370 375 380

Leu Lys Ile Lys Glu Glu Glu Glu Gly Ala Glu Ala Ser Ala Arg Ser
385 390 395 400

Pro Arg Ser Tyr Leu Val Ala Gly Ala Asn Pro Ala Ala Phe Pro Asp
405 410 415

Phe Pro Leu Gly Pro Pro Pro Pro Leu Pro Pro Arg Ala Thr Pro Ser
420 425 430

Arg Pro Gly Glu Ala Ala Val Thr Ala Ala Pro Ala Ser Ala Ser Val
435 440 445

Ser Ser Ala Ser Ser Ser Gly Ser Thr Leu Glu Cys Ile Leu Tyr Lys
450 455 460

Ala Glu Gly Ala Pro Pro Gln Gln Gly Pro Phe Ala Pro Pro Pro Cys
465 470 475 480

Lys Ala Pro Gly Ala Ser Gly Cys Leu Leu Pro Arg Asp Gly Leu Pro
485 490 495

Ser Thr Ser Ala Ser Ala Ala Ala Ala Gly Ala Ala Pro Ala Leu Tyr
500 505 510

Pro Ala Leu Gly Leu Asn Gly Leu Pro Gln Leu Gly Tyr Gln Ala Ala
515 520 525

Val Leu Lys Glu Gly Leu Pro Gln Val Tyr Pro Pro Tyr Leu Asn Tyr
530 535 540

Leu Arg Pro Asp Ser Glu Ala Ser Gln Ser Pro Gln Tyr Ser Phe Glu
545 550 555 560

Ser Leu Pro Gln Lys Ile Cys Leu Ile Cys Gly Asp Glu Ala Ser Gly
565 570 575

Cys His Tyr Gly Val Leu Thr Cys Gly Ser Cys Lys Val Phe Phe Lys
580 585 590

Arg Ala Met Glu Gly Gln His Asn Tyr Leu Cys Ala Gly Arg Asn Asp
595 600 605

Cys Ile Val Asp Lys Ile Arg Arg Lys Asn Cys Pro Ala Cys Arg Leu
610 615 620

Arg Lys Cys Cys Gln Ala Gly Met Val Leu Gly Gly Arg Lys Phe Lys
625 630 635 640

Lys Phe Asn Lys Val Arg Val Val Arg Ala Leu Asp Ala Val Ala Leu
645 650 655

Pro Gln Pro Val Gly Val Pro Asn Glu Ser Gln Ala Leu Ser Gln Arg
660 665 670

Phe Thr Phe Ser Pro Gly Gln Asp Ile Gln Leu Ile Pro Pro Leu Ile
675 680 685

Asn Leu Leu Met Ser Ile Glu Pro Asp Val Ile Tyr Ala Gly His Asp
690 695 700

Asn Thr Lys Pro Asp Thr Ser Ser Ser Leu Leu Thr Ser Leu Asn Gln

705 710 715 720

Leu Gly Glu Arg Gln Leu Leu Ser Val Val Lys Trp Ser Lys Ser Leu
725 730 735

Pro Gly Phe Arg Asn Leu His Ile Asp Asp Gln Ile Thr Leu Ile Gln
740 745 750

Tyr Ser Trp Met Ser Leu Met Val Phe Gly Leu Gly Trp Arg Ser Tyr
755 760 765

Lys His Val Ser Gly Gln Met Leu Tyr Phe Ala Pro Asp Leu Ile Leu
770 775 780

Asn Glu Gln Arg Met Lys Glu Ser Ser Phe Tyr Ser Leu Cys Leu Thr
785 790 795 800

Met Trp Gln Ile Pro Gln Glu Phe Val Lys Leu Gln Val Ser Gln Glu
805 810 815

Glu Phe Leu Cys Met Lys Val Leu Leu Leu Leu Asn Thr Ile Pro Leu
820 825 830

Glu Gly Leu Arg Ser Gln Thr Gln Phe Glu Glu Met Arg Ser Ser Tyr
835 840 845

Ile Arg Glu Leu Ile Lys Ala Ile Gly Leu Arg Gln Lys Gly Val Val
850 855 860

Ser Ser Ser Gln Arg Phe Tyr Gln Leu Thr Lys Leu Leu Asp Asn Leu
865 870 875 880

His Asp Leu Val Lys Gln Leu His Leu Tyr Cys Leu Asn Thr Phe Ile
885 890 895

Gln Ser Arg Ala Leu Ser Val Glu Phe Pro Glu Met Met Ser Glu Val
900 905 910

Ile Ala Ala Gln Leu Pro Lys Ile Leu Ala Gly Met Val Lys Pro Leu
915 920 925

Leu Phe His Lys Lys
930

<210> 7
<211> 2760
<212> DNA
<213> Artificial

<220>
<223> Seq ID Nr. 7: Gen, das den humanen Androgen-Rezeptor kodiert, Kodongebrauch angepasst an Arxula adeninivorans

<220>
<221> CDS
<222> (1)..(2757)
<223> kodiert die Aminosäuresequenz des natürlichen humanen Androgen-Rezeptors

<400> 7

```
atg gag gtg cag ctg gga ctg gga cga gtg tac cct cga cct cct tct     48
Met Glu Val Gln Leu Gly Leu Gly Arg Val Tyr Pro Arg Pro Pro Ser
1               5                   10                  15

aag act tac cga gga gct ttc cag aac ctg ttc cag tct gtg cga gag     96
Lys Thr Tyr Arg Gly Ala Phe Gln Asn Leu Phe Gln Ser Val Arg Glu
            20                  25                  30

gtg att cag aac cct gga cct cga cac cct gag gct gct tct gct gct    144
Val Ile Gln Asn Pro Gly Pro Arg His Pro Glu Ala Ala Ser Ala Ala
        35                  40                  45

cct cct gga gct tct ctg ctg ctg ctg cag cag cag cag cag cag cag    192
Pro Pro Gly Ala Ser Leu Leu Leu Leu Gln Gln Gln Gln Gln Gln Gln
    50                  55                  60

cag cag cag cag cag cag cag cag cag cag cag cag cag cag gag act    240
Gln Gln Gln Gln Gln Gln Gln Gln Gln Gln Gln Gln Gln Gln Glu Thr
65                  70                  75                  80

tct cct cga cag cag cag cag cag cag gga gag gac gga tct cct cag    288
Ser Pro Arg Gln Gln Gln Gln Gln Gln Gly Glu Asp Gly Ser Pro Gln
                85                  90                  95

gct cac cga cga gga cct act gga tac ctg gtg ctg gac gag gag cag    336
Ala His Arg Arg Gly Pro Thr Gly Tyr Leu Val Leu Asp Glu Glu Gln
            100                 105                 110

cag cct tct cag cct cag tct gct ctg gag tgc cac cct gag cga gga    384
Gln Pro Ser Gln Pro Gln Ser Ala Leu Glu Cys His Pro Glu Arg Gly
        115                 120                 125

tgc gtg cct gag cct gga gct gct gtg gct gct tct aag gga ctg cct    432
Cys Val Pro Glu Pro Gly Ala Ala Val Ala Ala Ser Lys Gly Leu Pro
    130                 135                 140

cag cag ctg cct gct cct cct gac gag gac gac tct gct gct cct tct    480
Gln Gln Leu Pro Ala Pro Pro Asp Glu Asp Asp Ser Ala Ala Pro Ser
145                 150                 155                 160

act ctg tct ctg ctg gga cct act ttc cct gga ctg tct tct tgc tct    528
Thr Leu Ser Leu Leu Gly Pro Thr Phe Pro Gly Leu Ser Ser Cys Ser
                165                 170                 175

gct gac ctg aag gac att ctg tct gag gct tct act atg cag ctg ctg    576
Ala Asp Leu Lys Asp Ile Leu Ser Glu Ala Ser Thr Met Gln Leu Leu
            180                 185                 190

cag cag cag cag cag gag gct gtg tct gag gga tct tct tct gga cga    624
Gln Gln Gln Gln Gln Glu Ala Val Ser Glu Gly Ser Ser Ser Gly Arg
        195                 200                 205

gct cga gag gct tct gga gct cct act tct tct aag gac aac tac ctg    672
Ala Arg Glu Ala Ser Gly Ala Pro Thr Ser Ser Lys Asp Asn Tyr Leu
    210                 215                 220

gga gga act tct act att tct gac aac gct aag gag ctg tgc aag gct    720
Gly Gly Thr Ser Thr Ile Ser Asp Asn Ala Lys Glu Leu Cys Lys Ala
225                 230                 235                 240

gtg tct gtg tct atg gga ctg gga gtg gag gct ctg gag cac ctg tct    768
Val Ser Val Ser Met Gly Leu Gly Val Glu Ala Leu Glu His Leu Ser
```

```
                245                     250                     255

cct gga gag cag ctg cga gga gac tgc atg tac gct cct ctg ctg gga          816
Pro Gly Glu Gln Leu Arg Gly Asp Cys Met Tyr Ala Pro Leu Leu Gly
            260                     265                     270

gtg cct cct gct gtg cga cct act cct tgc gct cct ctg gct gag tgc          864
Val Pro Pro Ala Val Arg Pro Thr Pro Cys Ala Pro Leu Ala Glu Cys
        275                     280                     285

aag gga tct ctg ctg gac gac tct gct gga aag tct act gag gac act          912
Lys Gly Ser Leu Leu Asp Asp Ser Ala Gly Lys Ser Thr Glu Asp Thr
    290                     295                     300

gct gag tac tct cct ttc aag gga gga tac act aag gga ctg gag gga          960
Ala Glu Tyr Ser Pro Phe Lys Gly Gly Tyr Thr Lys Gly Leu Glu Gly
305                     310                     315                     320

gag tct ctg gga tgc tct gga tct gct gct gct gga tct tct gga act         1008
Glu Ser Leu Gly Cys Ser Gly Ser Ala Ala Ala Gly Ser Ser Gly Thr
                325                     330                     335

ctg gag ctg cct tct act ctg tct ctg tac aag tct gga gct ctg gac         1056
Leu Glu Leu Pro Ser Thr Leu Ser Leu Tyr Lys Ser Gly Ala Leu Asp
            340                     345                     350

gag gct gct gct tac cag tct cga gac tac tac aac ttc cct ctg gct         1104
Glu Ala Ala Ala Tyr Gln Ser Arg Asp Tyr Tyr Asn Phe Pro Leu Ala
        355                     360                     365

ctg gct gga cct cct cct cct cct cct cct cct cac cct cac gct cga         1152
Leu Ala Gly Pro Pro Pro Pro Pro Pro Pro Pro His Pro His Ala Arg
    370                     375                     380

att aag ctg gag aac cct ctg gac tac gga tct gct tgg gct gct gct         1200
Ile Lys Leu Glu Asn Pro Leu Asp Tyr Gly Ser Ala Trp Ala Ala Ala
385                     390                     395                     400

gct gct cag tgc cga tac gga gac ctg gct tct ctg cac gga gct gga         1248
Ala Ala Gln Cys Arg Tyr Gly Asp Leu Ala Ser Leu His Gly Ala Gly
                405                     410                     415

gct gct gga cct gga tct gga tct cct tct gct gct gct tct tct tct         1296
Ala Ala Gly Pro Gly Ser Gly Ser Pro Ser Ala Ala Ala Ser Ser Ser
            420                     425                     430

tgg cac act ctg ttc act gct gag gag gga cag ctg tac gga cct tgc         1344
Trp His Thr Leu Phe Thr Ala Glu Glu Gly Gln Leu Tyr Gly Pro Cys
        435                     440                     445

gga gga gga gga gga gga gga gga gga gga gga gga gga gga gga gga         1392
Gly Gly Gly Gly Gly Gly Gly Gly Gly Gly Gly Gly Gly Gly Gly Gly
    450                     455                     460

gga gga gga gga gga gga gga gga gag gct gga gct gtg gct cct tac         1440
Gly Gly Gly Gly Gly Gly Gly Gly Glu Ala Gly Ala Val Ala Pro Tyr
465                     470                     475                     480

gga tac act cga cct cct cag gga ctg gct gga cag gag tct gac ttc         1488
Gly Tyr Thr Arg Pro Pro Gln Gly Leu Ala Gly Gln Glu Ser Asp Phe
                485                     490                     495

act gct cct gac gtg tgg tac cct gga gga atg gtg tct cga gtg cct         1536
Thr Ala Pro Asp Val Trp Tyr Pro Gly Gly Met Val Ser Arg Val Pro
            500                     505                     510

tac cct tct cct act tgc gtg aag tct gag atg gga cct tgg atg gac         1584
Tyr Pro Ser Pro Thr Cys Val Lys Ser Glu Met Gly Pro Trp Met Asp
        515                     520                     525
```

```
tct tac tct gga cct tac gga gac atg cga ctg gag act gct cga gac      1632
Ser Tyr Ser Gly Pro Tyr Gly Asp Met Arg Leu Glu Thr Ala Arg Asp
    530                 535                 540

cac gtg ctg cct att gac tac tac ttc cct cct cag aag act tgc ctg      1680
His Val Leu Pro Ile Asp Tyr Tyr Phe Pro Pro Gln Lys Thr Cys Leu
545                 550                 555                 560

att tgc gga gac gag gct tct gga tgc cac tac gga gct ctg act tgc      1728
Ile Cys Gly Asp Glu Ala Ser Gly Cys His Tyr Gly Ala Leu Thr Cys
                565                 570                 575

gga tct tgc aag gtg ttc ttc aag cga gct gct gag gga aag cag aag      1776
Gly Ser Cys Lys Val Phe Phe Lys Arg Ala Ala Glu Gly Lys Gln Lys
            580                 585                 590

tac ctg tgc gct tct cga aac gac tgc act att gac aag ttc cga cga      1824
Tyr Leu Cys Ala Ser Arg Asn Asp Cys Thr Ile Asp Lys Phe Arg Arg
        595                 600                 605

aag aac tgc cct tct tgc cga ctg cga aag tgc tac gag gct gga atg      1872
Lys Asn Cys Pro Ser Cys Arg Leu Arg Lys Cys Tyr Glu Ala Gly Met
    610                 615                 620

act ctg gga gct cga aag ctg aag aag ctg gga aac ctg aag ctg cag      1920
Thr Leu Gly Ala Arg Lys Leu Lys Lys Leu Gly Asn Leu Lys Leu Gln
625                 630                 635                 640

gag gag gga gag gct tct tct act act tct cct act gag gag act act      1968
Glu Glu Gly Glu Ala Ser Ser Thr Thr Ser Pro Thr Glu Glu Thr Thr
                645                 650                 655

cag aag ctg act gtg tct cac att gag gga tac gag tgc cag cct att      2016
Gln Lys Leu Thr Val Ser His Ile Glu Gly Tyr Glu Cys Gln Pro Ile
            660                 665                 670

ttc ctg aac gtg ctg gag gct att gag cct gga gtg gtg tgc gct gga      2064
Phe Leu Asn Val Leu Glu Ala Ile Glu Pro Gly Val Val Cys Ala Gly
        675                 680                 685

cac gac aac aac cag cct gac tct ttc gct gct ctg ctg tct tct ctg      2112
His Asp Asn Asn Gln Pro Asp Ser Phe Ala Ala Leu Leu Ser Ser Leu
    690                 695                 700

aac gag ctg gga gag cga cag ctg gtg cac gtg gtg aag tgg gct aag      2160
Asn Glu Leu Gly Glu Arg Gln Leu Val His Val Val Lys Trp Ala Lys
705                 710                 715                 720

gct ctg cct gga ttc cga aac ctg cac gtg gac gac cag atg gct gtg      2208
Ala Leu Pro Gly Phe Arg Asn Leu His Val Asp Asp Gln Met Ala Val
                725                 730                 735

att cag tac tct tgg atg gga ctg atg gtg ttc gct atg gga tgg cga      2256
Ile Gln Tyr Ser Trp Met Gly Leu Met Val Phe Ala Met Gly Trp Arg
            740                 745                 750

tct ttc act aac gtg aac tct cga atg ctg tac ttc gct cct gac ctg      2304
Ser Phe Thr Asn Val Asn Ser Arg Met Leu Tyr Phe Ala Pro Asp Leu
        755                 760                 765

gtg ttc aac gag tac cga atg cac aag tct cga atg tac tct cag tgc      2352
Val Phe Asn Glu Tyr Arg Met His Lys Ser Arg Met Tyr Ser Gln Cys
    770                 775                 780

gtg cga atg cga cac ctg tct cag gag ttc gga tgg ctg cag att act      2400
Val Arg Met Arg His Leu Ser Gln Glu Phe Gly Trp Leu Gln Ile Thr
785                 790                 795                 800
```

```
cct cag gag ttc ctg tgc atg aag gct ctg ctg ctg ttc tct att att      2448
Pro Gln Glu Phe Leu Cys Met Lys Ala Leu Leu Leu Phe Ser Ile Ile
                805                 810                 815

cct gtg gac gga ctg aag aac cag aag ttc ttc gac gag ctg cga atg      2496
Pro Val Asp Gly Leu Lys Asn Gln Lys Phe Phe Asp Glu Leu Arg Met
            820                 825                 830

aac tac att aag gag ctg gac cga att att gct tgc aag cga aag aac      2544
Asn Tyr Ile Lys Glu Leu Asp Arg Ile Ile Ala Cys Lys Arg Lys Asn
        835                 840                 845

cct act tct tgc tct cga cga ttc tac cag ctg act aag ctg ctg gac      2592
Pro Thr Ser Cys Ser Arg Arg Phe Tyr Gln Leu Thr Lys Leu Leu Asp
    850                 855                 860

tct gtg cag cct att gct cga gag ctg cac cag ttc act ttc gac ctg      2640
Ser Val Gln Pro Ile Ala Arg Glu Leu His Gln Phe Thr Phe Asp Leu
865                 870                 875                 880

ctg att aag tct cac atg gtg tct gtg gac ttc cct gag atg atg gct      2688
Leu Ile Lys Ser His Met Val Ser Val Asp Phe Pro Glu Met Met Ala
                885                 890                 895

gag att att tct gtg cag gtg cct aag att ctg tct gga aag gtg aag      2736
Glu Ile Ile Ser Val Gln Val Pro Lys Ile Leu Ser Gly Lys Val Lys
            900                 905                 910

cct att tac ttc cac act cag taa                                      2760
Pro Ile Tyr Phe His Thr Gln
        915
```

<210> 8
<211> 919
<212> PRT
<213> Artificial

<220>
<223> Synthetic Construct

<400> 8

```
Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Gln Gln Gln Gln Glu Thr
65                  70                  75                  80

Ser Pro Arg Gln Gln Gln Gln Gln Gln Gly Glu Asp Gly Ser Pro Gln
                85                  90                  95

Ala His Arg Arg Gly Pro Thr Gly Tyr Leu Val Leu Asp Glu Glu Gln
            100                 105                 110
```

Gln Pro Ser Gln Pro Gln Ser Ala Leu Glu Cys His Pro Glu Arg Gly
115 120 125

Cys Val Pro Glu Pro Gly Ala Ala Val Ala Ala Ser Lys Gly Leu Pro
130 135 140

Gln Gln Leu Pro Ala Pro Pro Asp Glu Asp Asp Ser Ala Ala Pro Ser
145 150 155 160

Thr Leu Ser Leu Leu Gly Pro Thr Phe Pro Gly Leu Ser Ser Cys Ser
165 170 175

Ala Asp Leu Lys Asp Ile Leu Ser Glu Ala Ser Thr Met Gln Leu Leu
180 185 190

Gln Gln Gln Gln Gln Glu Ala Val Ser Glu Gly Ser Ser Ser Gly Arg
195 200 205

Ala Arg Glu Ala Ser Gly Ala Pro Thr Ser Ser Lys Asp Asn Tyr Leu
210 215 220

Gly Gly Thr Ser Thr Ile Ser Asp Asn Ala Lys Glu Leu Cys Lys Ala
225 230 235 240

Val Ser Val Ser Met Gly Leu Gly Val Glu Ala Leu Glu His Leu Ser
245 250 255

Pro Gly Glu Gln Leu Arg Gly Asp Cys Met Tyr Ala Pro Leu Leu Gly
260 265 270

Val Pro Pro Ala Val Arg Pro Thr Pro Cys Ala Pro Leu Ala Glu Cys
275 280 285

Lys Gly Ser Leu Leu Asp Asp Ser Ala Gly Lys Ser Thr Glu Asp Thr
290 295 300

Ala Glu Tyr Ser Pro Phe Lys Gly Gly Tyr Thr Lys Gly Leu Glu Gly
305 310 315 320

Glu Ser Leu Gly Cys Ser Gly Ser Ala Ala Ala Gly Ser Ser Gly Thr
325 330 335

Leu Glu Leu Pro Ser Thr Leu Ser Leu Tyr Lys Ser Gly Ala Leu Asp
340 345 350

Glu Ala Ala Ala Tyr Gln Ser Arg Asp Tyr Tyr Asn Phe Pro Leu Ala
355 360 365

Leu Ala Gly Pro Pro Pro Pro Pro Pro Pro Pro His Pro His Ala Arg
370 375 380

Ile Lys Leu Glu Asn Pro Leu Asp Tyr Gly Ser Ala Trp Ala Ala Ala
385 390 395 400

Ala Ala Gln Cys Arg Tyr Gly Asp Leu Ala Ser Leu His Gly Ala Gly
405 410 415

Ala Ala Gly Pro Gly Ser Gly Ser Pro Ser Ala Ala Ala Ser Ser Ser
420 425 430

Trp His Thr Leu Phe Thr Ala Glu Glu Gly Gln Leu Tyr Gly Pro Cys
435 440 445

Gly Gly Gly Gly Gly Gly Gly Gly Gly Gly Gly Gly Gly Gly Gly Gly
450 455 460

Gly Gly Gly Gly Gly Gly Gly Gly Glu Ala Gly Ala Val Ala Pro Tyr
465 470 475 480

Gly Tyr Thr Arg Pro Pro Gln Gly Leu Ala Gly Gln Glu Ser Asp Phe
485 490 495

Thr Ala Pro Asp Val Trp Tyr Pro Gly Gly Met Val Ser Arg Val Pro
500 505 510

Tyr Pro Ser Pro Thr Cys Val Lys Ser Glu Met Gly Pro Trp Met Asp
515 520 525

Ser Tyr Ser Gly Pro Tyr Gly Asp Met Arg Leu Glu Thr Ala Arg Asp
530 535 540

His Val Leu Pro Ile Asp Tyr Tyr Phe Pro Pro Gln Lys Thr Cys Leu
545 550 555 560

Ile Cys Gly Asp Glu Ala Ser Gly Cys His Tyr Gly Ala Leu Thr Cys
565 570 575

Gly Ser Cys Lys Val Phe Phe Lys Arg Ala Ala Glu Gly Lys Gln Lys
580 585 590

Tyr Leu Cys Ala Ser Arg Asn Asp Cys Thr Ile Asp Lys Phe Arg Arg
595 600 605

Lys Asn Cys Pro Ser Cys Arg Leu Arg Lys Cys Tyr Glu Ala Gly Met
610 615 620

Thr Leu Gly Ala Arg Lys Leu Lys Lys Leu Gly Asn Leu Lys Leu Gln
625 630 635 640

Glu Glu Gly Glu Ala Ser Ser Thr Thr Ser Pro Thr Glu Glu Thr Thr
645 650 655

Gln Lys Leu Thr Val Ser His Ile Glu Gly Tyr Glu Cys Gln Pro Ile
660 665 670

Phe Leu Asn Val Leu Glu Ala Ile Glu Pro Gly Val Val Cys Ala Gly
675 680 685

His Asp Asn Asn Gln Pro Asp Ser Phe Ala Ala Leu Leu Ser Ser Leu
690 695 700

Asn Glu Leu Gly Glu Arg Gln Leu Val His Val Val Lys Trp Ala Lys
705 710 715 720

Ala Leu Pro Gly Phe Arg Asn Leu His Val Asp Asp Gln Met Ala Val
725 730 735

Ile Gln Tyr Ser Trp Met Gly Leu Met Val Phe Ala Met Gly Trp Arg
740 745 750

Ser Phe Thr Asn Val Asn Ser Arg Met Leu Tyr Phe Ala Pro Asp Leu
755 760 765

Val Phe Asn Glu Tyr Arg Met His Lys Ser Arg Met Tyr Ser Gln Cys
770 775 780

Val Arg Met Arg His Leu Ser Gln Glu Phe Gly Trp Leu Gln Ile Thr
785 790 795 800

Pro Gln Glu Phe Leu Cys Met Lys Ala Leu Leu Leu Phe Ser Ile Ile
805 810 815

Pro Val Asp Gly Leu Lys Asn Gln Lys Phe Phe Asp Glu Leu Arg Met
820 825 830

Asn Tyr Ile Lys Glu Leu Asp Arg Ile Ile Ala Cys Lys Arg Lys Asn
835 840 845

Pro Thr Ser Cys Ser Arg Arg Phe Tyr Gln Leu Thr Lys Leu Leu Asp
850 855 860

Ser Val Gln Pro Ile Ala Arg Glu Leu His Gln Phe Thr Phe Asp Leu
865 870 875 880

Leu Ile Lys Ser His Met Val Ser Val Asp Phe Pro Glu Met Met Ala
885 890 895

Glu Ile Ile Ser Val Gln Val Pro Lys Ile Leu Ser Gly Lys Val Lys
900 905 910

Pro Ile Tyr Phe His Thr Gln
915

<210> 9

<211> 2334
<212> DNA
<213> Artificial

<220>
<223> seq ID Nr. 9: Gen, das den humanen Glucocorticoid-Rezeptor kodiert, Kodongebrauch angepasst an Arxula adeninivorans

<220>
<221> CDS
<222> (1)..(2331)
<223> kodiert die Aminosäuresequenz des natürlichen humanen Glucocorticoid-Rezeptors

<400> 9

```
atg gac tct aag gag tct ctg act cct gga cga gag gag aac cct tct      48
Met Asp Ser Lys Glu Ser Leu Thr Pro Gly Arg Glu Glu Asn Pro Ser
1               5                   10                  15

tct gtg ctg gct cag gag cga gga gac gtg atg gac ttc tac aag act      96
Ser Val Leu Ala Gln Glu Arg Gly Asp Val Met Asp Phe Tyr Lys Thr
            20                  25                  30

ctg cga gga gga gct act gtg aag gtg tct gct tct tct cct tct ctg     144
Leu Arg Gly Gly Ala Thr Val Lys Val Ser Ala Ser Ser Pro Ser Leu
        35                  40                  45

gct gtg gct tct cag tct gac tct aag cag cga cga ctg ctg gtg gac     192
Ala Val Ala Ser Gln Ser Asp Ser Lys Gln Arg Arg Leu Leu Val Asp
    50                  55                  60

ttc cct aag gga tct gtg tct aac gct cag cag cct gac ctg tct aag     240
Phe Pro Lys Gly Ser Val Ser Asn Ala Gln Gln Pro Asp Leu Ser Lys
65                  70                  75                  80

gct gtg tct ctg tct atg gga ctg tac atg gga gag act gag act aag     288
Ala Val Ser Leu Ser Met Gly Leu Tyr Met Gly Glu Thr Glu Thr Lys
                85                  90                  95

gtg atg gga aac gac ctg gga ttc cct cag cag gga cag att tct ctg     336
Val Met Gly Asn Asp Leu Gly Phe Pro Gln Gln Gly Gln Ile Ser Leu
            100                 105                 110

tct tct gga gag act gac ctg aag ctg ctg gag gag tct att gct aac     384
Ser Ser Gly Glu Thr Asp Leu Lys Leu Leu Glu Glu Ser Ile Ala Asn
        115                 120                 125

ctg aac cga tct act tct gtg cct gag aac cct aag tct tct gct tct     432
Leu Asn Arg Ser Thr Ser Val Pro Glu Asn Pro Lys Ser Ser Ala Ser
    130                 135                 140

act gct gtg tct gct gct cct act gag aag gag ttc cct aag act cac     480
Thr Ala Val Ser Ala Ala Pro Thr Glu Lys Glu Phe Pro Lys Thr His
145                 150                 155                 160

tct gac gtg tct tct gag cag cag cac ctg aag gga cag act gga act     528
Ser Asp Val Ser Ser Glu Gln Gln His Leu Lys Gly Gln Thr Gly Thr
                165                 170                 175

aac gga gga aac gtg aag ctg tac act act gac cag tct act ttc gac     576
Asn Gly Gly Asn Val Lys Leu Tyr Thr Thr Asp Gln Ser Thr Phe Asp
            180                 185                 190

att ctg cag gac ctg gag ttc tct tct gga tct cct gga aag gag act     624
Ile Leu Gln Asp Leu Glu Phe Ser Ser Gly Ser Pro Gly Lys Glu Thr
        195                 200                 205

aac gag tct cct tgg cga tct gac ctg ctg att gac gag aac tgc ctg     672
```

```
Asn Glu Ser Pro Trp Arg Ser Asp Leu Leu Ile Asp Glu Asn Cys Leu
    210                 215                 220

ctg tct cct ctg gct gga gag gac gac tct ttc ctg ctg gag gga aac      720
Leu Ser Pro Leu Ala Gly Glu Asp Asp Ser Phe Leu Leu Glu Gly Asn
225                 230                 235                 240

tct aac gag gac tgc aag cct ctg att ctg cct gac act aag cct aag      768
Ser Asn Glu Asp Cys Lys Pro Leu Ile Leu Pro Asp Thr Lys Pro Lys
                245                 250                 255

att aag gac aac gga gac ctg gtg ctg tct tct cct tct aac gtg act      816
Ile Lys Asp Asn Gly Asp Leu Val Leu Ser Ser Pro Ser Asn Val Thr
            260                 265                 270

ctg cct cag gtg aag act gag aag gag gac ttc att gag ctg tgc act      864
Leu Pro Gln Val Lys Thr Glu Lys Glu Asp Phe Ile Glu Leu Cys Thr
        275                 280                 285

cct gga gtg att aag cag gag aag ctg gga act gtg tac tgc cag gct      912
Pro Gly Val Ile Lys Gln Glu Lys Leu Gly Thr Val Tyr Cys Gln Ala
    290                 295                 300

tct ttc cct gga gct aac att att gga aac aag atg tct gct att tct      960
Ser Phe Pro Gly Ala Asn Ile Ile Gly Asn Lys Met Ser Ala Ile Ser
305                 310                 315                 320

gtg cac gga gtg tct act tct gga gga cag atg tac cac tac gac atg     1008
Val His Gly Val Ser Thr Ser Gly Gly Gln Met Tyr His Tyr Asp Met
                325                 330                 335

aac act gct tct ctg tct cag cag cag gac cag aag cct att ttc aac     1056
Asn Thr Ala Ser Leu Ser Gln Gln Gln Asp Gln Lys Pro Ile Phe Asn
            340                 345                 350

gtg att cct cct att cct gtg gga tct gag aac tgg aac cga tgc cag     1104
Val Ile Pro Pro Ile Pro Val Gly Ser Glu Asn Trp Asn Arg Cys Gln
        355                 360                 365

gga tct gga gac gac aac ctg act tct ctg gga act ctg aac ttc cct     1152
Gly Ser Gly Asp Asp Asn Leu Thr Ser Leu Gly Thr Leu Asn Phe Pro
    370                 375                 380

gga cga act gtg ttc tct aac gga tac tct tct cct tct atg cga cct     1200
Gly Arg Thr Val Phe Ser Asn Gly Tyr Ser Ser Pro Ser Met Arg Pro
385                 390                 395                 400

gac gtg tct tct cct cct tct tct tct tct act gct act act gga cct     1248
Asp Val Ser Ser Pro Pro Ser Ser Ser Ser Thr Ala Thr Thr Gly Pro
                405                 410                 415

cct cct aag ctg tgc ctg gtg tgc tct gac gag gct tct gga tgc cac     1296
Pro Pro Lys Leu Cys Leu Val Cys Ser Asp Glu Ala Ser Gly Cys His
            420                 425                 430

tac gga gtg ctg act tgc gga tct tgc aag gtg ttc ttc aag cga gct     1344
Tyr Gly Val Leu Thr Cys Gly Ser Cys Lys Val Phe Phe Lys Arg Ala
        435                 440                 445

gtg gag gga cag cac aac tac ctg tgc gct gga cga aac gac tgc att     1392
Val Glu Gly Gln His Asn Tyr Leu Cys Ala Gly Arg Asn Asp Cys Ile
    450                 455                 460

att gac aag att cga cga aag aac tgc cct gct tgc cga tac cga aag     1440
Ile Asp Lys Ile Arg Arg Lys Asn Cys Pro Ala Cys Arg Tyr Arg Lys
465                 470                 475                 480

tgc ctg cag gct gga atg aac ctg gag gct cga aag act aag aag aag     1488
Cys Leu Gln Ala Gly Met Asn Leu Glu Ala Arg Lys Thr Lys Lys Lys
```

```
                485                     490                     495

att aag gga att cag cag gct act act gga gtg tct cag gag act tct      1536
Ile Lys Gly Ile Gln Gln Ala Thr Thr Gly Val Ser Gln Glu Thr Ser
            500                 505                 510

gag aac cct gga aac aag act att gtg cct gct act ctg cct cag ctg      1584
Glu Asn Pro Gly Asn Lys Thr Ile Val Pro Ala Thr Leu Pro Gln Leu
        515                 520                 525

act cct act ctg gtg tct ctg ctg gag gtg att gag cct gag gtg ctg      1632
Thr Pro Thr Leu Val Ser Leu Leu Glu Val Ile Glu Pro Glu Val Leu
    530                 535                 540

tac gct gga tac gac tct tct gtg cct gac tct act tgg cga att atg      1680
Tyr Ala Gly Tyr Asp Ser Ser Val Pro Asp Ser Thr Trp Arg Ile Met
545                 550                 555                 560

act act ctg aac atg ctg gga gga cga cag gtg att gct gct gtg aag      1728
Thr Thr Leu Asn Met Leu Gly Gly Arg Gln Val Ile Ala Ala Val Lys
                565                 570                 575

tgg gct aag gct att cct gga ttc cga aac ctg cac ctg gac gac cag      1776
Trp Ala Lys Ala Ile Pro Gly Phe Arg Asn Leu His Leu Asp Asp Gln
            580                 585                 590

atg act ctg ctg cag tac tct tgg atg ttc ctg atg gct ttc gct ctg      1824
Met Thr Leu Leu Gln Tyr Ser Trp Met Phe Leu Met Ala Phe Ala Leu
        595                 600                 605

gga tgg cga tct tac cga cag tct tct gct aac ctg ctg tgc ttc gct      1872
Gly Trp Arg Ser Tyr Arg Gln Ser Ser Ala Asn Leu Leu Cys Phe Ala
    610                 615                 620

cct gac ctg att att aac gag cag cga atg act ctg cct tgc atg tac      1920
Pro Asp Leu Ile Ile Asn Glu Gln Arg Met Thr Leu Pro Cys Met Tyr
625                 630                 635                 640

gac cag tgc aag cac atg ctg tac gtg tct tct gag ctg cac cga ctg      1968
Asp Gln Cys Lys His Met Leu Tyr Val Ser Ser Glu Leu His Arg Leu
                645                 650                 655

cag gtg tct tac gag gag tac ctg tgc atg aag act ctg ctg ctg ctg      2016
Gln Val Ser Tyr Glu Glu Tyr Leu Cys Met Lys Thr Leu Leu Leu Leu
            660                 665                 670

tct tct gtg cct aag gac gga ctg aag tct cag gag ctg ttc gac gag      2064
Ser Ser Val Pro Lys Asp Gly Leu Lys Ser Gln Glu Leu Phe Asp Glu
        675                 680                 685

att cga atg act tac att aag gag ctg gga aag gct att gtg aag cga      2112
Ile Arg Met Thr Tyr Ile Lys Glu Leu Gly Lys Ala Ile Val Lys Arg
    690                 695                 700

gag gga aac tct tct cag aac tgg cag cga ttc tac cag ctg act aag      2160
Glu Gly Asn Ser Ser Gln Asn Trp Gln Arg Phe Tyr Gln Leu Thr Lys
705                 710                 715                 720

ctg ctg gac tct atg cac gag gtg gtg gag aac ctg ctg aac tac tgc      2208
Leu Leu Asp Ser Met His Glu Val Val Glu Asn Leu Leu Asn Tyr Cys
                725                 730                 735

ttc cag act ttc ctg gac aag act atg tct att gag ttc cct gag atg      2256
Phe Gln Thr Phe Leu Asp Lys Thr Met Ser Ile Glu Phe Pro Glu Met
            740                 745                 750

ctg gct gag att att act aac cag att cct aag tac tct aac gga aac      2304
Leu Ala Glu Ile Ile Thr Asn Gln Ile Pro Lys Tyr Ser Asn Gly Asn
        755                 760                 765
```

```
att aag aag ctg ctg ttc cac cag aag taa                                   2334
Ile Lys Lys Leu Leu Phe His Gln Lys
    770                 775
```

<210> 10
<211> 777
<212> PRT
<213> Artificial

<220>
<223> Synthetic Construct

<400> 10

```
Met Asp Ser Lys Glu Ser Leu Thr Pro Gly Arg Glu Glu Asn Pro Ser
1               5                   10                  15

Ser Val Leu Ala Gln Glu Arg Gly Asp Val Met Asp Phe Tyr Lys Thr
            20                  25                  30

Leu Arg Gly Gly Ala Thr Val Lys Val Ser Ala Ser Ser Pro Ser Leu
        35                  40                  45

Ala Val Ala Ser Gln Ser Asp Ser Lys Gln Arg Arg Leu Leu Val Asp
    50                  55                  60

Phe Pro Lys Gly Ser Val Ser Asn Ala Gln Gln Pro Asp Leu Ser Lys
65                  70                  75                  80

Ala Val Ser Leu Ser Met Gly Leu Tyr Met Gly Glu Thr Glu Thr Lys
                85                  90                  95

Val Met Gly Asn Asp Leu Gly Phe Pro Gln Gln Gly Gln Ile Ser Leu
            100                 105                 110

Ser Ser Gly Glu Thr Asp Leu Lys Leu Leu Glu Glu Ser Ile Ala Asn
        115                 120                 125

Leu Asn Arg Ser Thr Ser Val Pro Glu Asn Pro Lys Ser Ser Ala Ser
    130                 135                 140

Thr Ala Val Ser Ala Ala Pro Thr Glu Lys Glu Phe Pro Lys Thr His
145                 150                 155                 160

Ser Asp Val Ser Ser Glu Gln Gln His Leu Lys Gly Gln Thr Gly Thr
                165                 170                 175

Asn Gly Gly Asn Val Lys Leu Tyr Thr Thr Asp Gln Ser Thr Phe Asp
            180                 185                 190

Ile Leu Gln Asp Leu Glu Phe Ser Ser Gly Ser Pro Gly Lys Glu Thr
        195                 200                 205

Asn Glu Ser Pro Trp Arg Ser Asp Leu Leu Ile Asp Glu Asn Cys Leu
```

```
        210                 215                 220

Leu Ser Pro Leu Ala Gly Glu Asp Asp Ser Phe Leu Leu Glu Gly Asn
225                 230                 235                 240

Ser Asn Glu Asp Cys Lys Pro Leu Ile Leu Pro Asp Thr Lys Pro Lys
                245                 250                 255

Ile Lys Asp Asn Gly Asp Leu Val Leu Ser Ser Pro Ser Asn Val Thr
            260                 265                 270

Leu Pro Gln Val Lys Thr Glu Lys Glu Asp Phe Ile Glu Leu Cys Thr
        275                 280                 285

Pro Gly Val Ile Lys Gln Glu Lys Leu Gly Thr Val Tyr Cys Gln Ala
    290                 295                 300

Ser Phe Pro Gly Ala Asn Ile Ile Gly Asn Lys Met Ser Ala Ile Ser
305                 310                 315                 320

Val His Gly Val Ser Thr Ser Gly Gly Gln Met Tyr His Tyr Asp Met
                325                 330                 335

Asn Thr Ala Ser Leu Ser Gln Gln Gln Asp Gln Lys Pro Ile Phe Asn
            340                 345                 350

Val Ile Pro Pro Ile Pro Val Gly Ser Glu Asn Trp Asn Arg Cys Gln
        355                 360                 365

Gly Ser Gly Asp Asp Asn Leu Thr Ser Leu Gly Thr Leu Asn Phe Pro
    370                 375                 380

Gly Arg Thr Val Phe Ser Asn Gly Tyr Ser Ser Pro Ser Met Arg Pro
385                 390                 395                 400

Asp Val Ser Ser Pro Pro Ser Ser Ser Ser Thr Ala Thr Thr Gly Pro
                405                 410                 415

Pro Pro Lys Leu Cys Leu Val Cys Ser Asp Glu Ala Ser Gly Cys His
            420                 425                 430

Tyr Gly Val Leu Thr Cys Gly Ser Cys Lys Val Phe Phe Lys Arg Ala
        435                 440                 445

Val Glu Gly Gln His Asn Tyr Leu Cys Ala Gly Arg Asn Asp Cys Ile
    450                 455                 460

Ile Asp Lys Ile Arg Arg Lys Asn Cys Pro Ala Cys Arg Tyr Arg Lys
465                 470                 475                 480

Cys Leu Gln Ala Gly Met Asn Leu Glu Ala Arg Lys Thr Lys Lys Lys
                485                 490                 495
```

Ile Lys Gly Ile Gln Gln Ala Thr Thr Gly Val Ser Gln Glu Thr Ser
500 505 510

Glu Asn Pro Gly Asn Lys Thr Ile Val Pro Ala Thr Leu Pro Gln Leu
515 520 525

Thr Pro Thr Leu Val Ser Leu Leu Glu Val Ile Glu Pro Glu Val Leu
530 535 540

Tyr Ala Gly Tyr Asp Ser Ser Val Pro Asp Ser Thr Trp Arg Ile Met
545 550 555 560

Thr Thr Leu Asn Met Leu Gly Gly Arg Gln Val Ile Ala Ala Val Lys
565 570 575

Trp Ala Lys Ala Ile Pro Gly Phe Arg Asn Leu His Leu Asp Asp Gln
580 585 590

Met Thr Leu Leu Gln Tyr Ser Trp Met Phe Leu Met Ala Phe Ala Leu
595 600 605

Gly Trp Arg Ser Tyr Arg Gln Ser Ser Ala Asn Leu Leu Cys Phe Ala
610 615 620

Pro Asp Leu Ile Ile Asn Glu Gln Arg Met Thr Leu Pro Cys Met Tyr
625 630 635 640

Asp Gln Cys Lys His Met Leu Tyr Val Ser Ser Glu Leu His Arg Leu
645 650 655

Gln Val Ser Tyr Glu Glu Tyr Leu Cys Met Lys Thr Leu Leu Leu Leu
660 665 670

Ser Ser Val Pro Lys Asp Gly Leu Lys Ser Gln Glu Leu Phe Asp Glu
675 680 685

Ile Arg Met Thr Tyr Ile Lys Glu Leu Gly Lys Ala Ile Val Lys Arg
690 695 700

Glu Gly Asn Ser Ser Gln Asn Trp Gln Arg Phe Tyr Gln Leu Thr Lys
705 710 715 720

Leu Leu Asp Ser Met His Glu Val Val Glu Asn Leu Leu Asn Tyr Cys
725 730 735

Phe Gln Thr Phe Leu Asp Lys Thr Met Ser Ile Glu Phe Pro Glu Met
740 745 750

Leu Ala Glu Ile Ile Thr Asn Gln Ile Pro Lys Tyr Ser Asn Gly Asn
755 760 765

```
Ile Lys Lys Leu Leu Phe His Gln Lys
    770                 775
```

## Beschreibung

Die vorliegende Erfindung betrifft stabil transformierte Hefezellen, die einen Hormonrezeptor und ein für den Hormonrezeptor spezifisches Response-Element in Kopplung mit einem Reportergen aufweisen, die Verwendung dieser Hefezellen in einer Messvorrichtung, z.B. einem hochempfindlichen Biosensor, den Biosensor und ein Messverfahren für den Nachweis von Hormonen oder von Substanzen mit Hormonwirkung. Weiterhin betrifft die Erfindung eine Zusammenstellung der Hefezellen mit Zusammensetzungen, die für das Messverfahren geeignet sind, z.B. in Form eines Detektionskits. Die Hefezellen zeichnen sich durch die mitotische Stabilität der Integration der Gene für einen Hormonrezeptor, das rezeptorspezifische Response-Element und den Reporter aus, sowie vorzugsweise durch das Fehlen eines Gens für eine Resistenz gegen ein Biozid, z.B. das Fehlen eines Antibiotikum - oder Antimykotikumresistenzgens. Vorzugsweise weist der Biosensor eine auxotrophe Hefezelle auf, die ein in das Genom integriertes Hormonrezeptorgen, ein in einen Hefepromotor integriertes rezeptorspezifisches Response-Element, ein Reportergen und ein die Auxotrophie komplementierendes Strukturgen enthält. Vorzugsweise ist der Promotor mit dem darin integrierten rezeptorspezifischen Response-Element unmittelbar funktional mit dem Reportergen fusioniert. Die erfindungsgemäße Messvorrichtung bzw. der Biosensor und das erfindungsgemäße Verfahren zeichnen sich dadurch aus, dass neben einer sehr hohen Empfindlichkeit auch die Reproduzierbarkeit der Messwerte für stark kontaminierte Proben erreicht wird, insbesondere bei Verwendung einer erfindungsgemäßen Hefe als Detektor für Stoffe mit hormoneller Wirkung.

## Stand der Technik

Die DE 103 45 386 A1 beschreibt einen Östrogensensor auf Basis rekombinanter Hefezellen, die ein Expressionsplasmid für einen Östrogenrezeptor enthalten und ein Reportergen, das hinter einem induzierbaren Promotor mit mindestens einer ERE-Sequenz (Estrogen (Östrogen) Response Element) angeordnet ist, die ein cis-aktivierender DNA-Abschnitt ist.

Der Östrogenrezeptor wird durch die Gegenwart von Östrogen oder von Substanzen mit Östrogenwirkung transaktiviert und bindet an die ERE-Sequenz, die im Promotor der Expressionskassette des Reportergens angeordnet ist und induziert so die Expression des Reportergens. Als Reportergen werden Fluoreszenzgene, z.B. GFP, EYFP und dsRed erwähnt, sowie als sekretorisches Enzym Phytase, Glucoamylasen, Glucanasen und Xylosidasen. Eine besonders bevorzugte Hefe ist *Arxula adeninivorans,* der bevorzugte Östrogenrezeptor ist hER$\alpha$ und hER$\beta$. Zu den dort beschriebenen Transformanten wird keine Kopienzahl der integrierten Expressionskassetten für den Östrogenrezeptor oder das Reportergen angegeben.

Hahn, Tag, Riedel, Uhlig, Baronian, Gellissen und Kunze beschreiben in Biosens. Bioelectron. Jan 19, 2006, rekombinante Hefezellen, die nach genetischer Manipulation den humanen Östrogenrezeptor $\alpha$ und das Phytasegen von *Klebsiella* (phyK) als Reportergen unter Kontrolle des durch Insertion von Östrogen-Response-Elementen veränderten Glucoamylasepromotors (GAA-Promotor) enthalten, zum Nachweis von 17$\beta$-Estradiol.

Jungbauer und Beck beschreiben in Journal of Chromatography B, 167-178 (2002) ein Reportergensystem in Hefe zur Bestimmung der Östrogenaktivität, bei dem die Hefe mit zwei Plasmiden transformiert wird, von denen das eine den Östrogenrezeptor kodiert und das andere LacZ als Reportergen unter der Kontrolle eines Promotors mit dem Östrogen-Response-Element.

Die US 4,513,280 beschreibt einen Biosensor, bei dem in zwei parallelen Messzellen ein Mikroorganismus enthalten ist, wobei in eine Zelle die zu untersuchende Probe eingebracht wird, und aus der relativen Änderung von CO2 in den beiden Zellen auf die Konzentration des Analyten in der Probe geschlossen wird.

Der Jahresforschungsbericht 2004 des IPK Gatersleben enthält auf Seiten 125-127 die schematische Darstellung einer Hefe, die das Phytasegen unter der Kontrolle eines Promotors mit dem Östrogen-Response-Element enthält und das Gen des menschlichen Östrogenrezeptors unter der Kontrolle eines konstitutiven Promotors.

Karube und Nakanishi beschreiben in I.E.E.E Engineering in Medicine and Biology 364-374 (1994) Sensoren, die immobilisierte Mikroorganismen aufweisen.

Wang et al. beschreiben in Appl. Microbiol. Biotechnol. in das Hefegenom von *Kluyveromyces lactis* integrierende Vektoren, die als Selektionsmarker das Gen für die Antibiotikumresistenz gegen Neomycin (neo) enthielten, um mehrfach nacheinander die Integration durchzuführen.

Die WO 97/44658 beschreibt eine Messvorrichtung, der auf einer Gelöstsauerstoffelektrode als physikalischem Transducer immobilisierte *A. adeninivorans* aufweist, die in wässriger Lösung ein vom Substrat abhängiges Signal erzeugen.

## Aufgabe der Erfindung

Gegenüber dem bekannten Stand der Technik ist es Aufgabe der vorliegenden Erfindung, eine für einen Biosensor verwendbare Hefe mit mitotisch stabil integrierten heterologen Genen, insbesondere ohne Gen für Biozidresistenz bereitzustellen, sowie einen Biosensor und ein Messverfahren mit verbesserter Empfindlichkeit, mit dem überdies Konzentrationen von Hormonen und Substanzen mit Hormonwirkung, z.B. Östrogen und Substanzen mit Östrogenwirkung, auch aus kontaminierten wässrigen Proben reproduzierbar bestimmt werden können.

## Allgemeine Beschreibung der Erfindung

Die Erfindung löst die vorgenannte Aufgabe durch Bereitstellung genetisch veränderter Hefen nach Anspruch 1, einer als Biosensor einsetzbaren Messvorrichtung, die eine genetisch veränderte Hefe nach Anspruch 1 enthält, und eines Messverfahrens, das unter Verwendung einer genetisch veränderten Hefe nach Anspruch 1 zur Bestimmung der Konzentration von Hormonen und Substanzen mit Hormonwirkung in wässrigen Proben mit hoher Sensitivität und Reproduzierbarkeit auch bei Kontamination der wässrigen Proben mit Fremdstoffen geeignet ist. Die erfindungsgemäße Hefe ermöglicht bei Verwendung in einem erfindungsgemäßen Biosensor ein Verfahren zur Bestimmung der Konzentration von Hormonen und Substanzen mit Hormonwirkung aus wässrigen Proben, die kontaminiert sein können, z.B. von Östrogen und von Stoffen mit Östrogenwirkung, mit hoher Empfindlichkeit und guter Reproduzierbarkeit. So konnten für die Östrogenwirkung Konzentrationen in Wasser bis minimal 1 ng/L, berechnet als 17β-Estradiol, reproduzierbar bestimmt werden, in Abwasser bis minimal 10 ng/L.

Weiterhin stellt die Erfindung ein Verfahren zur Herstellung erfindungsgemäßer genetisch manipulierter Hefen bereit, die in erfindungsgemäßen Biosensoren und Messverfahren Verwendung finden.

Der erfindungsgemäße Biosensor weist einen Reporter-Mikroorganismus zur Erzeugung eines Reportersignals spezifisch für ein Hormon oder Substanzen mit der Wirkung dieses Hormons auf, insbesondere erfindungsgemäß genetisch manipulierte Hefen, die z.B. in einer Kopienzahl von wenigstens 2 bis 10, vorzugsweise wenigstens 20, besonders bevorzugt 30 bis 70, insbesondere 50 Kopien pro Genom unabhängig voneinander eine Expressionskassette eines Hormonrezeptors, z.B. eines Glucocorticoid-, Progesteron- und/oder Androgenrezeptors, z.B. eines Östrogenrezeptors, vorzugsweise des menschlichen Östrogenrezeptors *hERα* und/oder *hERβ,* mit einem starken konstitutiven Promotor und eine Expressionskassette eines Reportergens, das ein detektierbares Translationsprodukt erzeugt oder dessen Translationsprodukt ein Substrat zu einem detektierbaren Produkt umsetzt, aufweisen. Dabei ist das Reportergen funktionell in 3' zu einem Promotor angeordnet, der zu seiner Steuerung mindestens eine Erkennungssequenz in funktioneller Anordnung zur Wechselwirkung mit dem Hormonrezeptor aufweist, vorzugsweise 1 bis 5, besonders bevorzugt 2 bis 3 gleiche oder verschiedene rezeptorspezifische RE (Response-Element)-Sequenzen als Erkennungssequenzen, z.B. ERE für Östrogenrezeptoren, GRE für Glucocorticoidrezeptoren, PRE für Progesteronrezeptoren, und ARE (Androgen-Response-Element). Als rezeptorspezifisches RE kann die in der Literatur jeweils beschriebene Bindungssequenz verwendet werden, die von den vorgenannten Rezeptoren erkannt wird bzw. mit einem dieser in funktionelle Wechselwirkung tritt.

Eine erfindungsgemäße Hefezelle umfasst daher eine Hefezelle, die eine Auxotrophie aufweist und ein in das Genom integriertes erstes Nukleinsäurekonstrukt mit einer ersten Expressionskassette, die eine einen Hormonrezeptor kodierende Nukleinsäuresequenz aufweist, einer zweiten Expressionskassette, die ein Reportergen unter der Kontrolle eines von zumindest einem für den Hormonrezeptor spezifischen Response-Element-Abschnitt regulierten Promotors aufweist, wobei das erste Nukleinsäurekonstrukt eine dritte Expressionskassette umfasst, die ein die Auxotrophie komplementierendes Strukturgen aufweist, wobei der Promotor der dritten Expressionskassette eine auf maximal 75% der Aktivität des natürlichen Promotors des komplementierenden Strukturgens, das ihm funktional zugeordnet ist, verminderte Aktivität hat, wobei die Hefezelle ein zweites Nukleinsäurekonstrukt mit zumindest einer Expressionskassette des die Auxotrophie komplementierenden Strukturgens unter der Kontrolle eines Promotors mit gegenüber dem natürlichen Promotor unveränderten Aktivität enthält und eine Mehrzahl von Kopien des ersten Nukleinsäurekonstrukts in das Genom integriert ist, vorzugsweise kein Biozidresistenzgen. Anstelle der einen Hormonrezeptor kodierenden Nukleinsäuresequenz kann die erste Expressionskassette ein anderes Strukturgen enthalten. Die zweite Expressionskassette ist optional, wenn das von der ersten Expressionkassette kodierte Strukturgen für die erwünschte Wirkung kein ko-exprimiertes zweites Strukturgen benötigt.

Die Auxotrophie der Hefe bezieht sich z.B. auf ein ursprüngliches genomisches Defektgen, das vor der Integration der erfindungsgemäßen Nukleinsäurekonstrukte phänotypisch ist, und das die Auxotrophie komplementierende Strukturgen hat vorzugsweise die Wildtypsequenz des genomischen Defektgens. Vorzugsweise ist die Auxotrophie ein Defektgen im Synthesestoffwechsel, besonders bevorzugt im Aminosäuresynthesestoffwechsel, z.B. das Defektgen für die Trpytophanauxotropie der *atrp1*-Mutante und das komplementierende Gen das *ATRP1*-Gen (Sequenz erhältlich unter Zugangsnummer AM261500, EMBL), d.h. die Nukleinsäuresequenz des Wildtypgens. Beispielhafte komplementierende Strukturgene sind *ALEU2* (Sequenz erhältlich unter Zugangsnummer AJ488496, EMBL) (Wartmann et al., FEMS Yeast Research 3: 223-232 (2003)), das eine Leucinauxotrophie komplementiert, und *AILV1* (Sequenz erhältlich unter Zugangsnummer AJ222772, EMBL) (Wartmann et al., Yeast 14: 1017-1025 (1998)), das eine Valin-Isoleucin-Auxotrophie komplementiert. Besonders bevorzugt hat das die Auxotrophie komplementierende Strukturgen eine Homologie der DNA-Sequenz von mindestens 80, bevorzugt 90, bevorzugter 95% zum genomischen Defektgen, besonders bevorzugt 99%.

Vorzugsweise kodieren die in die Hefe integrierten Hormonrezeptorgene die natürliche menschliche Aminosäuresequenz, wobei die Nukleinsäuresequenz die Kodons enthalten kann, die von der Hefe bevorzugt sind, z.B. durch Änderung der humanen Kodons an die von Hefe bevorzugten Kodons.

Vorzugsweise ist das Response-Element in zwei benachbarten, z.B. aneinander angrenzenden Kopien in die Promotorsequenz eingefügt, besonders bevorzugt im Bereich -120 bis -90 nt, insbesondere an Position -107, gemessen vom Translationsstart. Als Promotor wird vorzugsweise der homologe Glucoamylase-Promotor verwendet, für die Hefe *Arxula adeninivorans* insbesondere der als *GAA*-Promotor (Promotor des Glucoamylasegens von *Arxula adeninivorans*) bezeichnete Promotor, der *ATRP1*-Promotor von *ATRP1* (Sequenz erhältlich mit *ATRP1*) und der *AILV1*-Promotor (Sequenz erhältlich mit *AIL V1*)

Als eine Folge der mehrfachen Integration von Expressionskassetten zumindest einer der Expressionskassetten mit Hormonrezeptorgen oder Reportergen, vorzugsweise beider, hat die erfindungsgemäße Hefe und daher das damit durchgeführte Messverfahren und der erfindungsgemäße Biosensor eine hohe Empfindlichkeit gegenüber Hormonen und Substanzen mit Hormonwirkung, d.h. der Reporter wird bereits bei niedrigen Konzentrationen von Hormon und Substanzen mit Hormonwirkung erzeugt, und damit ein detektierbares Signal.

Besonders bevorzugt ist das Reportergen das Phytasegen, dessen Translationsprodukt die Hydrolyse phosphathaltiger Verbindungen unter Freisetzung von Phosphat katalysiert. Entsprechend weist der erfindungsgemäße Biosensor vorzugsweise einen Transducer auf, z.B. eine Einrichtung zur amperometrischen oder voltmetrischen Erfassung, mit dem die enzymatische Aktivität der Phytase detektiert werden kann. Alternativ ist auch möglich, dass das Translationsprodukt des Reportergens, d.h. der Reporter, aus einem Substrat ein kolorimetrisch und/oder luminometrisch erfassbares Reaktionsprodukt erzeugt, sodass zur Detektion ein optischer Detektor, beispielsweise ein Photometer oder Luminometer einsetzbar ist.

Bei kontaminierten wässrigen Probenzusammensetzungen, beispielsweise Oberflächenwasser, Rohabwasser, behandeltem Abwasser, z.B. Proben aus dem Kläranlagenzulauf oder -ablauf, ist es bevorzugt, die zu analysierende Probe auf über 75 °C, vorzugsweise auf 95 bis 100 °C für einen Zeitraum von 10s bis 20 min, vorzugsweise 5 bis 15 min, insbesondere für 10 min zu erwärmen. Entsprechend weist der erfindungsgemäße Biosensor vorzugsweise eine Erwärmungseinrichtung auf, mit der eine zu messende Probe auf mindestens 50 °C, vorzugsweise 75 °C bis 100 °C für eine Zeit von 10 s bis 20 min, vorzugsweise 5 bis 15 min, besonders bevorzugt ca. 10 min erwärmbar ist.

Bei der Vorbereitung der vorliegenden Erfindung hat sich herausgestellt, dass genetisch modifizierte Hefestämme, insbesondere der bevorzugten Gattung *Arxula,* nicht ohne weiteres mehrfache Kopien der Expressionskassetten aufweisen. Weiterhin wurde beobachtet, dass Klone, die eine Mehrzahl ins Genom integrierter Expressionskassetten aufweisen, mitotisch nicht stabil sind, sondern die Kopienzahl dieser Expressionskassetten mit zunehmender Kultivierungsdauer reduziert wird, bzw. verloren gehen. Dies entspricht der Beobachtung, dass Integranten von *Arxula* mitotisch instabil sind, wie in DD 298821 A5 und Kunze et al., Arxula adeninivorans in: Non-conventional yeasts in Biotechnology (Hrsg. Wolff), Springer Verlag, Berlin - Heidelberg - New York, Seiten 389-409 (1996)) beschrieben ist.

Die für den erfindungsgemäßen Biosensor einsetzbare Hefe *Arxula,* besonders bevorzugt *Arxula adeninivorans,* wird durch Verwenden von Nukleinsäurekonstrukten mit einer Mehrzahl von Kopien der Expressionskassette für zumindest einen Hormonrezeptor und zumindest einen Reporter versehen, indem das eingesetzte Nukleinsäurekonstrukt neben jeweils terminal angeordneten, zumindest teilweise homologen Abschnitten, vorzugsweise homolog zur 25S rDNA, ein eine Auxotrophie der genetisch zu manipulierenden Hefe komplementierendes Strukturgen unter der Kontrolle eines stark geschwächten Promotors aufweist. Entsprechend weist die Hefe vor der erfindungsgemäßen genetischen Veränderung zumindest eine Auxotrophie auf. Der stark geschwächte Promotor, vorzugsweise mit einer Aktivität von maximal 50%, bevorzugter von max. 30%, besonders bevorzugt von maximal 20% der ursprünglichen Aktivität des mit dem komplementierenden Strukturgen gekoppelten Promotors führt dazu, dass nach primärer Integration von meist nur einer Kopie dieser Expressionskassette des komplementierenden Strukturgens mit dem stark geschwächten Promotor sehr gering prototrophe und deshalb sehr langsam wachsende Transformanten erhalten werden, die im Vergleich zu normal prototrophen Transformanten einem hohen Selektionsdruck ausgesetzt sind.

Aufgrund der multiplen Kopienzahl der chromosomalen rDNA können singulär integrierte Expressionskassetten durch Rekombination spontan amplifiziert und die dadurch bewirkte verstärkte Prototrophie zur Anreicherung und dadurch zur Isolierung multipler Integranten benutzt werden. Entsprechend führt die Integration eines erfindungsgemäßen Nukleinsäurekonstrukts zur mehrfachen Integration des die Auxotrophie der Hefe komplementierenden Strukturgens mit seinem abgeschwächten Promotor, und in Verbindung damit zur mehrfachen Integration der Expressionskassette, die das Hormonrezeptorgen und das Reportergen enthält.

Überraschenderweise hat sich jedoch ausgestellt, dass derartige Transformanten auch bei mehrfacher Integration von Expressionskassetten mit dem eine Auxotrophie komplementierenden Strukturgen mit abgeschwächtem Promotor mitotisch instabil sind.

Daher ist erfindungsgemäß vorgesehen, derartige Transformanten mit mehrfach integrierten erfindungsgemäßen Nukleinsäurekonstrukten in einem zweiten Schritt mit einer Expressionskassette des die Auxotrophie komplementierenden Strukturgens zu transformieren, die einen Promotor mit natürlicher Aktivität aufweist, vorzugsweise den natürlichen Promotor des komplementierenden Strukturgens. Die so erhaltenen Transformanten können auch als zweifach auxotrophie-komplementierte Transformanten bezeichnet werden. Diese zweifach auxotrophie-komplementierten Transformanten sind mitotisch stabil, auch bezüglich der mehrfach integrierten Expressionskassetten für zumindest einen Hormonrezeptor und zumindest ein Reportergen unter der Kontrolle eines Promotors, der ein rezeptorspezifisches Response-Element aufweist, und zeichnen sich überdies dadurch aus, dass sie kein während der genetischen Manipulation eingebrachtes Resistenzgen gegen ein Biozid enthalten.

Erfindungsgemäße, genetisch manipulierte Hefen sind mitotisch stabil und sind erhältlich durch

1) Transformation einer Hefe, die zumindest eine Auxotrophie aufweist, mit einem ersten Nukleinsäurekonstrukt, das zumindest einen homologen Sequenzabschnitt, vorzugsweise jeweils einen endständigen, zur 255 rDNA der Hefe homologen Sequenzabschnitt aufweist und damit verbunden zumindest drei Expressionskassetten aufweist, nämlich

a) eine erste Expressionskassette, die zumindest ein Gen für einen Hormonrezeptor unter Kontrolle eines starken konstitutiven Promotors aufweist, alternativ eines starken induzierbaren Promotors,
b) eine zweite Expressionskassette, die einen starken hormoninduzierbaren Promotor mit Erkennungssequenzen für den Hormonrezeptor, vorzugsweise RE-Sequenzen aufweist, vorzugsweise 2 bis 5 RE-Sequenzen, in funktioneller Anordnung innerhalb des Promotorabschnitts in 5' zu dem Strukturgen angeordnet, welches ein einen Reporter kodierendes Reportergen ist, vorzugsweise ein lumineszentes Protein, besonders bevorzugt Phytase kodiert, und
c) eine dritte Expressionskassette, die ein die Auxotrophie der zu transformierenden Hefe komplementierendes Strukturgen unter der Kontrolle eines Promotors aufweist, der gegenüber dem natürlichen Promotor des komplementierenden Strukturgens eine stark verminderte Aktivität, vorzugsweise von maximal 50%, besonders bevorzugt von maximal 20% im Verhältnis zur Aktivität des natürlichen Promotors des komplementierenden Strukturgens aufweist;

und, in einem zweiten nachfolgenden Schritt, vorzugsweise nach Kultivierung der nach Schritt 1) erhältlichen Transformanten in Medium, das das Substrat, für das die ursprüngliche Auxotrophie der Hefe besteht, nicht enthält,

2) Transformation von aus Schritt 1 erhaltenen Integranten mit einem zweiten Nukleinsäurekonstrukt, das

das die Auxotrophie der Hefe komplementierende Strukturgen unter der Kontrolle eines Promotors aufweist, der zumindest etwa die Aktivität des natürlichen Promotors dieses Gens aufweist; vorzugsweise ist der Promotor des komplementierenden Strukturgens im zweiten Nukleinsäurekonstrukt der natürliche Promotor dieses.

Für die Herstellung mitotisch stabiler Transformanten ohne integriertes Resistenzgen ist die zweistufige Transformation einer auxotrophen Hefe mit einem ersten Nukleinsäurekonstrukt erforderlich, das eine erste Expressionkassette mit einer zu integrierenden Nukleinsäuresequenz, eine zweite Expressionskassette enthält, sowie eine dritte Expressionskassette mit einem die Auxotrophie der zu transformierenden Hefe komplementierenden Strukturgen unter der Kontrolle eines Promotors, dessen Aktivität gegenüber dem natürlichen Promotor des zu komplementierenden Strukturgens vermindert ist, vorzugsweise auf 50 bis 20%, gefolgt von einer zweiten Transformation mit einem zweiten Nukleinsäurekonstrukt, das ein die Auxotrophie der Hefe komplementierendes Strukturgen unter der Kontrolle eines Promotors enthält, der zumindest etwa die Aktivität des natürlichen Promotors dieses Gens aufweist, vorzugsweise mit Kultivierung der Transformanten zwischen der Transformation mit dem ersten und dem zweiten Nukleinsäurekonstrukt.

Es hat sich gezeigt, das die Transformation der Integranten, die im ersten Schritt erhalten wurden, im zweiten Schritt mit dem die Auxotrophie komplementierenden Strukturgen unter Kontrolle eines Promotors mit einer der natürlichen Aktivität des Promotors entsprechenden Aktivität zur mitotischen Stabilität der Integranten führt, sodass in diesen Transformanten die Kopienzahlen von erster und zweiter Expressionskassette im Wesentlichen beibehalten wird.

Aufgrund des erfindungsgemäßen Klonierungsverfahrens zur genomischen Integration der Expressionskassetten für Hormonrezeptor und Reporter weisen die erfindungsgemäßen Hefen keine Resistenzmarker auf und sind daher frei von Selektionsmarkern, zum Beispiel Antibiotikaresistenzen, insbesondere von in der Umwelt unerwünschten Antibiotikaresistenzen.

Die Hormonrezeptorgene sind ausgewählt aus der Gruppe, die den Östrogenrezeptor kodieren, insbesondere *hER*$\alpha$ und *hER*$\beta$, den Progesteronrezeptor, den Androgenrezeptor und den Glucocorticoidrezeptor kodieren, jeweils vorzugsweise den humanen Rezeptor. Als Response-Element wird jeweils das rezeptorspezifische Response-Element bevorzugt, insbesondere das Östrogen-Response-Element (*ERE*) das humane Hormon-Response-Element (HRE), das mit allen vorgenannten Rezeptoren in Anwesenheit des spezifischen Hormons in Wechselwirkung tritt und den Promotor, in dem es enthalten ist, aktiviert bzw. dessen Blockade aufhebt. Es wurde gefunden, dass gelegentlich identische Nukleinsäuresequenzen für das HRE Element und das Glucocorticoid-Response-Element (GRE), das Progesteron-Response-Element (PRE) und das Androgen-Response-Element (ARE) angegeben sind.

Erfindungsgemäße Hefen sind stark osmotolerant, insbesondere stark salztolerant und physiologisch stabil. Besonders bevorzugt sind erfindungsgemäß zu verwendende Hefen überdies temperaturtolerant, beispielsweise bis 40 °C, bevorzugt bis 50 °C tolerant.

Erfindungsgemäße Hefen sind solche der Gattung *Arxula,* insbesondere *Arxula adeninivorans* oder *Arxula terrestris,* beispielsweise erhältlich unter der Zugangsnummer ATCC 76597 bzw. ATTC 60136.

Das erfindungsgemäße Messverfahren umfaßt zumindest die Messung eines Signals, z.B. eines optisch (insbesondere photometrisch oder luminometrisch), voltmetrisch, kapazitiv oder amperometrisch erfaßbaren Signals, das von dem Reporter in Anwesenheit von Hormon oder Substanzen mit Hormonwirkung verursacht wird. Entsprechend weist die Messvorrichtung z.B. einen Photodetektor auf, insbesondere eine Spannungs-, Kapazitäts- oder Strommesseinrichtung, z.B. einen Transducer. Vorzugsweise wird die Auswertung des Messsignals dadurch verbessert, dass zusätzlich eine Probe mit einer zusätzlichen bekannten Konzentration des zu bestimmenden Hormons dotiert und ebenfalls dem Messverfahren unterzogen wird.

Das Messverfahren, zu dessen Durchführung die Messvorrichtung eingerichtet ist, zur Bestimmung der Konzentration von Hormonen und von Stoffen mit Hormonwirkung, nachfolgend zusammenfassend auch Hormonwirkungskonzentration genannt, auf Basis von Messwerten geeignet, die mittels einer erfindungsgemäßen Hefe erzeugbar sind.

Das erfindungsgemäße Messverfahren umfasst die Aufnahme von Messwerten und die Auswertung der Messwerte durch Berechnung einer Hormonwirkungskonzentration, einer dieser zugeordneten Messunsicherheit und eines der Hormonwirkungskonzentration zugeordneten Vertrauensintervalls. Das Messverfahren umfasst auch die Bestimmung einer Kalibrierkurve durch Bestimmung von Messwerten für Zusammensetzungen bekannter Hormonwirkungskonzentrationen, Bestimmung von Messwerten für Probe, unverdünnt und in bekannten Verdünnungsstufen und/oder Bestimmung von Messwerten für Probe nach Dotierung um eine bekannte Hormonwirkungskonzentration.

Die Messunsicherheit wird nach dem Bootstrap-Verfahren berechnet, mit den Schritten der Erzeugung zufälliger Variationen aller in die Bestimmung der stoffspezifischen Konzentration, z.B. der Hormonwirkungskonzentration, eingehenden Einzelmesswerte, Berechnung der Hormonwirkungskonzentration für jede der erzeugten Variationen mit den Berechnungsschritten

1) Berechnung der auf 1 bis 4 freien Parametern (A, B, C, D) basierenden Kalibrierkurve, deren Messwerte für bekannte Hormonwirkungskonzentrationen bestimmt wurden. Grundlage für die Berechnung sind die jeweils erhaltenen Messwerte bei unterschiedlichen Hormonwirkungskonzentrationen (Leer- bzw. Hintergrundprobe, sowie mindestens eine bekannte Hormonwirkungskonzentration). Die Berechnung basiert auf einem iterativen Algorithmus zur Erzeugung einer Maximum-Likelihood-Schätzung.

2) Berechnung des Inhibierungsfaktors sowie der Hormonwirkungskonzentration der unverdünnten Probe nach Inhibierungskorrektur. Die Berechnung erfolgt auf Basis der Messwerte, die für a) Probe, vorzugsweise zusätzlich b) zumindest einer Probe nach Verdünnung in bekanntem Verhältnis, c) zumindest einer dotierten Probe als Positivkontrolle, und c) von Probe, vorzugsweise zusätzlich zumindest einer verdünnten Probe, jedoch mit Messfühler, der keinen für die Hormonwirkungskonzentration spezifischen Rezeptor aufweist, insbesondere mit Hefe ohne Hormonrezeptor (Hintergrundkontrolle).

3) Erzeugung von mindestens 10, vorzugsweise mindestens 100 simulierten und zufällig variierten Kalibriermesswerten, jeweils mit denselben Dotierungen und Verdünnungen wie für die tatsächlich durchgeführte Kalibriermessung. Die zufällige Variation dieser Kalibrierexperimente nimmt eine relative Standardabweichung von 4% - 6% je Einzelmessung an, bzw. berücksichtigt eine solche relative Standardabweichung. Für jede dieser 100 simulierten Kalibriermessungen werden die Berechnungen nach Schritt 1 durchgeführt und die Kalibrierparameter ermittelt. Aus den berechneten Ergebnissen der simulierten Kalibriermesswerte werden die zugehörigen Standardfehler für die Kalibrierkurve ermittelt.

4) Erzeugung von mindestens 100, vorzugsweise mindestens 1000 simulierten und zufällig variierten Probenmessungen, jeweils mit erfindungsgemäßer Hefe, ohne Dotierung, mit Dotierung, sowie mit der Kontrollhefe ohne hormonspezifischen Rezeptor. Die zufällige Variation dieser Kalibriermessungen basiert auf der Annahme einer relativen Standardabweichung von 4% - 6% je Einzelmessung, bzw. berücksichtigt eine solche Standardabweichung. Für jede dieser simulierten Probenmessungen werden auch die Kalibrierparameter gemäß der Ergebnisse der Kalibriermesswerte, die aus Schritt 3) erhalten wurden, zufällig variiert. Aus den Ergebnissen der gemäß Schritt 2) erhaltenen Hormonwirkungskonzentration wird die Messunsicherheit sowie das Vertrauensintervall berechnet.

5) Schritt 4) wird vorzugsweise für verschiedene Dotierungen der Probe um z.B. Zusatz einer Hormonwirkungskonzentration um 20 ng/L, 40 ng/L, 60 ng/L, 80 ng/L und 100 ng/L, und für alternative Verdünnungen der Probe, z.B. auf 1%, 5%, 10%, 20%, 40%; 70%, 100% in Verdünner, z.B. Wasser, ausgeführt. Dabei wird wahlweise die Anzahl der Erzeugungen simulierter Probenmessungen auf 1/10 reduziert, um ein schnelleres Rechnen zu ermöglichen. Aus den berechneten Messunsicherheiten bzw. Vertrauensintervallen werden jene ausgewählt, bei der die ermittelte Messunsicherheit am kleinsten ist. Vorzugsweise sieht das Verfahren dann, wenn sich ein Ergebnis einer erheblich reduzierten Messunsicherheit ergibt, die automatische Ausgabe einer Empfehlung zur Wiederholung der Messung vor.

Schematisch können die Berechnungsschritte folgendermaßen dargestellt werden:

| |
|---|
| 1: Berechnung der Kalibrierkurve |
| |
| 2: Berechnung von Inhibierungsfaktor und Hormonwirkungskonzentration |
| |
| 3: Simulation des Kalibrierexperimentes |
| |
| 4: Simulation der Berechnung von Inhibierungsfaktor und Hormonwirkungskonzentration |
| |
| 5: Optimierung von Dotierung und Verdünnung (Simulationsexperiment) |

Es hat sich gezeigt, dass das erfindungsgemäße Messverfahren durch die Schritte der Auswertung beobachtete starke Schwankungen berücksichtigt, die z.B. von der Dotierung und von der Verdünnung einer Probe abhängen.

Neben dem Verfahren zur Berechnung der Messunsicherheit und der Vertrauensgrenzen erlaubt das Verfahren die Ermittlung optimaler Verdünnungsfaktoren und Dotierungen. Dazu wird auf der Basis der ermittelten Hormonwirkungskonzentration und der berechneten Inhibierung für alternative Dotierungsstufen, z.B. für Östrogen von Dotierungen um 20 ng/L, 40 ng/L, 60 ng/L, 80 ng/L und 100 ng/L, und alternative Verdünnungen, z.B. 1%, 5%, 10%, 20%, 40%; 70%, 100%, der jeweils erwartete Messwert für die Messungen mit spezifischem Rezeptor erfindungsgemäße Hefe, z.B. Hefe mit Östrogenrezeptor) und Kontrollmessungen ohne spezifischen Rezeptor (z.B. Hefe ohne Hormonrezeptor) extrapoliert, wobei die beschriebenen Berechnungsverfahren, insbesondere zur Kalibration mit verdünnten und/oder dotierten Proben verwendet werden. Auf der Basis der so berechneten Ergebnisse zur Hormonwirkungskonzentration wird wiederum die zu erwartende Messunsicherheit bestimmt, um schließlich jene Kombination von Verdünnung und Dotierung zu ermitteln bzw. auszuwählen, die eine minimale Messunsicherheit ergibt. Dieses Verfahren führt z.B. in Beispiel 5 zu einer idealen Kombination der unverdünnten Probe mit 60 ng/L Dotierung. Bei dieser Kombination aus Verdünnung und Dotierung der Probe ergibt sich eine bessere Messunsicherheit von 15%. Generell ist zu beachten, dass eine Verdünnung in der Regel nur bei noch stärker inhibierenden Proben zu einer Verringerung der Messunsicherheit führt.

Eine erfindungsgemäße Messvorrichtung weist einen Rechner auf, der eingerichtet ist, das erfindungsgemäße Verfahren zur Berechnung durchzuführen, und weist eine erfindungsgemäße Hefe auf.

Weiter bevorzugt umfaßt die Messung die Bestimmung eines Hintergrundsignals mit demselben Aufbau und Verfahren mit der Änderung, dass die verwendete Hefe nicht transformiert ist, oder mit einem ersten Nukleinsäurekonstrukt transformiert ist, das keinen Hormonrezeptor und/oder kein RE aufweist. Besonders bevorzugt umfaßt die Messung des Hintergrundsignals, d.h. eines nicht analytenspezifischen, insbesondere eines nicht hormonspezifischen Messwerts, neben der Messung der Probe auch die Messung der Probe nach deren Dotierung mit einer bekannten zusätzlichen Konzentration eines oder mehrerer Hormone.

Die Auswertung der Messsignale erfolgt vorzugsweise rechnergestützt automatisch mit einem Kalibrierkurvenverfahren, bei dem Parameter für die mathematische Auswertung für jede Art von Probe nur einmal bestimmt werden müssen, z.B. für Kläranlagenzuläufe, Kläranlagenabläufe, Abwässer einer Produktionsanlage bzw. klares Wasser (Mineralwässer, natürliche Oberflächengewässer) jeweils separat. Besonders bevorzugt erfolgt die Bestimmung der Parameter zur Kalibrierung mit jedem erfindungsgemäßem Sensor separat, besonders bevorzugt zusätzlich vor jeder Messreihe. Die Programmcode-Informationen zur Ausführung des Auswertungsverfahrens sind vorzugsweise auf einem von einem Rechner lesbaren Speichermedium gespeichert.

Das Kalibrierkurvenverfahren zur Auswertung umfaßt die Bestimmung sogenannter Stützstellen, auch Kalibratorkonzentrationen genannt. Das Messsignal, z.B. abhängig vom Reportergen und/oder einem vom Reporter umgesetzten Substrat das photometrische Signal oder ein amperometrisches Signal, erhalten aus der Messung der Probe und der Messung der dotierten Probe mit der erfindungsgemäß genetisch manipulierten Hefe, besonders bevorzugt in Kombination mit Signalen aus der Messung der Probe und/oder der dotierten Probe mit einer Hefe, die optional das Reportergen jedoch keinen Hormonrezeptor und/oder kein RE in 5' vor dem Reportergen aufweist.

Für die Auswertung der Messsignale, insbesondere zur Bestimmung der Kalibratorkonzentrationen ist es bevorzugt, neben Messungen der Probe, zumindest einem verdünntem Aliquot der Probe und zumindest einem mit einer bekannten Konzentration einer Substanz mit Hormonwirkung dotierten Aliquot der Probe auch Messungen mit denselben Dotierungen wie sie für die Probenaliquots vorgenommen wurden, mit Wasser oder Puffer durchzuführen. Diese Kalibriermessungen mit Wasser oder Puffer, auch als Kalibrierlösung bezeichnet, ergeben Messwerte, die zur Bestimmung der Parameter der mathematischen Auswertung verwendet werden können. Bevorzugt wird zumindest eine Kalibriermessung mit Kalibrierlösung und zumindest einer mit einer bekannten Konzentration einer Substanz mit Hormonwirkung dotierten Kalibrierlösung vorgenommen, d.h. mit einer Kalibrierlösung bekannter Konzentration der Substanz mit Hormonwirkung, z.B. Östrogenwirkung. Diese Kalibriermessungen werden zur Bestimmung der Parameter für die mathematische Auswertung zumindest einmal vorgenommen, optional zusätzlich vor und/oder nach Probenmessungen.

Das Kalibrierkurvenverfahren geht von einem sigmoidalen Verlauf des Messsignals $y_{ij}$ in Abhängigkeit von dem Messsignal bzw. der Hormonwirkungskonzentration aus. Dabei werden die Stützstellen durch die Parameter A, B, C und D abgebildet und in die Formel I eingesetzt, in der $e_{ij}$ einen zugehörigen Fehler darstellt, mit dem Index i für die Messreihe und Index j für den Messwert dieser Messreihe:

$$y_{ij} = \frac{A - D}{1 + \left(\frac{x}{C}\right)^B} + D + \varepsilon_{ij} \qquad \text{(I)}$$

Es wurde gefunden, dass der Fehler annähernd proportional zum Messwert ist, so dass die logarithmische Transformation zur nachfolgenden Formel II

$$\ln(y_{ij}) = \ln\left(\frac{A - D}{1 + \left(\frac{x}{C}\right)^B} + D\right) + \varepsilon_{ij} \qquad \text{(II)}$$

zur bevorzugten Fassung führt. Diese Berechnungsformel trifft vor allem dann zu, wenn die Aktivität des Reporters nicht durch Probenbestandteile gehemmt wird. Die Parameter A, B, C und D werden vorzugsweise für jeden Messlauf neu bestimmt, indem zuvor Kalibriermessungen mit Proben bekannter Hormonwirkungskonzentration durchgeführt werden.

Im einzelnen ist Parameter D verhältnismäßig unkritisch und liegt typischerweise im Bereich von 1 bis 3, vorzugsweise 1,5 bis 2. Auch Parameter B kann im Rahmen einer Grundkalibrierung verbindlich bestimmt werden, so dass für spezifische Messlaufkalibrierungen vorzugsweise nur Parameter B und C jeweils zu ermitteln sind.

Für den Fall, dass die Aktivität des Reporters durch Probenbestandteile gehemmt wird, wird die Berechnung der Hormonwirkungskonzentration vorzugsweise mit der folgenden Formel III durchgeführt, in der λ ein Inhibierungsfaktor ist:

$$\ln(y_{ij}) = \ln\left(\frac{A - D}{1 + \left(\frac{x}{\lambda_i + C}\right)^B} + D\right) + \varepsilon_{ij} \qquad \text{(III)}$$

Der Inhibierungsfaktor λ wird vorzugsweise für jeden Messlauf, z.B. für jede Probenart neu festgelegt. Zur Festlegung werden z.B. die Phytaseaktivität in der ursprünglichen Probe und in mindestens einer, vorzugsweise mindestens 2 verschiedenen Verdünnungsverhältnissen der Probe mit Wasser bestimmt, vorzugsweise zusätzlich in Parallelansätzen der Probe und der Verdünnungsverhältnisse der Probe in Wasser mit Dotierung an Substanzen mit Hormonwirkung, die eine bekannte Konzentration der Hormonwirkung, z.B. Östrogen zufügt. Der Inhibierungsfaktor λ läßt sich bestimmen mit der Formel IV, in der q das Verdünnungsverhältnis der originalen Probe in der Verdünnung mit Wasser angibt:

$$\lambda^{-1} = \frac{1-d}{1+\left(\frac{q}{c}\right)^{b}} + d \qquad \text{(IV)}$$

Dabei beschreiben die Parameter a=1, b, c, d die sigmoidale Kurve. Wenn zwei Verdünnungsverhältnisse verwendet werden, kann b=1 gesetzt werden, für eine größere Anzahl an Verdünnungsverhältnissen ist b durch Grundkalibrierung bestimmbar. Die Werte für die Parameter c und d werden dadurch aus den Daten der Verdünnungsstufen hergeleitet, dass sie durch nichtlineare Optimierung auf Basis der logarithmierten Messwerte ermittelt werden.

In bevorzugter Ausführungsform wird die Berechnung der Hormonwirkungskonzentration aus den Messwerten des Reportersignals mit der folgenden Formel V durchgeführt, bei der nicht auf östrogene Wirkung zurückgehende Messeffekte vermindert bzw. eliminiert werden:

$$\ln(y_{ij}) = \ln\left(\frac{A-D}{1+\left(\frac{x}{\lambda_i + C}\right)^{B}} + D + \theta\cdot q\right) + \varepsilon_{ij} \qquad \text{(V)},$$

wobei $\theta$ die erwartete Zunahme des Messwerts, z.B. der optischen Dichte für den Fall der Messung eines photometrischen Reportersignals ist.

Als Beispiel für Kalibrierungsmessungen können die folgenden Verdünnungen originaler Probe mit Dotierung durch Zugabe einer bekannten Hormonwirkungskonzentration für Östrogen durchgeführt werden, wobei als Kontrollversuch Hefe ohne Östrogenrezeptor (Kontrollklon), jedoch mit Reportergen eingesetzt wird:

| Hefe | Verdünnungsfaktor der originalen Probe mit Wasser | Dotierung mit Östrogen |
|---|---|---|
| erfindungsgemäß | 1 | 0 |
| erfindungsgemäß | 0,5 | 0 |
| erfindungsgemäß | 1 | 20 ng/L |
| erfindungsgemäß | 0,5 | 20 ng/L |
| Kontrollklon | 1 | 0 |
| Kontrollklon | 0,5 | 0 |

**Genaue Beschreibung der Erfindung.**

Die Erfindung wird nun genauer anhand von Beispielen mit Bezug auf die Figuren beschrieben, in denen

- Figur 1 eine schematische Darstellung eines Vektors zeigt, in dem ein erfindungsgemäßes erstes Nukleinsäurekonstrukt mit Expressionskassetten für ein Reportergen, einen humanen Östrogenrezeptor und ein eine Auxotrophie komplementierendes Strukturgen zwischen zwei homologen Abschnitten enthalten ist,
- Figur 2 schematisch einen erfindungsgemäßen Biosensor zeigt

- Figur 3 schematisch ein Beispiel für Fluidströme eines erfindungsgemäßen Biosensors zeigt, und
- Figur 4 eine weitere Ausführungsform der Fluidströme eines erfindungsgemäßen Biosensors zeigt.

Beispiel 1: Herstellung genetisch manipulierter Hefe mit einer Mehrzahl von Kopien der Expressionskassette für den Östrogenrezeptor und den Reporter

Zur Herstellung von Hefe-Transformanten mit einer Mehrzahl von in das Genom integrierten Kopien der Expressionskassette des Östrogenrezeptors und des Reporters wurde ein für Tryptophan auxotropher Stamm von *Arxul a adeninivorans* mit dem Auxotrophiemarker *atrp1* (G1211) in einem ersten Schritt mit einem Nukleinsäurekonstrukt transformiert, das als Beispiel für einen Östrogenrezeptor das Gen für den menschlichen Östrogenrezeptor α (*hERα*) in einer ersten Expressionskassette, das *phyK*-Gen, das für Phytase kodiert, in einer zweiten Expressionskassette und das *ATRP1-Gen* in einer dritten Expressionskassette unter der Kontrolle eines stark abgeschwächten Promotors enthielt. Die Selektion der Transformanten wurde mittels Kultivierung in Minimalmedium ohne Tryptophanzusatz erreicht. In einem zweiten Schritt wurden Transformanten mit einem zweiten Nukleinsäurekonstrukt, das das *ATRP1-* Gen unter der Kontrolle eines Promotors mit gegenüber dem natürlichen Promotor nicht verringerter Aktivität enthielt.

Das erste Nukleinsäurekonstrukt, dessen Sequenz als Seq.-ID Nr. 1 dargestellt ist, weist die kodierende Sequenz für den *hERα*-Rezeptor unter der Kontrolle des Promotors *TEF1* auf. Der *TEF1*-Promotor (Translations-Elongationsfaktor 1α- Gen) ist ein starker konstitutiver eukaryotischer Promotor. Alternativ können der *AHSB4-* Promotor (Histon H4 - Gen) oder der *ARFC 3* - Promotor (Replikationsfaktor C - Gen) verwendet werden. Die den kodierenden Leserahmen (orf) jeweils zugeordneten regulatorischen Elemente und deren relative Orientierung zueinander sind in der Figur 1 dargestellt, in der auch die Richtung der Transkription mittels Pfeilspitzen gezeigt ist.

In 3' des *hERα*-Gens ist ein eukaryotischer Terminator angeordnet, hier der Terminator *PHO5* von *Saccharomyces cerevisiae.* Die erste Expressionskassette kontrolliert daher in Hefe die konstitutive starke Expression des hERα-Rezeptors. Bei Anwesenheit von Östrogen oder Verbindungen, die eine östrogenartige Wirkung haben, binden diese an den hERα-Rezeptor, der anschließend dimerisiert. Alternativ zu dem für *hERα* oder *hER*β kodierenden Gen kann auch ein anderer Östrogenrezeptor eingesetzt werden, der bei Interaktion mit einem oder mehreren Östrogenen oder einer Substanz mit Östrogenwirkung an eine regulatorische Sequenz (ERE-Sequenz) bindet, die in Anwesenheit von Östrogen oder von Substanzen mit Östrogenwirkung die Transkription des Reportergens erlaubt, beispielsweise wenn diese regulatorische Sequenz in funktionellem Zusammenhang mit einem Promotor angeordnet ist, der sich vor dem Reportergen befindet.

Die zweite Expressionskassette, nämlich die für den Reporter, ist in Figur 1 beispielhaft anhand der Phytase K (*phyK*) dargestellt, die zwischen einem modifizierten Promotor und einem Terminator, hier wiederum der *PHO5* - Terminator, angeordnet ist. Der Promotor für die zweite Expressionskassette, die das Reportergen enthält, weist einen regulatorischen Abschnitt auf, der in Anwesenheit von Östrogen bzw. einer Substanz mit Östrogenwirkung in Verbindung mit dem Östrogenrezeptor zu einer Aktivierung des Promotors führt, der in 5'zu dem Reportergen angeordnet ist. Entsprechend einer bevorzugten Ausführungsform ist das regulatorische Element ein ERE, vorzugsweise zwei ERE menschlichen Ursprungs, die beabstandet sind oder aneinander angrenzen, innerhalb der Promotorsequenz funktional vor dem Reportergen angeordnet. In Seq.-ID Nr. 1 ist der *GAA*-Promotor mit zwei unmittelbar aneinander angrenzenden ERE-Sequenzen, die in Position -107 vom Translationsstart des Reportergens in den Promotor integriert sind, als GAA2ERE107 gezeigt.

Der Promotor dieser zweiten Expressionskassette kann schwach konstitutiv oder induzierbar sein, vorzugsweise durch die Art der Kohlenstoffquelle regulierbar. Eine bevorzugter Promotor ist der Glucoamylase - Promotor *(GAA),* insbesondere bevorzugt der *GAA* - Promotor von *Arxul aadeninivorans* LS3. Der *GAA* - Promotor ist regulierbar; er wird durch die Art der Kohlenstoffquelle aktiviert bzw. inhibiert, z.B. durch Maltose oder Stärke aktiviert und durch Glucose fast vollständig reprimiert. Ein für die zweite Expressionskassette weiterer bevorzugter Promotor ist der Promotor des Xylitol-Dehydrogenasegens (*AXDH*-Gen*)* von *Arxula adeninivorans,* beispielsweise beschrieben in der PCT/ EP 01/05225. Alternativ kann der aus *Arxula adeninivorans* stammende *APHO1*-Promotor (saure Phosphatase mit Phytaseaktivität APHO1, beschrieben von P. Kaur, A. Lingner, B.Singh, E. Boer, J. Polajeva, G. Steinborn, R. Bode, G. Gellissen, T. Satyanarayana, G. Kunze (Antonie van Leeuwenhoek 91: 45-55 (2007)) für die zweite Expressionskassette verwendet werden.

Als vom Östrogenrezeptor abhängige Regulatorelemente, auch als Verstärkungselemente zu bezeichnen, werden vorzugsweise eine oder zwei *ERE*-Sequenzen eingesetzt. Vorzugsweise werden 1 bis 3, insbesondere zwei *ERE*-Sequenzen in den Promotor der zweiten Expressionskassette integriert, vorzugsweise in Positionen im Bereich von -100 bis -250, gerechnet vom Translationsstart des *phyK*-Gens. Für den *GAA* - Promotor sind die Positionen um -107, -150 und -203, insbesondere um -107, mit einem Bereich von +/- 20 Nukleotiden, insbesondere in 5' vor den Nukleotiden der genannten Positionen, gerechnet vom Translationsstart des *phyK*-Gens, bevorzugte Integrationsstelle für die vom Östrogenrezeptor in Gegenwart von Östrogen aktivierbaren ERE-Sequenzen.

Die dritte Expressionskassette enthält ein eine Auxotrophie des zu transformierenden Hefestammes komplementierendes Strukturgen. Da der in diesem Beispiel eingesetzte *Arxula adeninivorans* G1212 eine Tryptophan-Auxotrophie aufweist, wird diese Auxotrophie durch das *ATRP1-Gen* komplementiert. Erfindungsgemäß ist vorgesehen, dass die dritte Expressionskassette, die die für die mehrfache Integration vorgesehene erste und zweite Expressionskassette enthält, einen Promotor mit im Vergleich zum natürlichen Promotor, dessen natürliche Form das die Auxotrophie verursachende Gen reguliert, stark verminderter Aktivität aufweist, z.B. mit stark verminderter Transkriptionsrate. Vorliegend ist das die Auxotrophie komplementierende Strukturgen unter der Kontrolle eines veränderten Promotors des *ALEU2-Gens,* der Deletionen aufweist und auf 53bp verkützt ist, die seine Aktivität auf ca. 10 bis 30%, besonders bevorzugt auf maximal 20% vermindern.

In Figur 1 ist dargestellt, dass die erste, zweite und dritte Expressionskassette jeweils in einfacher Kopie in einem Nukleinsäurekonstrukt vorliegen. Es ist jedoch auch möglich, in diesem ersten Nukleinsäurekonstrukt zwei oder mehr Kopien der ersten und/oder zweiten Expressionskassette anzuordnen.

Die erste, zweite und dritte Expressionskassette sind auf dem erfindungsgemäßen ersten Nukleinsäurekonstrukt zwischen zwei DNA - Abschnitten angeordnet, die eine ausreichende Homologie zu natürlichen Sequenzen des Hefegenoms aufweisen, so dass eine Integration des vollständigen Nukleinsäurekonstrukts zwischen den homologen DNA - Abschnitten in die entsprechend homologen genomischen DNA - Abschnitte möglich ist. Vorzugsweise sind die homologen Abschnitte zu einem Bereich der 25S oder 18S rDNA homolog.

Bei Transformation von Hefe mit dem erfindungsgemäßen ersten Nukleinsäurekonstrukt erfolgt eine Anreicherung und ein Screening von Transformanten mit multipler Kopienzahl durch Kultivierung in Minimalmedium und nachfolgende Plattierung auf Minimalmedium-Platten. Dabei ergeben Transformanten mit multipler Integration nach 3 bis 4 Tagen Inkubation unter Wachstumsbedingungen bei 30 °C isolierbare Kolonien, die auf Grund erhöhter Produktion des Reporters identifizierbar sind. Im Unterschied dazu ergeben primäre Transformanten mit nur einer Kopie der Expressionskassette erst nach Wachstum von 10 bis 30 Tagen optisch sichtbare Kolonien. Die ein- oder beidseitig die Expressionskassetten flankierenden Sequenzabschnitte der 25S oder 18 S rDNA sind ausreichend lang, um in der Hefe die homologe Rekombination ablaufen zu lassen, so dass die Transformanten die aufgenommene DNA als in das Genom integrierte Kopie aufweisen. Wegen des Fehlens genetischer Elemente, die für die Aufrechterhaltung von Nukleinsäure in Form eines Plasmids in Hefe erforderlich sind, weisen Transformanten die aufgenommene DNA als in das Genom integrierte Kopie auf. Während es vorteilhaft ist, für die Herstellung des erfindungsgemäßen Nukleinsäurekonstrukts bakterielle Plasmide zu nutzen, die beispielsweise in Figur 1 mit Replikationsursprung (f1 origin, ColE1 ori) und einer Kanamycin-Resistenz (kan (R)) dargestellt sind, kann das erfindungsgemäße Nukleinsäurekonstrukt linearisiert sein und allein die erfindungsgemäß erforderlichen Expressionskassetten, vorzugsweise zwischen jeweils terminal angeordneten homologen rDNA - Abschnitten angeordnet, aufweisen.

Zur Transformation von *Arxula adeninivorans* wurde das in Figur 1 dargestellte Plasmid mit Restriktionsenzymen zweifach geschnitten und das erfindungsgemäße Nukleinsäurekonstrukt der drei Expressionskassetten einschließlich der terminal flankierenden, zur 25S rDNA homologen Abschnitte isoliert, das als Seq. ID Nr. 1 gezeigt ist, so dass vorteilhafterweise keine Plasmid-DNA bakteriellen Ursprungs integriert wurde. Die Transformation von *Arxula adeninivorans* erfolgte nach Standardverfahren.

Im Anschluss an die Transformation wurden die Zellen zunächst für 24 h in Komplettmedium kultiviert, anschließend in Minimalmedium, das keine Tryptophanquelle enthielt. Nach Vereinzelung von Transformanten durch Ausplattieren auf Minimalmedium wurden einzelne Transformanten in einem zweiten Schritt mit dem erfindungsgemäß einzusetzenden zweiten Nukleinsäurekonstrukt transformiert, nämlich enthaltend eine Expressionskassette, die das die Auxotrophie komplementierende Strukturgen unter der Kontrolle des Promotors mit einer der natürlichen Aktivität entsprechenden Aktivität aufweist. Nachdem vorliegend das zur Herstellung der Prototrophie zu komplementierende Strukturgen *ATRP1* war, wies das zweite Nukleinsäurekonstrukt das *ATRP1* - Gen unter der Kontrolle eines unveränderten Promotors *ALEU2* oder alternativ des natürlichen *ATRP1* - Promotors auf. Die Transformation erfolgte wiederum nach Standardverfahren mit einem linearisierten Nukleinsäurekonstrukt, das außer der Expressionskassette mit dem komplementierenden Strukturgen, der Rezeptorgen-Expressionskassette und der Reportergen-Expressionskassette keine weiteren funktionalen Elemente aufwies. Zur Selektion von Transformanten wurde wiederum in Minimalmedium kultiviert.

Bei einem Vergleich der Transformanten, die nur das erste Nukleinsäurekonstrukt enthielten, mit Transformanten, die zusätzlich das zweite Nukleinsäurekonstrukt enthielten, konnte festgestellt werden, dass nur Transformanten, die zusätzlich zu dem ersten Nukleinsäurekonstrukt auch das zweite Nukleinsäurekonstrukt aufwiesen, d.h. bei denen das komplementierende Strukturgen auch unter der Kontrolle eines Promotors mit unverminderter Aktivität enthalten war, mitotisch hoch stabil waren.

Das zweite Nukleinsäurekonstrukt ist in der Seq. ID Nr. 2 als Leserahmen *ATRP1* unter der Kontrolle eines nicht in seiner Aktivität beeinträchtigten *ALEU2*-Promotors enthalten und konnte daraus isoliert bzw. spezifisch durch PCR amplifiziert und für die zweite Transformation eingesetzt werden.

Bei Transformation von *Arxula adeninivorans* mit dem Nukleinsäurefragment von Seq. ID Nr. 2 (Zuordnung der regulatorischen Elemente und Orientierung der Leserahmen wie in Fig. 1), das das Selektionsmarkergen unter der Kontrolle eines Promotors mit unverminderter Aktivität enthält, konnten ebenfalls Transformanten isoliert werden. Diese Transformanten enthielten jedoch zumindest nach Kultivierung über mehrere Generationen nur eine geringe Kopienzahl der ersten und zweiten Expressionskassette und werden daher gegenwärtig als Vergleich zu erfindungsgemäßen Transformanten eingestuft.

Insbesondere als regulierbarer Promotor bevorzugt für *A. adeninivorans* ist der Glucoamylasepromotor (*GAA*), dessen Nukleotidsequenz als Seq ID Nr. 3 angegeben ist, sowie mit 2 aneinander angrenzenden, in 5' vor Nukleotidposition -107 vom Translationsstart gerechnet, inserierten RE, beispielhaft dargestellt von den *HRE*-Sequenzen, als Seq ID Nr. 4 angegeben.

Ein in Alternative zum Östrogenrezeptorgen verwendbareres Hormonrezeptorgen ist das Gen des humanen Progesteron-Rezeptors als Seq ID Nr. 5 angegeben, bei dem die humane Sequenz durch Anpassung der Kodons an den Kodongebrauch von *Arxula* für die Expression in *Arxula* optimiert ist. Die kodierte Aminosäuresequenz, in Seq ID Nr. 6 dargestellt, ist unverändert die humane Sequenz. Entsprechend zeigt der heterolog in der manipulierten Hefe exprimierte Hormonrezeptor dieselbe Reaktion auf die spezifische Bindung von Hormonen und von Substanzen mit entsprechender Hormonwirkung, wie der natürliche humane Rezeptor.

Ein weiteres Gen für einen erfindungsgemäß verwendbaren Hormonrezeptor ist der Androgen-Rezeptor, dessen natürliche humane Aminosäuresequenz als Seq ID Nr. 8 gezeigt ist, die kodierende Nukleinsäuresequenz als Seq ID Nr. 7, bei der wiederum der humane Kodongebrauch an den von *Arxula* angepasst ist.

Ein noch weiteres Gen für einen erfindungsgemäß verwendbaren Hormonrezeptor ist der Glucocorticoid-Rezeptor, dessen natürliche humane Aminosäuresequenz als Seq ID Nr. 10 gezeigt ist, die kodierende Nukleinsäuresequenz als Seq ID Nr. 9, bei der wiederum der humane Kodongebrauch an den von *Arxula* angepasst ist.

Entsprechend einer Ausführungsform der Erfindung kann die transformierte erfindungsgemäße Hefe als Bestandteil eines sogenannten Testkits in Zusammenstellung mit den Reagenzien zum Nachweis eines Hormons oder von Substanzen mit Hormonwirkung enthalten sein. Vorzugsweise enthält ein Testkit zusätzlich zur erfindungsgemäßen Hefe eine Hefe, die den hormonspezifischen Rezeptor und/oder das RE nicht enthält, zur Aufnahme von Messwerten, z.B. als Hintergrundkontrolle, insbesondere zur Verwendung bei der Erzeugung von Messwerten und Berechnung einer Kalibrierkurve. Als Reagenzien in einem Testkit enthalten sind vorzugsweise das rezeptorspezifische Hormon oder eine Substanz mit bekannter Hormonwirkung für den Rezeptor, die einer Probe zur Dotierung um eine bekannte zusätzliche Konzentration an Hormonwirkung zugesetzt werden kann, Lösungen bekannter Hormonwirkungskonzentration zur Erzeugung einer Kalibrierkurve, und Substrat für das Reportergen.

<u>Beispiel 2: Optische Bestimmung von Verbindungen mit Östrogenwirkung in wässrigen Proben</u>

Nach Beispiel 1 hergestellte, genetisch manipulierte *Arxula adeninivorans* wurde im Mikrotiterplattentest mit östrogenhaltigen wässrigen Proben versetzt. Für die Messung konnten die Hefezellen an der inneren Oberfläche der Näpfe einer Mikrotiterplatte immobilisiert werden, beispielsweise durch Vorbehandeln der Oberfläche durch mechanische Aufrauhung und anschließende Kontaktierung der Hefezellen in Suspension für ca. 60 min, anschließendes Absaugen und Spülen der Näpfe mit Wasser. Für diese Art der Immobilisierung sind die Näpfe vorzugsweise aus Polypropylen. Alternativ zur oberflächlichen Immobilisierung wurden die Hefezellen zu $10^7$ Zellen/mL Endvolumen Reaktionsmischung vorgelegt. Weiter alternativ werden die Hefezellen durch Einschließen in eine Matrix immobilisiert, z.B. in PVA, erhältlich unter der Bezeichnung Lentikats von Genialab, Braunschweig, oder in Alginat. Vorzugsweise wurde die Probe vor Mischung mit Puffer für 10 min auf 100 °C erwärmt.

Die wässrige Probe wurde durch Puffersubstanzen auf pH 3,9 eingestellt, vorzugsweise in der folgenden wässrigen Pufferzusammensetzung: 0,5 M Na-Citrat, pH 3,9.

Die Inkubation mit der gepufferten wässrigen Probe erfolgte über eine Zeitdauer von 40 min bis 12 h bei 30 °C unter leichtem Schütteln. Die Detektion erfolgte durch Zugabe des Substrats für den Reporter, im vorliegenden Fall p-Nitrophenylphosphat, das durch die Phytase umgesetzt wird. Die Reaktion konnte durch Zugabe von Trichloressigsäure zu 15% Endkonzentration abgestoppt werden. Nach Zugabe von Natronlauge auf eine Endkonzentration von 500 mM wurde die erzeugte Gelbfärbung photometrisch bei 405 nm gemessen.

Als Probe wurde Abwasser vom Kläranlageneinlauf einer kommunalen Kläranlage verwendet. Für den Parameter $\theta$ wurde ein Wert von 0,05 bestimmt. Die Messreihen 1 bis 4 ergaben für die Parameter A, B, C und D die folgenden Werte:

| | Messreihe | | | |
|---|---|---|---|---|
| Parameter | 1 | 2 | 3 | 4 |

(fortgesetzt)

| | sigmoidales Modell für λ | | | |
|---|---|---|---|---|
| a | 1 | 1 | 1 | 1 |
| b | 0,28 | 1,07 | 3,86 | 1,87 |
| c | 0,40 | 0,085 | 0,070 | 0,060 |
| d | 0 | 0,37 | 0,51 | 0,36 |
| | | | | |
| | sigmoidales Modell für Messwerte (opt. Dichte) | | | |
| A | 0,10 | 0,10 | 0,10 | 0,11 |
| B | 1,48 | 1,68 | 1,43 | 1,65 |
| C | 87,7 | 86,2 | 82,7 | 64,7 |
| D | 1,75 | 1,75 | 1,75 | 1,75 |
| RSD | 0,1308 | 0,0706 | 0,0921 | 0,1271 |

Aus den Messergebnissen wurden die folgenden Konzentrationen an Substanzen mit Östrogenwirkung bestimmt:

| Messreihe | Konzentration an Substanzen mit Östrogenwirkung [als 17β-Estradiol (ng/L)] |
|---|---|
| 1 | 64,95 |
| 2 | 72,80 |
| 3 | 43,87 |
| 4 | 57,47 |

Für Wasser, z.B. Reinstwasser wurden untere Nachweisgrenzen von 1 ng/L, bevorzugt 0,5 ng/L gefunden, jeweils angegeben als 17β-Estradiol. Für Kläranlageneinläufe wurden untere Nachweisgrenzen von ca. 8 ng/L (als 17β-Estradiol) bei einem Inhibierungsfaktor von 1,95 gefunden, bei einem Inhibierungsfaktor von 2,30 von ca. 19,2 ng/L (als 17β-Estradiol), bei einem Inhibierungsfaktor von 2,40 und 2,72 von ca. 16,8 bzw. 21,9 ng/L (als 17β-Estradiol).

Vorzugsweise werden die Proben vor einer Messung für 10 min auf 100 °C erwärmt, abgekühlt und mit Pufferkonzentrat zu einer Endkonzentration von 125 mM zu pH 3,9 versetzt.

Nach einer Messdauer von 60 min bei Temperierung auf 30 °C bzw. nach Erwärmung auf 95°C bis 100 °C für 60 s wurden für 17β-Estradiol in Wasser bzw. in realen Abwasserproben eine Erhöhung der Messgenauigkeit gefunden. So zeigten die in erwärmten und erwärmten und dotierten Proben eine geringere Schwankungsbreite der Messergebnisse. Diese verbesserte Messgenauigkeit bzw. verbesserte Reproduzierbarkeit der Messung wird gegenwärtig darauf zurückgeführt, dass durch die Erwärmung der Einfluß andere Inhaltsstoffe der Proben vermindert werden, z.B. der Einfluß von Mikroorganismen.

Vorzugsweise umfasst das Messverfahren neben der Messung der Probe zumindest die Messung einer zusätzlich mit Wasser verdünnten Probe in demselben Puffer, z.B. mit Zusatz eines gleichen Volumens Wasser und/oder die Dotierung der interessierenden Probe mit einer bekannten Östrogenmenge.

Bei der vorliegenden optischen Detektion des Reporters, bzw. des vom Reporter aus dem Substrat erzeugten optischen Signals, ist es bevorzugt, optische Effekte durch nicht von Östrogen ausgelöste Signale zu eliminieren. Dazu wurden Vergleichsversuche mit einer nach Beispiel 1 unter ansonsten identischen Bedingungen transformierten Hefe durchgeführt, bei der das erste Nukleinsäurekonstrukt nicht die erfindungsgemäße erste Expressionskassette aufwies. Auf diese Weise konnten Hintergrundeffekte durch die Zellen selbst und ein unspezifisches Detektorsignal aus dem Messwert der Probe herausgerechnet werden.

Für Östrogen in Reinstwasser ergeben sich Nachweisgrenzen von 5,4 bis 8,4 ng/L, für Proben aus dem Zulauf einer Kläranlage Nachweisgrenzen von 10,5 bis 21,9 ng/L bezogen auf 17β-Estradiol. Aufgrund der Messungen von dotierten Proben und verdünnten Proben ließ sich ein Inhibierungsmittel von 1,95 bzw. 2,72 errechnen, das die Kontaminationen im Abwasser widerspiegelt.

Für die Auswertung von Messergebnissen für erwärmte Proben wird dasselbe Berechnungsverfahren zur Bestimmung der Konzentration von Substanzen mit Östrogenwirkung verwendet, wie für nicht zusätzlich erwärmte Proben.

Bei Austausch des Östrogenrezeptorgens durch eine Nukleinsäure, die einen alternativen Hormonrezeptor kodiert, z.B. den humanen Progesteronrezeptor, kodiert durch Seq ID Nr. 5, den humanen Androgenrezeptor, kodiert durch Seq ID Nr. 7, oder den humanen Glucocorticoidrezeptor, kodiert durch Seq ID Nr. 9, konnten die rezeptorspezifischen Analyten, im Beispiel die jeweiligen Hormone und Substanzen mit jeweiliger Hormonwirkung bestimmt werden. Die Expressionskassette für das Reportergen *phyK* wies funktional in 5' vor dem *phyK*-Gen angeordnet den GAA-Promotor mit in 5' vor dem Nukleotid -107, gerechnet vom Translationsstart an, zwei aneinander angrenzende rezeptorspezifische Response-Element auf, nämlich jeweils *HRE.* Der *GAA*-Promotor mit den in 5' vor Nukleotid -107 vor dem Translationsstart inserierten zwei *HRE* ist als Seq ID Nr. 4 gezeigt.

<u>Beispiel 3: Optische Bestimmung von Verbindungen mit Östrogenwirkung in wässrigen Proben mit fluoreszierendem Reporter</u>

In Abwandlung von Beispiel 1 wurde das erste Nukleinsäurekonstrukt mit der für GFP oder dsRed kodierenden Sequenz hergestellt und zur ansonsten identischen zweistufigen Transformation von *Arxula adeninivorans* eingesetzt.

Der Nachweis von Substanzen mit Östrogenwirkung in einer Probe erfolgte mit immobilisierten oder suspendierten Hefezellen, wobei zur Detektion die erzeugte Fluoreszenz nach jeweils gleicher Inkubationsdauer optisch bestimmt wurde. Die erhaltenen Messwerte entsprachen dem Signal-Östrogen-Verhältnis von Beispiel 2.

<u>Beispiel 4: Biosensor mit amperometrischer Messeinrichtung</u>

Nach Beispiel 2 hergestellte Hefezellen mit etwa 50 Kopien des schematisch in Figur 1 dargestellten ersten Nukleinsäurekonstrukts wurden zur amperometrischen Messung oberflächlich in Reaktionsgefäßen einer Messvorrichtung, die auch als Biosensor bezeichnet wird, immobilisiert. Der Biosensor ist schematisch in Figur 2 gezeigt, und weist in einem Reaktionsgefäß R1 Hefezellen 2 auf, die immobilisiert sind. Die Immobilisierung ist optional. Alternativ zur oberflächlichen Immobilisierung in einem Reaktionsgefäß können die Hefezellen in einer Matrix immobilisiert sein, z.B. in Alginat oder PVA.

In jedem Reaktionsgefäß R1 ist eine Elektrode 3 vorzugsweise seitlich angeordnet, deren Elektrodenkopf 4 durch den Deckel 5 geführt ist, um in einem Elektrodenanschluss 6 zu münden. Der Biosensor weist zur Befüllung der Reaktionsgefäße 1 zumindest jeweils eine Kanüle als Zulauf 7 auf, der vorzugsweise bis zum Boden des Reaktionsgefäßes 1 geht. Ein Thermostat 10 steuert die Temperatur der Reaktionsgefäße 1.

Zur amperometrischen Messung wird der Stromfluss an der Elektrode gemessen, die aus einer Arbeitselektrode, einer Gegenelektrode und einer Referenzelektrode besteht. Die Messungen finden vorzugsweise parallel in zwei gleich gebauten Reaktionsgefäßen statt, wobei in dem einen die erfindungsgemäße Hefe immobilisiert ist, die auf Östrogenwirksame Substanzen reagieren, und in dem anderen Reaktionsgefäß Hefe vorliegt, die mit Ausnahme der hER$\alpha$ kodierenden ersten Expressionskassette identisch genetisch verändert ist. Das letztgenannte Reaktionsgefäß dient wegen des Fehlens der Expressionskassette, die für den Östrogenrezeptor kodiert, zur Vergleichsmessung, um unspezifisches Messsignal aufzunehmen.

Für die Auswertung von Messergebnissen der Proben ist es bevorzugt, die Messergebnisse entsprechend Beispiel 2 umzurechnen, um die Beeinträchtigung der Messgenauigkeit durch Verunreinigungen der Probe mathematisch zu eliminieren.

Figur 3 zeigt schematisch die möglichen Fluidströme einer Ausführungsform eines erfindungsgemäßen Biosensors, der jeweils Vorratsbehälter für Probe, Wasser, zweifach (2x) konzentrierten Puffer, Kalibrierstandard (Standard) mit vorbekannter Konzentration einer Substanz mit Östrogenwirkung, z.B. 17$\beta$-Östradiol, Substrat für Reporter sowie für die zu analysierende Probe bzw. (Elektroden-) Testlösung aufweist. Die Vorratsbehälter sind jeweils durch Leitungen, in denen steuerbare Ventile V1 bis V6 vor den Pumpen 1 und 2 angeordnet sind, steuerbar alternativ über eine erste Leitung L1 mit einem ersten Reaktionsgefäß (R1) und über eine zweite Leitung mit einem zweiten Reaktionsgefäß (R2) verbindbar. Vorzugsweise sind die von den Vorratsbehältern abgehenden Leitungen an die erste Leitung L1 und die zweite parallele Leitung L2 anschließbar, in der ein Ventil V7 bzw. V9 vor den Pumpen P1 bzw. P2 angeordnet ist, und Ventile V7 bzw. V9 nach der Pumpe P1 bzw. P2. Die Pumpen P1 und P2 können als Kanäle einer Pumpe ausgestaltet sein.

In der erfindungsgemäßen Sensoranordnung können Probe, Puffer, Substrat und als Verdünner bzw. zur Pufferung für die Probe 1x Puffer oder Wasser bzw. 2x Puffer in das erste Reaktionsgefäß und das zweite Reaktionsgefäß dosiert werden, von denen eines zur Messung mit einer erfindungsgemäßen Hefe einsetzbar ist, das andere zur Kontrollmessung unspezifischen Signals mit einem Kontrollklon, der z.B. keinen funktionalen Östrogenrezeptor exprimiert. Da die Vorratsbehälter mit der parallelen ersten und zweiten Leitung L1, L2 verbunden sind, können alle Lösungen gesteuert mittels Pumpen P1, P2 und dosiert durch die Ventile V1 bis V6 über die Leitungen L1 bzw. L2 in die Reaktionsgefäße transportiert werden. Für das Mischen kann eine Rücklaufleitung LR1 bzw. LR2 vorgesehen sein, die das erste Reaktionsgefäß R1 bzw. das zweite Reaktionsgefäß R2 mittels des Ventils V7 bzw. V9 mit der Leitung L1 bzw. L2 verbindet. Bei entsprechender Stellung des Ventils V7 bzw. V9 kann die Rücklaufleitung LR1 bzw. LR2 zum Umpumpen und Mischen von Lösungen aus den Reaktionsgefäßen R1 bzw. R2 eingesetzt werden. Aus den Reaktionsgefäßen R1, R2 kann nach Ende der Messung Lösung abgepumpt und verworfen werden, z.B. mittels eines schematisch dargestellten Leitung L4 bzw. L5, die über ein Ventil V8 bzw. V10 aus der Leitung L1 bzw. L2 zugänglich ist. Bei dieser Ausführungsform ist die Rücklaufleitung LR1 bzw. LR2 zur Spülung der Leitungen L1, L2, LR1, LR2 bzw. der Reaktionsgefäße R1, R2 und zum Mischen mittels der Pumpe P1, P2 vorgesehen und z.B. an das Dreiwegeventil V7 bzw. V9 angeschlossen, so dass die Pumpe als Mischorgan verwendbar ist. Vorteilhafterweise kann die Pumpe P1 und P2 als parallel angeordnete oder parallel geschaltete Pumpenköpfe aufweisen, z.B. als parallele Pumpenköpfe einer Schlauchpumpe.

Eine Fortbildung der Ausführungsform von Figur 3 ist in Figur 4 gezeigt und weist einen langen Schlauch S1 bzw. S2 auf, der als Aufnahmevolumen zum Mischen von Reaktionslösung und, optional, als Ablauf zum Verwerfen dient. Dieser lange Schlauch ist als Aufnahmevolumen für das Mischen vorgesehen, wenn nämlich Reaktionsmischung vor der Messung in ein Reaktionsgefäß R1, R2 dosiert wurde und diese Mischung in den Schlauch S1, S2 gepumpt und anschließend in das jeweilige Reaktionsgefäß R1, R2 zurück gepumpt wird. Entsprechend hat der Schlauch S1, S2 in dieser Ausführungsform zumindest das innere Volumen, das von der Reaktionsmischung in einem Reaktionsgefäß R1, R2 eingenommen wird, z.B. dasselbe Volumen wie ein Reaktionsgefäß R1, R2. Die Ventile V8 bzw. V10 dienen zur Steuerung der Befüllung der Reaktionsgefäße bzw. zum Verwerfen (Abfall) von Reagenzien. Die Ventile V7 bzw. V9 dienen bei Umkehr der Pumprichtung der Pumpe P1 bzw. P2 zum Befüllen des langen Schlauchs S1, S2 und, nach erneuter Umkehr der Pumprichtung, zum Befüllen der Reaktionsgefäße R1, R2, um die Komponenten in den Reaktionsgefäßen R1, R2 zu mischen. Wenn der Schlauch S1, S2 zusätzlich zum Verwerfen von Reaktionslösung dient, wie in Figur 4 angedeutet, kann die Leitung L4 bzw. L5 entfallen. Für die Entnahme von Reaktionsmischung aus den Reaktionsgefäßen R1, R2 mittels der Zuführleitung L1 bzw. L2 ist deren Öffnung am Boden der Reaktionsgefäße R1, R2 angeordnet.

<u>Beispiel 5: Berechnung der Vertrauensgrenzen der Hormonwirkungskonzentration auf Basis amperometrischer Messwerte</u>

Mit einer Messvorrichtung nach Beispiel 4, die einen Rechner auwies, der zur Durchführung des erfindungsgemäßen Berechnungsverfahrens eingerichtet war, wurden Verdünnungen und Dotierungen einer Probe aus einem Kläranlagenzulauf nach der folgenden Tabelle hergestellt. Die Berechnung der Inhibierungsfaktoren, der Östrogenkonzentration, der relativen Messunsicherheit und der Vertrauensgrenzen erfolgte nach den Berechnungsschritten für die Hormonwirkungskonzentration nach dem Bootstrap-Verfahren, so dass sich für jede der erzeugten Kalibrier- und Probenmesswerte andere Werte für die Messunsicherheit und das Vertrauensintervall ergeben.

Verdünnungen und Dotierungen einer Probe und aus den Messwerten berechnete Werte:

| Verdünnungsfaktor der originalen Probe mit Wasser | Dotierung [ng/L] | Inhibierungsfaktor | ermittelte Östrogenkonzentration [ng/L] | Relative Messunsicherheit | Vertrauensgrenzen der Östrogenkonzentration [ng/L] | |
|---|---|---|---|---|---|---|
| | | | | | Untergrenze | Obergrenze |
| 0,72 | 20 | 0,38 | 34 | 29% | 20 | 59 |
| 0,72 | 40 | 0,44 | 38 | 21% | 24 | 56 |
| 0,72 | 60 | 0,54 | 46 | 19% | 30 | 65 |
| 0,54 | 20 | 0,47 | 39 | 39% | 15 | 71 |
| 0,54 | 40 | 0,45 | 38 | 28% | 18 | 56 |
| 0,54 | 60 | 0,53 | 44 | 25% | 23 | 64 |
| 0,36 | 20 | 0,44 | 57 | 38% | 22 | 97 |
| 0,36 | 40 | 0,50 | 64 | 28% | 27 | 95 |
| 0,36 | 60 | 0,44 | 57 | 24% | 25 | 77 |

Die kleinste Messunsicherheit ergibt sich mit dem Verdünnungsfaktor 0,72 und einer Dotierung von 60 ng/L. In diesem Falle liegt die Messunsicherheit bei 19% und die Vertrauensgrenzen bei 30-65 ng/L. Dies ist - auch im Vergleich zu sehr aufwändigen alternativen Messmethoden, z.B. LC/MS-MS - ein sehr genauer Wert. Hingegen ist bei einer Verdünnung von 0,54 und einer Dotierung von 20 ng/L eine weitaus schlechtere Messunsicherheit in Höhe von 29% festzustellen. In diesem Experiment wurden auch noch Messungen mit stärkeren Verdünnungen durchgeführt. Dabei zeigten sich noch deutlich höhere Messunsicherheiten und Vertrauensintervalle von 0 bis über 100 ng/L. Daraus ergibt sich, dass bei noch stärkerer Verdünnung unter Umständen keine valide Messung mehr möglich ist. Die aus solchen Verdünnungen resultierenden Messergebnisse sind dann als zu unsicher zu verwerfen.

Bezugszeichenliste:

| | |
|---|---|
| R1 erstes Reaktionsgefäß | V5 steuerbares Ventil |
| R2 zweites Reaktionsgefäß | V6 steuerbares Ventil |
| 2 Hefezellen | V7 steuerbares Dreiwegeventil |
| 3 Elektrode | V8 steuerbares Dreiwegeventil |
| 4 Elektrodenkopf | V9 steuerbares Dreiwegeventil |
| 5 Deckel | V10 steuerbares Dreiwegeventil |
| 6 Elektrodenanschluss | L1 erste Leitung |
| 7 Zulauf | L2 zweite Leitung |
| 10 Thermostat | L4 Leitung |
| V steuerbares Ventil | L5 Leitung |
| V2 steuerbares Ventil | LR1 erste Rücklaufleitung |
| V3 steuerbares Ventil | LR2 zweite Rücklaufleitung |
| V4 steuerbares Ventil | S1 erster Schlauch |
| | S2 zweiter Schlauch |

## Patentansprüche

1. Hefezelle der Gattung *Arxula,* die eine komplementierbare Auxotrophie aufweist und ein in das Genom integriertes erstes lineares Nukleinsäurekonstrukt mit einer ersten Expressionskassette, die eine einen Hormonrezeptor kodierende Nukleinsäuresequenz aufweist, mit einer zweiten Expressionskassette, die ein Reportergen unter der Kontrolle eines von zumindest einem für den Hormonrezeptor spezifischen Response-Element-Abschnitt regulierten Promotors aufweist, wobei das erste Nukleinsäurekonstrukt an beiden Enden zum Hefegenom homologe Bereiche der 25S rDNA aufweist,
das erste Nukleinsäurekonstrukt eine dritte Expressionskassette umfasst, die ein die Auxotrophie komplementierendes Strukturgen mit Wildtypsequenz des genomischen Defektgens aufweist,
wobei der Promotor der dritten Expressionskassette eine auf maximal 75% der Aktivität des natürlichen Promotors des Strukturgens mit Wildtypsequenz des genomischen Defektgens verminderte Aktivität hat,
wobei der kodierte Hormonrezeptor ein Östrogenrezeptor, hERα und/oder hERβ, ein Progesteronrezeptor, ein Androgenrezeptor und/oder ein Glucocorticoidrezeptor ist, **dadurch gekennzeichnet, dass**
die Hefezelle ein zweites Nukleinsäurekonstrukt mit zumindest einer Expressionskassette des die Auxotrophie komplementierenden Strukturgens mit Wildtypsequenz des genomischen Defektgens unter der Kontrolle eines Promotors mit gegenüber dem natürlichen Promotor unveränderten Aktivität enthält und
eine Mehrzahl von Kopien des ersten Nukleinsäurekonstrukts in das Genom integriert ist.

2. Hefezelle nach Anspruch 1, **dadurch gekennzeichnet, dass** das erste Nukleinsäurekonstrukt zwei oder mehr Kopien der ersten und/oder zweiten Expressionskassette enthält.

3. Hefezelle nach Anspruch 1, **dadurch gekennzeichnet, dass** der Hormonrezeptor von einer der Nukleinsäuresequenzen Seq ID Nr. 1, Nr. 5, Nr. 7 oder Nr. 9 kodiert ist.

4. Hefezelle nach einem der voranstehenden Ansprüche, **dadurch gekennzeichnet, dass** das Reportergen aus der Gruppe von Genen ausgewählt ist, die fluoreszierende Proteine, Enzyme, die ein Substrat zu einem optisch detektierbaren Produkt umsetzen, und Enzyme, die ein Substrat zu einem amperometrisch oder voltmetrisch detektierbaren Produkt umsetzen, kodieren, umfaßt.

5. Testkit zum Nachweis von Stoffen mit Hormonwirkung, das eine Zusammenstellung einer Hefezelle nach einem der voranstehenden Ansprüche mit zumindest einer Lösung bekannter Konzentration an Stoff mit Hormonwirkung und eine Hefezelle umfasst, die keinen Hormonrezeptorgen und/oder kein Response-Element (RE) aufweist.

6. Verfahren zur Herstellung einer Hefezelle nach einem der Ansprüche 1 bis 4 mit einem ersten genomisch integrierten ersten Nukleinsäurekonstrukt mit den Schritten der Transformation einer auxotrophen Hefezelle mit einem ersten Nukleinsäurekonstrukt, das an beiden Enden zum Hefegenom homologe Bereiche der 25S rDNA aufweist und eine erste Expressionskassette, die eine einen Hormonrezeptor kodierende Nukleinsäuresequenz aufweist, wobei der kodierte Hormonrezeptor ein Östrogenrezeptor, hERα und/oder hERβ, ein Progesteronrezeptor, ein Androgenrezeptor und/oder ein Glucocorticoidrezeptor ist, und zweite Expressionskassette, die ein Reportergen unter der Kontrolle eines von zumindest einem für den Hormonrezeptor spezifischen Response-Element-Abschnitt regulierten Promotors aufweist, und eine dritte Expressionskassette mit einem die Auxotrophie komplementierenden Strukturgen mit Wildtypsequenz des genomischen Defektgens unter der Kontrolle eines Promotors, der eine gegenüber dem natürlichen Promotor dieses Strukturgens auf maximal 75% der Aktivität des natürlichen Promotors des Strukturgens mit Wildtypsequenz des genomischen Defektgens verminderte Aktivität aufweist, umfasst,
der Kultivierung der erhaltenen Transformanten in Medium, das kein die Auxotrophie komplementierendes Substrat enthält, und
der Transformation kultivierter Transformanten mit einem zweiten Nukleinsäurekonstrukt, das ein die Auxotrophie komplementierendes Strukturgen mit Wildtypsequenz des genomischen Defektgens unter der Kontrolle eines Promotors mit einer gegenüber dem natürlichen Promotor mindestens gleichen Aktivität enthält.

7. Messvorrichtung, **gekennzeichnet durch** einen mit einer Hefezelle nach einem der Ansprüche 1 bis 4 versehenen Transducer zur Aufnahme von stoffspezifischen Messwerten, der mit einem Rechner zur Aufnahme verbindbar ist, und der Rechner eingerichtet ist, die Berechnungsschritte für die Messwerte durchzuführen:

Berechnung der auf 1 bis 4 freien Parametern (A, B, C, D) basierenden Kalibrierkurve, deren Messwerte für bekannte Hormonwirkungskonzentrationen bestimmt wurden, Berechnung des Inhibierungsfaktors sowie der Hormonwirkungskonzentration der unverdünnten Probe nach Inhibierungskorrektur,
Erzeugung von mindestens 10 simulierten und zufällig variierten Kalibriermesswerten, jeweils mit denselben Dotierungen und Verdünnungen wie für die tatsächlich durchgeführte Kalibriermessung,
Erzeugung von mindestens 100 simulierten und zufällig variierten Probenmessungen, jeweils mit stoffspezifischem Rezeptor, ohne Dotierung, mit Dotierung, sowie ohne stoffspezifischen Rezeptor, und
Berechnung der Messunsicherheit und des Vertrauensintervalls aus den Ergebnissen der in Schritt 2) erhaltenen Konzentration.

**8.** Messvorrichtung nach Anspruch 7 mit einem ersten Reaktionsgefäß (R1) zur Aufnahme von Hefezellen und mit einem zweiten Reaktionsgefäß (R2) zur Aufnahme von Hefezellen nach einem der Ansprüche 1 bis 7, ohne die erste Expressionskassette, wobei das erste und zweite Reaktionsgefäß (R1, R2) jeweils eine Elektrode (3) und zumindest einen Zulauf (7) aufweisen, wobei die Elektroden (3) von einer Messeinrichtung zur amperometrischen Detektion kontaktiert sind.

**9.** Messvorrichtung nach Anspruch 7 oder 8, **gekennzeichnet dadurch, dass** die Hefezellen (2) auf der inneren Oberfläche der Reaktionsgefäße (R1, R2) immobilisiert sind.

**10.** Messvorrichtung nach einem der Ansprüche 7 bis 9, **gekennzeichnet durch** eine Einrichtung zur Erwärmung einer Probe auf 75 °C bis 100 °C.

**11.** Verfahren zur Bestimmung des Gehalts einer Probe an rezeptorspezifischen Substanzen mit Verwendung einer Hefe nach einem der Ansprüche 1 bis 4, mit den Schritten der Messung von stoffspezifischen Messwerten mit den Schritten der Erzeugung von Messwerten für verdünnte und/oder dotierte Probe, **gekennzeichnet durch** die rechnergestützte Berechnung der Stoffkonzentration **durch** die Schritte Berechnung der auf 1 bis 4 freien Parametern (A, B, C, D) basierenden Kalibrierkurve, deren Messwerte für bekannte Hormonwirkungskonzentrationen bestimmt wurden, Berechnung des Inhibierungsfaktors sowie der Hormonwirkungskonzentration der unverdünnten Probe nach Inhibierungskorrektur,
Erzeugung von mindestens 10 simulierten und zufällig variierten Kalibriermesswerten, jeweils mit denselben Dotierungen und Verdünnungen wie für die tatsächlich durchgeführte Kalibriermessung,
Erzeugung von mindestens 100 simulierten und zufällig variierten Probenmessungen, jeweils mit stoffspezifischem Rezeptor, ohne Dotierung, mit Dotierung, sowie ohne stoffspezifischen Rezeptor, und
Berechnung der Messunsicherheit und des Vertrauensintervalls aus den Ergebnissen der in Schritt 2) erhaltenen Konzentration.

**12.** Verfahren nach Anspruch 11, **dadurch gekennzeichnet, dass** die stoffspezifischen Messwerte durch einen mit einem stoffspezifischem Rezeptor versehenen Transducer erzeugt werden.

**13.** Verfahren nach einem der Ansprüche 11 bis 12, **dadurch gekennzeichnet, dass** eine Probe für mindestens 30 s auf mindestens 75 °C erwärmt wird,
die Probe mit Puffer auf pH 3 bis 5 eingestellt wird und Messwerte durch optische Detektion aufgenommen werden.

**14.** Verfahren nach einem der Ansprüche 11 bis 13, **dadurch gekennzeichnet, dass** der Probe ein von dem Translationsprodukt des Reportergens zu einem amperometrisch detektierbaren Produkt umsetzbares Substrat zugegeben wird und die Detektion eine amperometrische Messung ist.

**15.** Verfahren nach einem der Ansprüche 11 bis 14, **dadurch gekennzeichnet, dass** für ein erstes Aliquot der Probe ein erster Messwert bestimmt wird,
für ein zweites Aliquot der Probe, das mit Wasser verdünnt wird, ein zweiter Messwert bestimmt wird, für ein drittes Aliquot der Probe nach Zugabe einer bekannten Konzentration einer Substanz mit Hormonwirkung bestimmt wird, und die Konzentration von Substanzen mit Hormonwirkung in dem ersten Aliquot der Probe durch Bestimmung der Parameter A, B, C und D mittels des Verfahrens der Kleinsten Quadrate für das zweite und dritte Aliquot durch Berechnung mit der Formel V:

$$\ln(y_{ij}) = \ln\left(\frac{A-D}{1+\left(\frac{x}{\lambda_i + C}\right)^B} + D + \theta \cdot q\right) + \varepsilon_{ij} \quad (V),$$

erfolgt, in der der Inhibierungsfaktor λ bestimmt ist gemäß Formel IV:

$$\lambda^{-1} = \frac{1-d}{1+\left(\frac{q}{c}\right)^b} + d \quad (IV),$$

in der q das Verdünnungsverhältnis der Probe ist und Parameter a, b, c und d durch sigmoidale Anpassung der logarithmierten Messwerte für Proben bei unterschiedlichen Verdünnungsverhältnissen ermittelt wurden, und θ die erwartete Zunahme der Messwerte ist, die nicht auf die Hormonwirkung zurückgehen.

**16.** Verfahren nach Anspruch 15, **dadurch gekennzeichnet, dass** zur Bestimmung der Parameter A, B, C und D Messwerte von Kalibriermessungen von Kalibratorlösung, die Wasser oder Puffer ist, und von zumindest einer Kalibratorlösung mit bekannter Konzentration von Substanz mit Hormonwirkung verwendet werden.

## Claims

**1.** Yeast cell of the genus *Arxula* having an auxotrophy to be complemented and having integrated into the genome a first linear nucleic acid construct having a first expression cassette comprising a nucleic acid sequence encoding a hormone receptor, a second expression cassette comprising a reporter gene under the control of a promoter regulated by at least one response element section specific for the hormone receptor, wherein the first nucleic acid construct at both ends comprises sections homologous to the yeast genome of the 25S rDNA,
the first nucleic acid construct comprising a third expression cassette, which comprises a structural gene complementing the auxotrophy and having wild-type sequence of the genomic defect gene,
wherein the promoter of the third expression cassette has an activity reduced to maximally 75% of the activity of the natural promoter of the structural gene having wild-type sequence of the genomic defect gene,
wherein the encoded hormone receptor is an oestrogen receptor, hERα and/or hERβ, a progesteron receptor, an androgen receptor and/or a glucocorticoid receptor, **characterized in that**
the yeast cell contains a second nucleic acid construct having at least an expression cassette of the structural gene complementing the auxotrophy and having wild-type sequence of the genomic defect gene under the control of a promoter of an activity unchanged in comparison to the natural promoter and a plurality of copies of the first nucleic acid construct is integrated into the genome.

**2.** Yeast cell according to claim 1, **characterized in that** the first nucleic acid construct contains two or more copies of the first and/or second expression cassette.

**3.** Yeast cell according to claim 1, **characterized in that** the hormon receptor is encoded by one of the nucleic acid sequences Seq ID No. 1, No. 5, No. 7 or No. 9.

**4.** Yeast cell according to one of the preceding claims, characterized that the reporter gene is selected from the group of genes comprising genes encoding fluorescent proteins, enzymes converting a substrate to an optically detectable product and enzymes converting a substrate to an amperometrically or voltamatrically detectable product.

**5.** Test kit for detection of compounds having hormone activity, which comprises a combination of a yeast cell according to one of the preceding claims with at least one solution of a known concentration of a compound having hormone activity and a yeast cell that contains no hormone receptor gene and/or no response element (RE).

**6.** Process for producing a yeast cell according to one of claims 1 to 4, having a first genomically integrated first nucleic acid construct with the steps of transforming an auxotrophic yeast cell with a first nucleic acid construct having at both ends sections homologous to the yeast genome of the 25S rDNA and a first expression cassette which comprises a nucleic acid sequence encoding a hormone receptor, wherein the encoded hormone receptor is an oestrogen receptor, hERα and/or hERβ, a progesteron receptor, an androgen receptor and/or a glucocorticoid receptor, and a second expression cassette comprising a reporter gene under the control of a promoter regulated by at least one response element section specific for the hormone receptor, and a third expression cassette having a structural gene complementing the auxotrophy and having wild-type sequence of the genomic defect gene under the control of a promoter which has a reduced activity of maximally 75% of the activity of the natural promoter of the structural gene having wild-type sequence of the genomic defect gene compared to the natural promoter of this structural gene, cultivating the transformants obtained in medium containing no substrate complementing the auxotrophy,
transforming cultivated transformants with a second nucleic acid construct that contains a structural gene complementing the auxotrophy and having wild-type sequence of the genomic defect gene under the control of a promoter having at least the same activity compared to the natural promoter.

**7.** Measuring device, **characterized by** a transducer provided with a yeast cell according to one of claims 1 to 4 for receiving compound specific measuring values, the transducer being connectable to a computer for reception, and the computer being disposed to perform the calculating steps for the measuring values:

calculating the calibration curve based on 1 to 4 free parameters (A, B, C, D), the measuring values of which are determined for known hormone effect concentrations,
calculating the inhibiting factor and the hormone activity concentration of the undiluted sample following inhibition correction,
generation of at least 10 simulated and randomly varied calibration measuring values, each with the same doping and dilutions as for the calibration measurement actually performed,
generation of at least 100 simulated and randomly varied sample measurements, each with a compound specific receptor, without doping, with doping, as well as without compound specific receptor and
calculating the measurement uncertainty and the confidence interval from the results of the concentrations obtained in step 2).

**8.** Measuring device according to claim 7, having a first reaction vessel (R1) for receiving yeast cells and having a second reaction vessel (R2) for receiving yeast cells according to one of claims 1 to 7, without the first expression cassette, wherein the first and the second reaction vessel (R1, R2) each comprise an electrode (3) and at least one inlet (7), wherein the electrodes (3) are contacted by a measuring device for amperometric detection.

**9.** Measuring device according to claim 7 or 8, **characterized in that** the yeast cells (2) are immobilized on the inner surface of the reaction vessels (R1, R2).

**10.** Measuring device according to one of claims 7 to 9, **characterized by** a device for heating a sample of 75 °C to 100 °C.

**11.** Process for determination of the content of receptor specific compounds of a sample compromising use of a yeast according to one of claims 1 to 4, having the steps of measuring compound specific measuring values using the steps of generating measuring values for diluted and/or doped sample, **characterized by** the computer supported calculation of the concentration of the compound by the steps of calculating the calibration curve based on 1 to 4 free parameters (A, B, C, D), the measurement values which are determined for known hormone activity concentrations, calculating the inhibition factor and the hormone effect concentration of the undiluted sample following inhibition correction,
generation of at least 10 simulated and randomly varied calibration measurement values, each with the same dopings and dilutions as for the calibration measurement actually performed,
generating at least 100 simulated and randomly varied sample measurements, each with a compound specific receptor, without doping, with doping, as well as without compound specific receptor and
calculating the measurement uncertainty and the confidence interval from the results of the concentration obtained in step 2).

**12.** Process according to claim 11, **characterized in that** the compound specific measurement values are generated by a transducer provided with a compound specific receptor.

13. Process according to one of claims 11 to 12, **characterized in that** the sample is heated for at least 30 s to at least 75 °C,
the sample is set to pH 3 to 5 using buffer, and the measurement values are received by optical detection.

14. Process according to one of claims 11 to 13, **characterized in that** a substrate is added to the sample, the substrate being convertible to an amperometrically detectable product by the translation product of the reporter gene, and the detection is an amperometric measurement.

15. Process according to one of claims 11 to 14, **characterized in that** a first measurement value is determined for a first aliquot of the sample,
a second measurement values is determined for a second aliquot of the sample diluted with water, for a third aliquot of the sample after addition of a known concentration of the compound having hormone activity is determined, and the concentration of compounds having a hormone effect in the first aliquot of the sample by determination of parameters A, B, C and D using the least squares procedure for the second and third aliquot by calculation using formula V:

$$\ln(y_{ij}) = \ln\left(\frac{A-D}{1+\left(\frac{x}{\lambda_i + C}\right)^B} + D + \theta \cdot q\right) + \varepsilon_{ij} \quad \text{(V)},$$

wherein the inhibition factor λ is determined according to formula IV:

$$\lambda^{-1} = \frac{1-d}{1+\left(\frac{q}{c}\right)^b} + d \quad \text{(IV)},$$

wherein q is the dilution relation of the sample and parameters a, b, c and d are determined by sigmoidal fit of the logarithmised measuring values for samples at different dilution relations, and θ is the expected increase of the measurement values which are not caused by the hormone effect.

16. Process according to claim 15, **characterized in that** for the determination of parameters A, B, C and D, measurement values of calibration measurements are used from calibrating solution, which is water or buffer, and from at least one calibrating solution of known concentration of the substance having a hormone effect.

## Revendications

1. Souche de levure de l'espèce *Arxula,* présentant une auxotrophie pouvant être complétée et un premier hybride d'acide nucléique linéaire intégré au génome avec une première cassette d'expression, présentant une séquence d'acide nucléique de codage de récepteur hormonal, avec une deuxième cassette d'expression, présentant un gène reporter sous le contrôle d'un promoteur régulé par au moins un fragment d'élément sensible spécifique au récepteur hormonal, où le premier hybride d'acide nucléique présente aux deux extrémités des zones de 25S rADN homologues au génome de levure,
le premier hybride d'acide nucléique comprend une troisième cassette d'expression, présentant un gène structurel complétant l'auxotrophie avec une séquence de type sauvage du gène défectueux génomique,

où le promoteur de la troisième cassette d'expression présente une activité réduite à 75% au maximum de l'activité du promoteur naturel du gène structurel avec une séquence de type sauvage du gène défectueux génomique, où le récepteur hormonal codé est un récepteur d'oestrogènes, hERα et/ou hERβ, un récepteur de progestérone, un récepteur d'androgènes et/ou un récepteur de glucocorticoïdes,
**caractérisé en ce que**
la souche de levure contient un second hybride d'acide nucléique avec au moins une cassette d'expression du gène structurel complétant l'auxotrophie avec une séquence de type sauvage du gène défectueux génomique sous le contrôle d'un promoteur avec une activité inchangée par rapport au promoteur naturel et une pluralité de copies du premier hybride d'acide nucléique est intégré au génome.

**2.** Souche de levure selon la revendication 1, **caractérisée en ce que** le premier hybride d'acide nucléique contient deux ou plusieurs copies de la première et/ou seconde cassette d'expression.

**3.** Souche de levure selon la revendication 1, **caractérisée en ce que** le récepteur hormonal est codé par l'une des séquences d'acide nucléique Séq ID No. 1, No. 5, No. 7 ou No. 9.

**4.** Souche de levure selon l'une des revendications précédentes, **caractérisée en ce que** le gène reporter est choisi parmi le groupe de gènes de codage, qui contient des protéines fluorescentes d'enzymes de conversion d'un substrat en produit détectable optiquement et d'enzymes de conversion d'un substrat en produit détectable par un ampèremètre ou un voltmètre.

**5.** Kit d'essai pour la détection de substances présentant un effet hormonal, test comprenant une composition de souche de levure selon l'une des revendications précédentes avec au moins une solution de concentration connue de substance présentant un effet hormonal et une souche de levure, sans gène récepteur hormonal et/ou élément sensible (RE).

**6.** Procédé de fabrication de souche de levure selon l'une quelconque des revendications 1 à 4, pourvue d'un premier hybride d'acide nucléique intégré génomiquement comprenant les phases de transformation d'une souche de levure auxotrophe munie d'un premier hybride d'acide nucléique, présentant aux deux extrémités des zones de 25S rADN homologues au génome de levure, et une première cassette d'expression, présentant une séquence d'acide nucléique de codage de récepteur hormonal, où le récepteur hormonal codé est un récepteur d'oestrogènes, hERα et/ou hERβ, un récepteur de progestérone, un récepteur d'androgènes et/ou un récepteur de glucocorticoïdes, et une deuxième cassette d'expression, présentant un gène reporter sous le contrôle d'un promoteur régulé par au moins un fragment d'élément sensible spécifique au récepteur hormonal, et contient une troisième cassette d'expression comprenant un gène structurel complétant l'auxotrophie avec une séquence de type sauvage du gène défectueux génomique sous le contrôle d'un promoteur, présente par rapport au promoteur naturel de ce gène structurel une activité réduite à 75% au maximum de l'activité du promoteur naturel du gène structurel avec une séquence de type sauvage du gène défectueux génomique,
phase de culture des transformants dans un milieu ne comprenant pas de substrat complétant l'auxotrophie, et phase de transformation de transformants cultivés avec un deuxième hybride d'acide nucléique, comprenant un gène structurel complétant l'auxotrophie avec une séquence de type sauvage du gène défectueux génomique sous le contrôle d'un promoteur avec une activité au moins identique par rapport au promoteur naturel.

**7.** Dispositif de mesure, **caractérisé par** un transducteur pourvu d'une souche de levure selon l'une des revendications 1 à 4, destiné à recevoir des valeurs de mesure spécifiques aux substances, que l'on peut connecter à un ordinateur destiné à recevoir les mesures, et l'ordinateur est configuré pour réaliser les phases de calcul pour les valeurs de mesure: calcul de la courbe d'étalonnage reposant sur 1 à 4 paramètres libres (A, B, C, D), dont on a défini les valeurs de mesure pour des concentrations connues des effets hormonaux, calcul du facteur d'inhibition de même que de la concentration des effets hormonaux de l'échantillon non-dilué après correction d'inhibition,
production d'au moins 10 valeurs de mesure d'étalonnage simulées et variées au hasard, à chaque fois avec les mêmes dopants et dilutions comme pour la mesure d'étalonnage effectivement réalisée,
production d'au moins 100 mesures d'échantillons simulées et variées au hasard,
à chaque fois avec un récepteur spécifique à la substance, sans dopant, avec dopant, de même que sans récepteur spécifique à la substance, et
calcul de l'incertitude de mesure et de l'intervalle de confiance à partir des résultats de la concentration obtenue dans la phase 2).

**8.** Dispositif de mesure selon la revendication 7 pourvue d'une première cuve de réaction (R1) permettant de recevoir des souches de levure et pourvue d'une seconde cuve de réaction (R2) permettant de recevoir des souches de levure selon l'une quelconque des revendications 1 à 7, sans la première cassette d'expression, où la première cuve de réaction et la seconde cuve de réaction (R1, R2) présentent chacune une électrode (3) et au moins une arrivée (7), où les électrodes (3) sont contactées par un dispositif de mesure pour une détection par ampèremètre.

**9.** Dispositif de mesure selon la revendication 7 ou 8, **caractérisé en ce que** les souches de levure (2) sont immobilisées à la surface interne des cuves de réaction (R1, R2).

**10.** Dispositif de mesure selon l'une des revendications 7 à 9, **caractérisé par** un dispositif permettant de chauffer un échantillon à 75°C -100 °C.

**11.** Procédé de détermination de la teneur d'un échantillon en substances spécifiques à un récepteur en utilisant une levure selon l'une des revendications 1 à 4, comprenant les phases de mesure de valeurs de mesure spécifiques aux substances comprenant les phases de production de valeurs de mesure pour échantillon dilué et/ou dopé, **caractérisé par** le calcul assisté par ordinateur de la concentration de substance par les phases de calcul de la courbe d'étalonnage reposant sur 1 à 4 paramètres libres (A, B, C, D), dont on a défini les valeurs de mesure pour des concentrations connues des effets hormonaux,
calcul du facteur d'inhibition de même que de la concentration des effets hormonaux du spécimen non-dilué après correction d'inhibition,
production d'au moins 10 valeurs de mesure d'étalonnage simulées et variées au hasard, à chaque fois avec les mêmes dopants et dilutions comme pour la mesure d'étalonnage effectivement réalisée,production d'au moins 100 mesures d'échantillons simulées et variées au hasard, à chaque fois avec un récepteur spécifique à la substance, sans dopant, avec dopant, de même que sans récepteur spécifique à la substance, et calcul de l'incertitude de mesure et de l'intervalle de confiance à partir des résultats de la concentration obtenue dans la phase 2).

**12.** Procédé selon la revendication 11, **caractérisé en ce que** les valeurs de mesure spécifiques aux substances sont produites par un transducteur muni d'un récepteur spécifique à la substance.

**13.** Procédé selon l'une des revendications 11 à 12, **caractérisé en ce qu'**un échantillon est chauffé pendant au moins 30 secondes à au moins 75°C,
l'on règle l'échantillon avec un tampon sur un pH de 3 à 5 et l'on enregistre les valeurs de mesure par détection optique.

**14.** Procédé selon l'une des revendications 11 à 13, **caractérisé en ce que** l'on ajoute à l'échantillon un substrat transformable par le produit de traduction du gène reporter, à un produit détectable par ampèremètre et la détection est une mesure par ampèremètre.

**15.** Procédé selon l'une des revendications 11 à 14, **caractérisé en ce qu'**on définit une première valeur de mesure pour une première aliquote de l'échantillon,
pour une seconde aliquote de l'échantillon, que l'on dilue avec de l'eau, on définit une deuxième valeur de mesure,
on définit une troisième aliquote après addition d'une concentration connue d'une substance aux effets hormonaux,
et l'on observe la concentration de substances aux effets hormonaux dans la première aliquote de l'échantillon par définition des paramètres A, B, C et D à l'aide de la méthode des moindres carrés pour la deuxième aliquote et la troisième aliquote par calcul à l'aide de la formule V:

$$\ln(y_{ij}) = \ln\left(\frac{A-D}{1+\left(\frac{x}{\lambda_i + C}\right)^{B}} + D + \theta \cdot q\right) + \varepsilon_{ij} \quad \text{(V)},$$

dans laquelle on définit le facteur d'inhibition selon la formule IV:

$$\lambda^{-1} = \frac{1-d}{1+\left(\frac{q}{c}\right)^{b}} + d \qquad \text{(IV)},$$

où q est le rapport de dilution de l'échantillon et l'on a déterminé les paramètres a, b, c, et d par adaptation sigmoïde des valeurs de mesure logarithmiques pour des échantillons à des rapports de dilution différentes, et $\theta$ est l'augmentation attendue des valeurs de mesure, qui ne sont pas dues à l'effet hormonal.

**16.** Procédé selon la revendication 15, **caractérisé en ce que** pour déterminer les paramètres A, B, C et D, on utilise des valeurs de mesures d'étalonnage de solution d'étalonnage, soit de l'eau ou un tampon, et d'au moins une solution d'étalonnage de concentration connue de substance aux effets hormonaux.

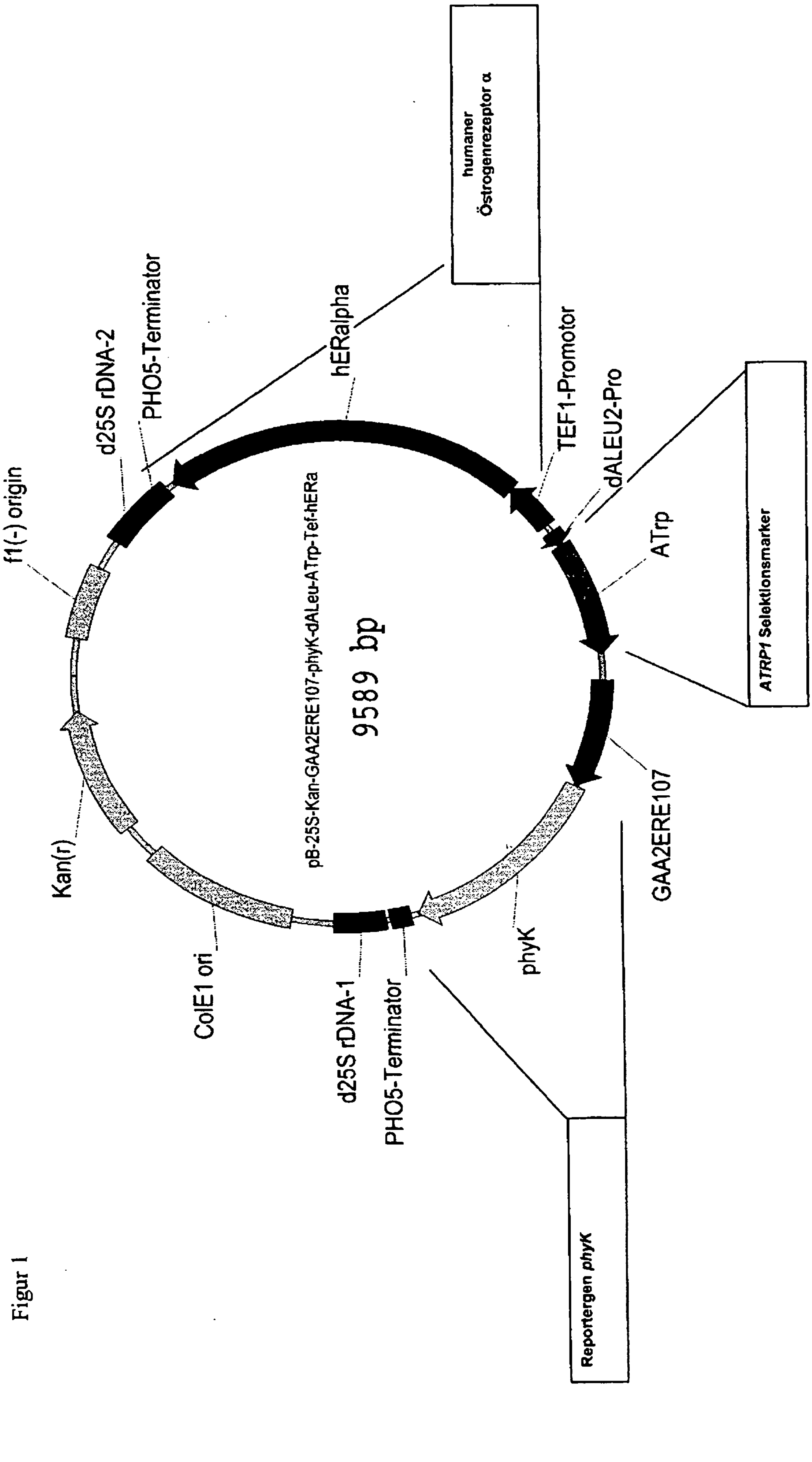
humaner Östrogenrezeptor α
f1(-) origin
d25S rDNA-2
PHO5-Terminator
hERalpha
TEF1-Promotor
dALEU2-Pro
ATrp
ATRP1 Selektionsmarker
pB-25S-Kan-GAA2ERE107-phyK-dALeu-ATrp-Tef-hERa
9589 bp
GAA2ERE107
phyK
Kan(r)
ColE1 ori
d25S rDNA-1
PHO5-Terminator
Reportergen phyK

Figur 1

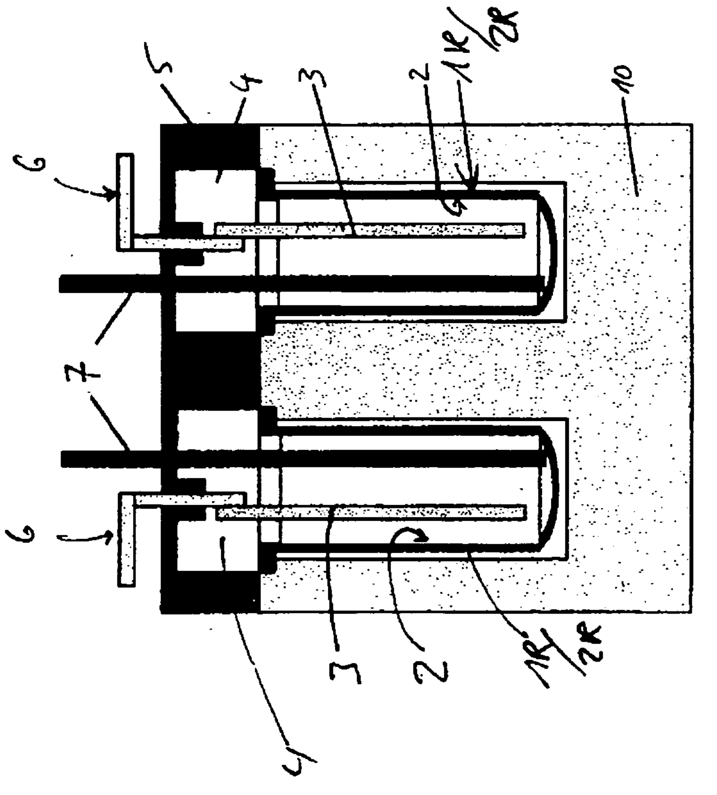


Figur 2

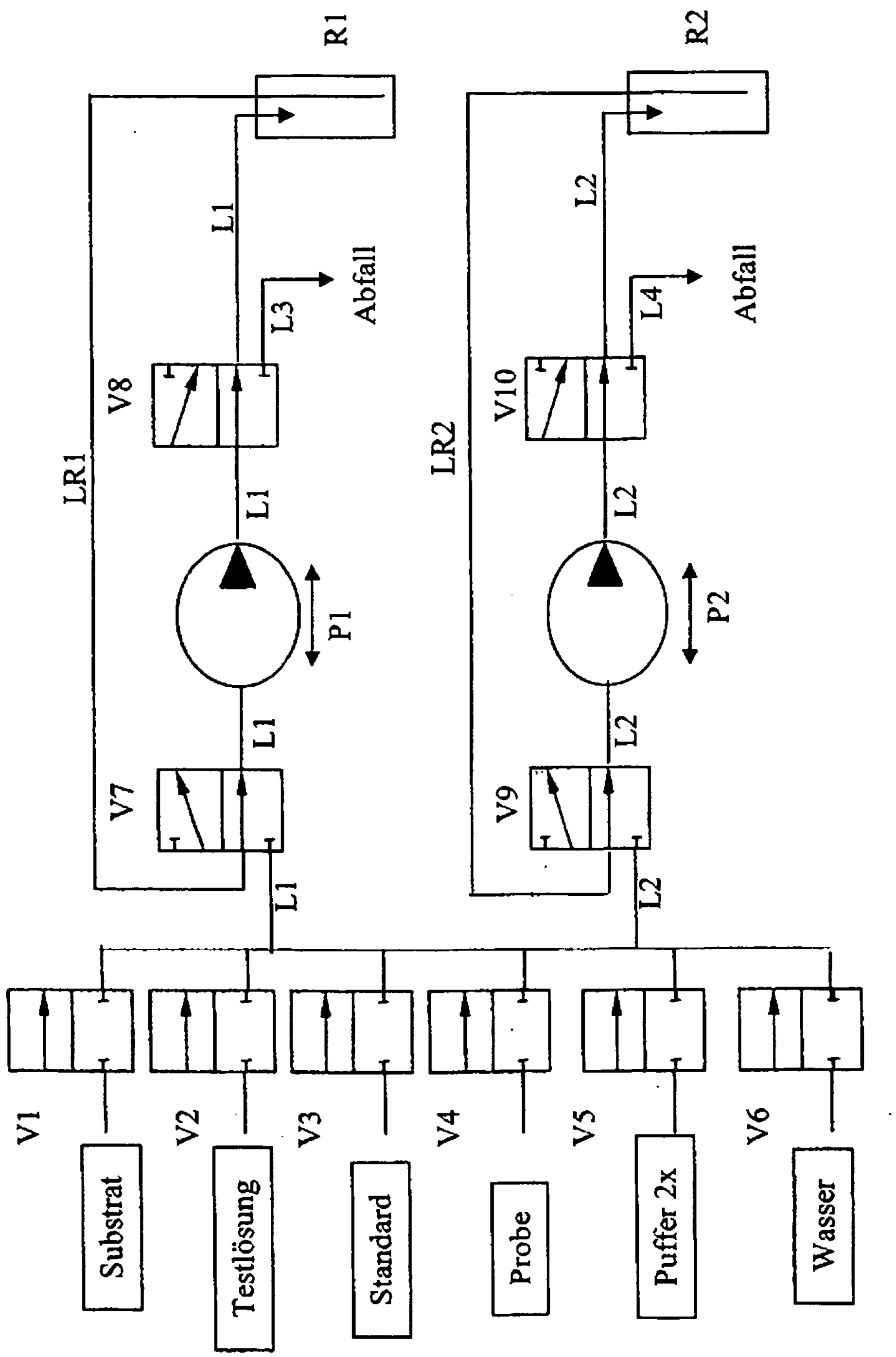
R1
R2
L1
L2
L3
L4
Abfall
Abfall
LR1
LR2
V7
V8
V9
V10
P1
P2
V1
V2
V3
V4
V5
V6
Substrat
Testlösung
Standard
Probe
Puffer 2x
Wasser

Figur 3

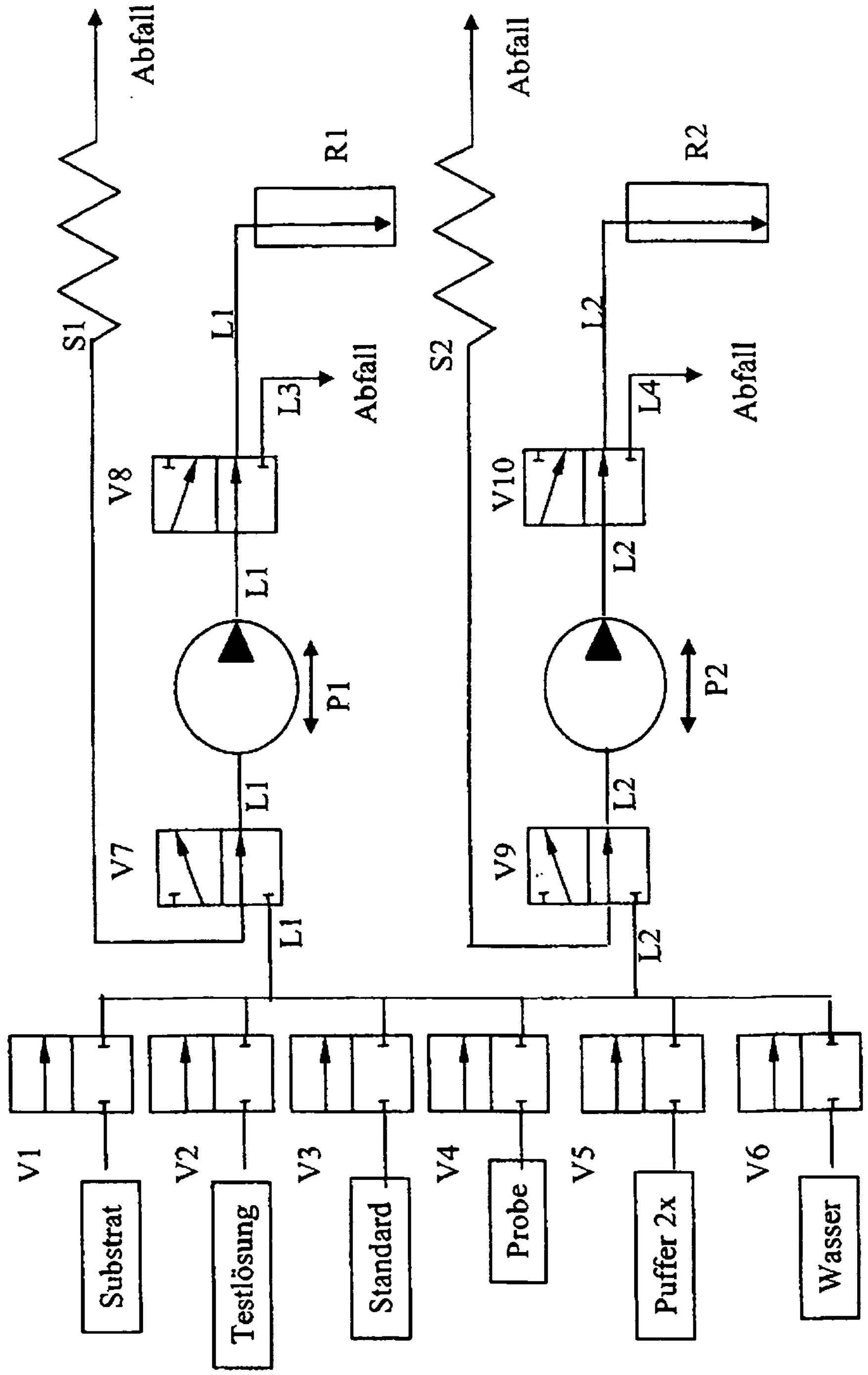
Abfall
Abfall
R1
R2
S1
S2
L1
L2
L3
L4
Abfall
Abfall
V8
V10
P1
P2
V7
V9
V1
V2
V3
V4
V5
V6
Substrat
Testlösung
Standard
Probe
Puffer 2x
Wasser

Figur 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10345386 A1
- US 4513280 A
- WO 9744658 A
- DD 298821 A5
- EP 0105225 W


### In der Beschreibung aufgeführte Nicht-Patentliteratur


- **HAHN ; TAG ; RIEDEL ; UHLIG ; BARONIAN ; GELLISSEN ; KUNZE.** *Biosens. Bioelectron,* 19. Januar 2006
- **JUNGBAUER ; BECK.** *Journal of Chromatography B,* 2002, 167-178
- *Der Jahresforschungsbericht 2004 des IPK Gatersleben,* 2004, 125-127
- **KARUBE ; NAKANISHI.** *I.E.E.E Engineering in Medicine and Biology,* 1994, 364-374
- **WANG et al.** *Appl. Microbiol. Biotechnol*
- **WARTMANN et al.** *FEMS Yeast Research,* 2003, vol. 3, 223-232
- **WARTMANN et al.** *Yeast,* 1998, vol. 14, 1017-1025
- Arxula adeninivorans in: Non-conventional yeasts. **KUNZE et al.** Biotechnology. Springer Verlag, 1996, 389-409
- **P. KAUR ; A. LINGNER ; B.SINGH ; E. BOER ; J. POLAJEVA ; G. STEINBORN ; R. BODE ; G. GELLISSEN ; T. SATYANARAYANA ; G. KUNZE.** *Antonie van Leeuwenhoek,* 2007, vol. 91, 45-55